# EUROPEAN PATENT APPLICATION

(11) **EP 2 062 920 A2**
(43) Date of publication of application: **27.05.2009**
(21) Application number: 08169730.2
(22) Date of filing: 21.11.2008
(51) Int. Cl.: C07K 16/44, C07K 16/40, C07K 16/18, G01N 33/53

(54) **Protein phosphorylation by basophilic serine/threonine kinases in insulin signalling pathways**

(30) Priority: 21.11.2007 US 3931 P
(71) Applicant: Hornbeck, Peter, Magnolia, MA 01930 (US); Guo, Ailan, Burlington, MA 01803 (US)
(72) Inventor: Hornbeck, Peter, Magnolia, MA 01930 (US); Guo, Ailan, Burlington, MA 01803 (US)
(74) Representative: Hill, Justin John

(57) **Abstract**

The invention discloses 142 novel phosphorylation sites identified in insulin signaling pathways, peptides (including AQUA peptides) comprising a phosphorylation site of the invention, antibodies specifically bind to a novel phosphorylation site of the invention, and diagnostic and therapeutic uses of the above.

## Description

### RELATED APPLICATIONS

Pursuant to 35 U.S.C. § 119(e) this application claims the benefit of, and priority to, provisional application U.S.S.N. 61/003,931 filed November 21, 2007, the disclosures of which is incorporated herein, in its entirety, by reference.

### FIELD OF THE INVENTION

This invention relates to novel Serine/Threonine (S/T) protein phosphorylation sites in insulin signaling pathways as well as methods and compositions for detecting, quantitating and modulating same.

### BACKGROUND OF THE INVENTION

The activation of proteins by post-translational modification is an important cellular mechanism for regulating most aspects of biological organization and control, including growth, development, homeostasis, and cellular communication. Protein phosphorylation, for example, plays a critical role in the etiology of many pathological conditions and diseases, including diabetes, cancer, developmental disorders, and autoimmune diseases. Yet, in spite of the importance of protein modification, it is not yet well understood at the molecular level, due to the extraordinary complexity of signaling pathways, and the slow development of technology necessary to investigate it.

Insulin and other growth factors such as epidermal growth factor (EGF) are activated upon ligand binding. Receptor activation rapidly sets in motion a biochemical cascade of enormous complexity involving thousands of different types of molecules. Cell signals that originate from the activated insulin receptor (InsR), which is itself a protein kinase, very quickly activate a number of downstream serine/threonine kinases, which integrate the signals from the receptor into a coordinated and complex cellular response.

The AGC protein kinase group contains 50 different kinases that share similar kinase domain structures and substrate preferences. The group includes PDK1, a master regulator of many other AGC kinases, and the Akt, protein kinase A (PKA), protein kinase C (PKC), ribosomal S6 kinase (RSK), serum- and glucocorticoid-induced kinase (SGK), and NDR/LATS kinase families **(**Mora et al, Semin Cell Dev Biol. 2004 15:161-70**).** AGC kinases play critical roles in regulating growth, metabolism, proliferation and survival.

All of the AGC kinases studied to date are basophilic, i.e. they prefer basic amino acids flanking the serines/threonines that they phosphorylate (see Figure 6). Some members of the AGC group have stringent requirements for basic residues at specific locations relative to the phosphorylated serine/threonine. For instance, the three Akt isoforms (Aktl-3) appear to have a nearly exclusive preference for arginine (R) at positions -5 and -3 relative to the phospho-acceptor residue at position 0. p70S6K and p90RSK can apparently tolerate lysine (K) or arginine (R) at position -5 better than the Akt kinases (Manning and Cantley, Cell. 2007 129:1261-74). Other kinases have more relaxed requirements for arginine on either side of the phospho-acceptor. PKA prefers at least one argine/lysine at the -1, -2 or -3 positions. PKCs can phosphorylate sequences with arginines or lysines either C-terminal or N-terminal to the phosphoacceptor site (see Figure 6).

A crucial early event in the insulin regulatory network is the activation of phosphatidylinositol 3-kinase (PI3K) and generation of phosphatidylinositol 3,4,5-trisphosphate (PIP3), a second messenger on the inner surface of the plasma membrane. PI3K phosphorylates phosphatidylinositol-4,5-bisphosphate (PIP2) to generate PIP3, in a reaction that can be reversed by the PIP3 phosphatase PTEN. PIP3 then recruits the AGC kinases PDK1 and Akt to the plasma membrane, where PDK1 is rapidly phosphorylated and activated (Cohen et al., FEBS Lett. 1997 Jun 23;410(1):3-10; Riojas et al, J Biol Chem. 2006 281:21588-93).

mTOR, another crucial substrate of PDK, is an typical protein kinase that is required for cell survival and regulates cell growth through the regulation of protein synthesis. When sufficient nutrients are available, mTOR is activated and regulates protein synthesis by phosphorylating and activating p70S6K, an AGC kinase with a specificity nearly identical to that of Akt, and phosphorylating and inactivating eukaryotic initiation factor 4E-binding protein (4E-BP1), a repressor of mRNA translation (Hay and Sonenberg, Genes Dev. 2004 18:1926-45).

Much of this control exerted by PDK1 and mTOR is mediated by their ability to phosphorylate key AGC kinases, which in turn regulate many downstream effector networks. PDK1 activates Akt and other members of the AGC group including PKC-delta, PKC-epsilon, PKC-zeta, PKN1, PKN2, SGK, SGK2, and SGK3. Many of these basophilic kinases in turn regulate other ser/thr kinases networks. For example, Akt1 or Akt2 phosphorylates ASK1, IKK-alpha, MLK3, SEK1, mTOR, QIK, Rafl, and WML1; PKC-delta phosphorylates LIMK2, and p38-alpha.

Signals from the insulin receptor set in motion a concerted response that touches virtually every compartment of cellular dynamics: metabolic regulation, DNA transcription, RNA processing, protein synthesis, vesicular transport, endocytosis, adhesion, molecular transport, and protein degradation. Much of this activity is coordinated by the basophilic AGC kinases, but very little of these processes are understood at the molecular level.

Despite the identification of a few key signaling molecules involved in insulin signaling and related disease progression are known, the vast majority of signaling protein changes and signaling pathways underlying the various associated disease types remain unknown. Therefore, there is presently an incomplete and inaccurate understanding of how protein activation within insulin signaling pathways drives various diseases including, among many others, various types of cancer and diabetes. Accordingly, there is a continuing and pressing need to unravel the molecular mechanisms of disease progression by identifying the downstream signaling proteins mediating cellular transformation in these diseases.

Presently, diagnosis of many insulin-signaling related diseases and cancer may made by tissue biopsy and detection of different cell surface markers. However, misdiagnosis can occur since some disease types can be negative for certain markers and because these markers may not indicate which genes or protein kinases may be deregulated. Although the genetic translocations and/or mutations characteristic of a particular form of a disease including cancer can be sometimes detected, it is clear that other downstream effectors of constitutively active signaling molecules having potential diagnostic, predictive, or therapeutic value, remain to be elucidated.

Accordingly, identification of downstream signaling molecules and phosphorylation sites involved in different types of diseases including for example, cancer or diabetes, and development of new reagents to detect and quantify these sites and proteins may lead to improved diagnostic/prognostic markers, as well as novel drug targets, for the detection and treatment of many diseases.

### SUMMARY OF THE INVENTION

The present invention provides in one aspect novel serine and threonine phosphorylation sites (Table 1) identified in insulin signaling pathways. The novel sites occur in proteins such as: Adaptor/Scaffold proteins, apoptosis proteins enzyme proteins, non-protein kinases, phosphatases, proteases, protein kinases Ser/Thr (non-receptor), vesicle proteins, g proteins or regulator proteins, chromatin or DNA binding/repair/replication proteins, cytoskeletal proteins, receptor/channel/transporter/cell surface proteins, RNA processing proteins, translation proteins, activator proteins, chaperone proteins, calcium binding proteins, transcriptional regulator proteins, tumor suppressor proteins, lipid binding proteins, secreted proteins, adhesion or extracellular matrix proteins, inhibitor proteins, mitochondrial proteins, endoplasmic reticulum or golgi apparatus proteins, cell cycle regulation proteins, transcriptional regulator proteins, ubiquitan conjugating proteins, proteins of unknown function and vesicle proteins.

In another aspect, the invention provides peptides comprising the novel phosphorylation sites of the invention, and proteins and peptides that are mutated to eliminate the novel phosphorylation sites.

In another aspect, the invention provides modulators that modulate serine and/or threonine phosphorylation at a novel phosphorylation sites of the invention, including small molecules, peptides comprising a novel phosphorylation site, and binding molecules that specifically bind at a novel phosphorylation site, including but not limited to antibodies or antigen-binding fragments thereof.

In another aspect, the invention provides compositions for detecting, quantitating or modulating a novel phosphorylation site of the invention, including peptides comprising a novel phosphorylation site and antibodies or antigen-binding fragments thereof that specifically bind at a novel phosphorylation site. In certain embodiments, the compositions for detecting, quantitating or modulating a novel phosphorylation site of the invention are Heavy-Isotype Labeled Peptides (AQUA peptides) comprising a novel phosphorylation site.

In another aspect, the invention discloses phosphorylation site specific antibodies or antigen-binding fragments thereof. In one embodiment, the antibodies specifically bind to an amino acid sequence comprising a phosphorylation site identified in Table 1 when the serine or threonine identified in Column D is phosphorylated, and do not significantly bind when the serine or threonine is not phosphorylated. In another embodiment, the antibodies specifically bind to an amino acid sequence comprising a phosphorylation site when the serine or threonine is not phosphorylated, and do not significantly bind when the serine or threonine is phosphorylated.

In another aspect, the invention provides a method for making phosphorylation site-specific antibodies.

In another aspect, the invention provides compositions comprising a peptide, protein, or antibody of the invention, including pharmaceutical compositions.

In a further aspect, the invention provides methods of treating or preventing insulin signaling pathway related disease in a subject, wherein the disease is associated with the phosphorylation state of a novel phosphorylation site in Table 1, whether phosphorylated or dephosphorylated. In certain embodiments, the methods comprise administering to a subject a therapeutically effective amount of a peptide comprising a novel phosphorylation site of the invention. In certain embodiments, the methods comprise administering to a subject a therapeutically effective amount of an antibody or antigen-binding fragment thereof that specifically binds at a novel phosphorylation site of the invention.

In a further aspect, the invention provides methods for detecting and quantitating phosphorylation at a novel serine or threonine phosphorylation site of the invention.

In another aspect, the invention provides a method for identifying an agent that modulates a serine and/or threonine phosphorylation at a novel phosphorylation site of the invention, comprising: contacting a peptide or protein comprising a novel phosphorylation site of the invention with a candidate agent, and determining the phosphorylation state or level at the novel phosphorylation site. A change in the phosphorylation state or level at the specified serine and/or threonine in the presence of the test agent, as compared to a control, indicates that the candidate agent potentially modulates serine and/or threonine phosphorylation at a novel phosphorylation site of the invention.

In another aspect, the invention discloses immunoassays for binding, purifying, quantifying and otherwise generally detecting the phosphorylation of a protein or peptide at a novel phosphorylation site of the invention.

Also provided are pharmaceutical compositions and kits comprising one or more antibodies or peptides of the invention and methods of using them.

### BRIEF DESCRIPTION OF THE DRAWINGS

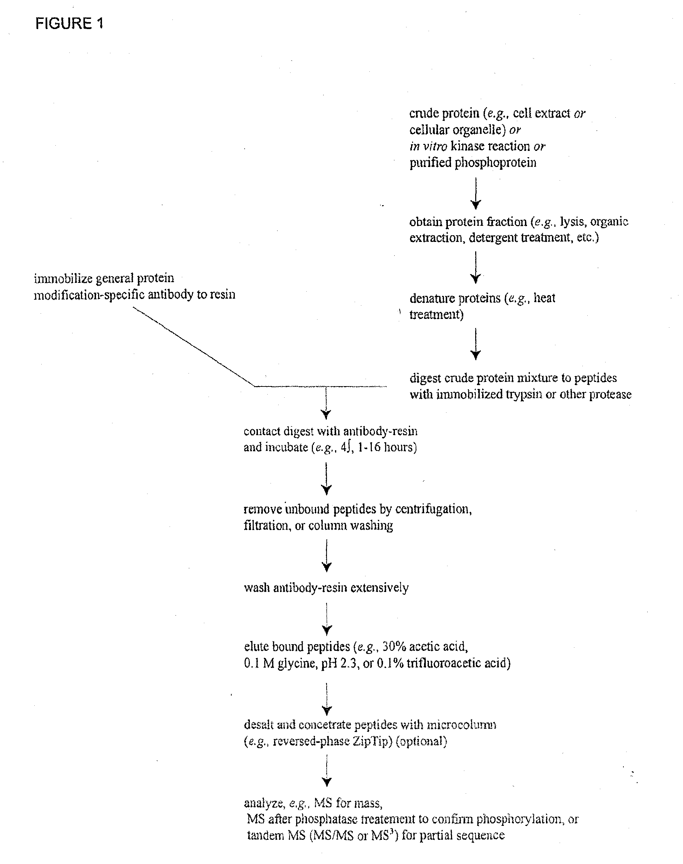

**FIGURE 1** is a diagram depicting the immuno-affinity isolation and mass-spectrometric characterization methodology (IAP) used in the Examples to identify the novel phosphorylation sites disclosed herein.

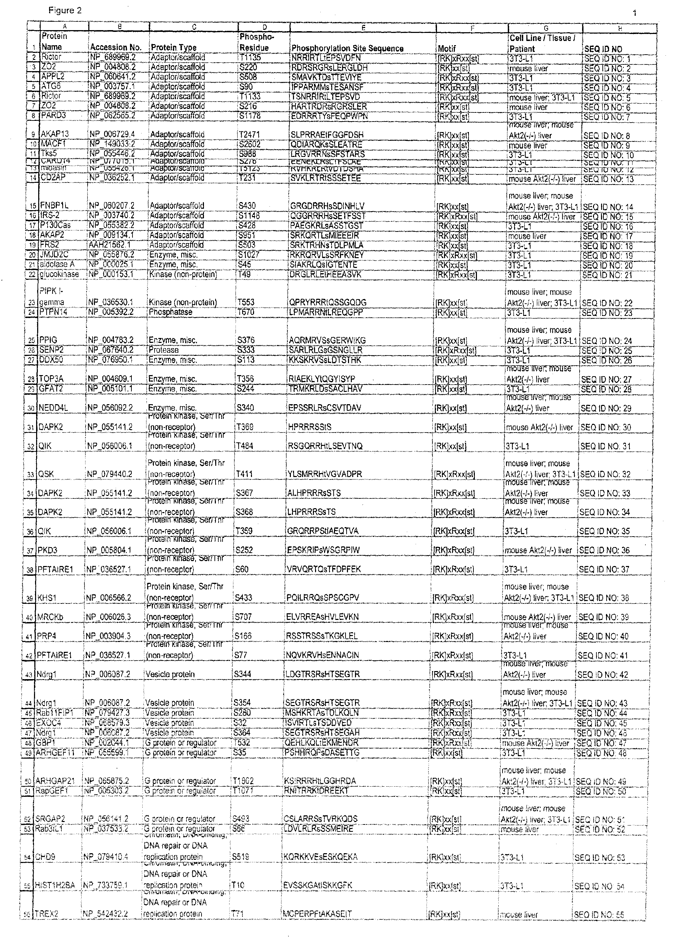

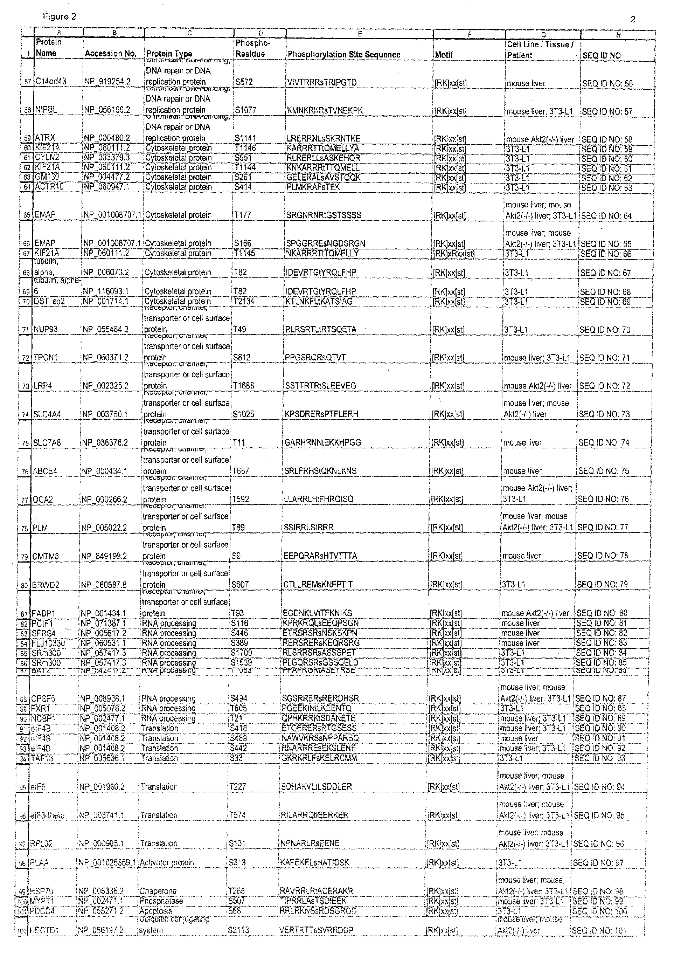

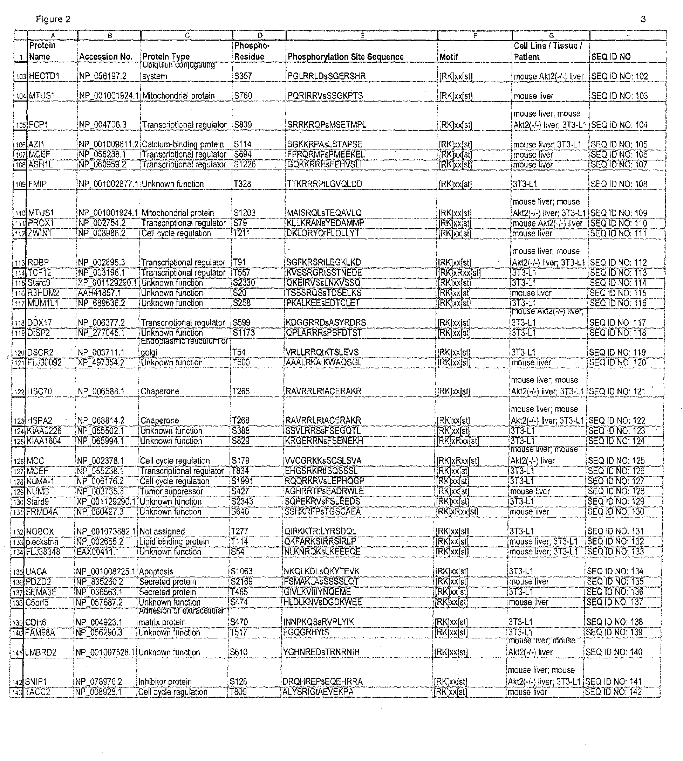

**FIGURE 2** is a table (corresponding to Table 1) summarizing the 142 novel phosphorylation sites of the invention: Column A = the parent proteins from which the phosphorylation sites are derived; Column B = the SwissProt accession number for the human homologue of the identified parent proteins; Column C = the protein type/classification; Column D = the serine and/or threonine residue at which phosphorylation occurs (each number refers to the amino acid residue position of the serine and/or threonine in the parent human protein, according to the published sequence retrieved by the SwissProt accession number); Column E = flanking sequences of the phosphorylatable serine and/or threonine residues; sequences (SEQ ID NOs: 1-142) were identified using Trypsin digestion of the parent proteins; in each sequence, the serine and/or threonine (see corresponding rows in Column D) appears in lowercase; Column F = the basophillic motif by which phosphorylation site can be characterized; Column G = the cell type(s)/ Tissue/ Patient Sample in which each of the phosphorylation site was discovered; and Column H= the SEQ ID NOs of the trypsin-digested peptides identified in Column E.

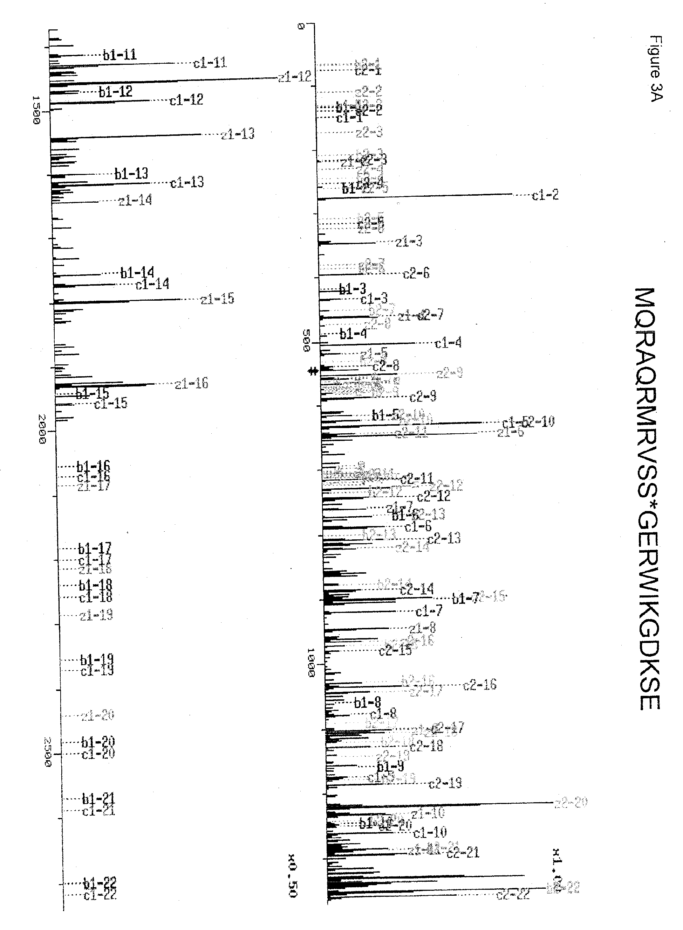

**FIGURE 3A** is an exemplary mass spectrograph depicting the detection of the phosphorylation of serine 376 in PPIG, as further described in Example 1 (red and blue indicate ions detected in MS/MS spectrum); S* indicates the phosphorylated serine (corresponds to lowercase "s" in Column E of Table 1; SEQ ID NO: 24).

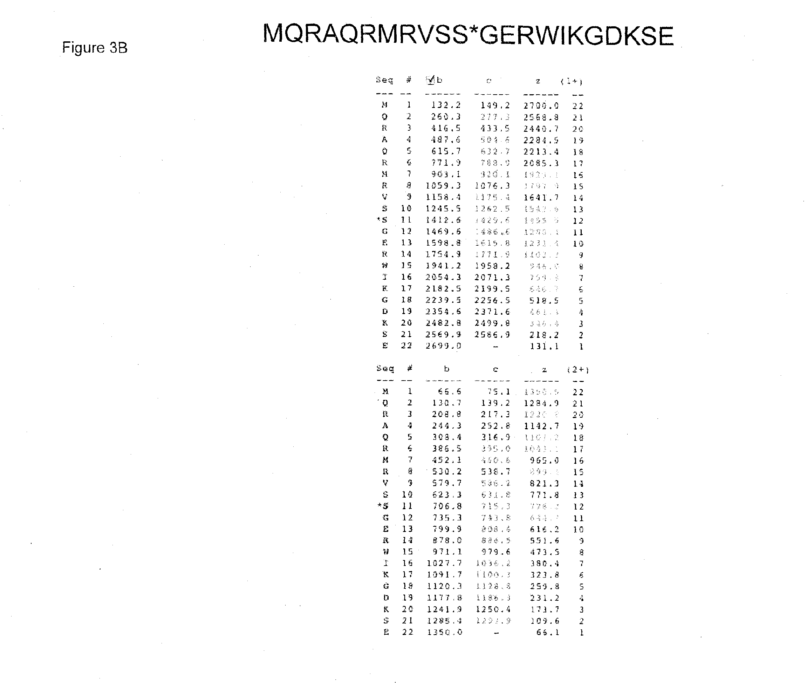

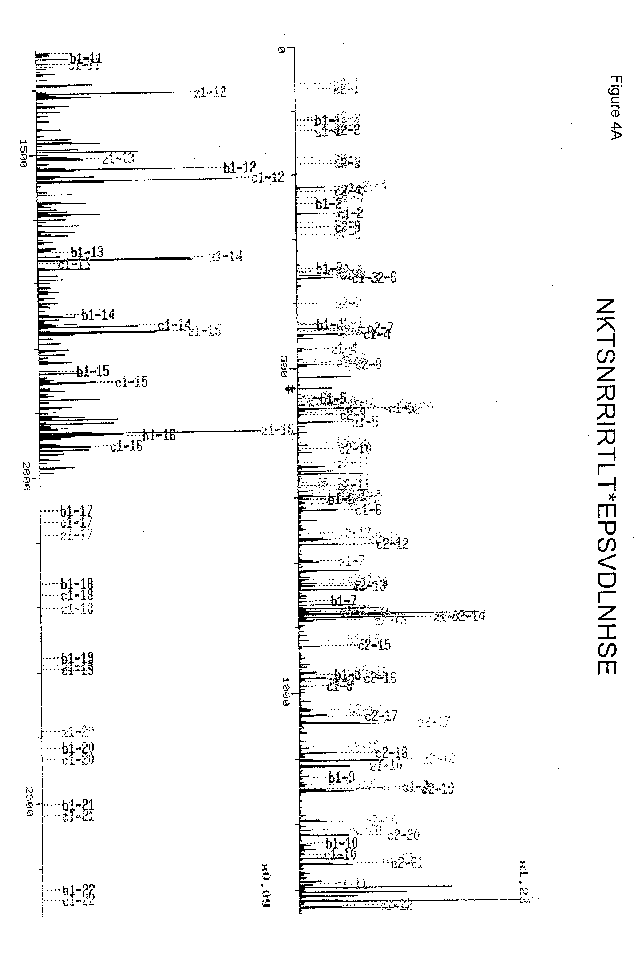

**FIGURE 3B** is the numerical data which correspond to the exemplary mass spectrograph of Figure 4A, depicting the detection of the phosphorylation of serine 376 in PPIG, as further described in Example 1 (red and blue indicate ions detected in MS/MS spectrum); S* indicates the phosphorylated serine (corresponds to lowercase "s" in Column E of Table 1; SEQ ID NO: 24).

**FIGURE 4A** is an exemplary mass spectrograph depicting the detection of the phosphorylation of threonine 1135 in Rictor, as further described in Example 1 (red and blue indicate ions detected in MS/MS spectrum); T* indicates the phosphorylated Threonine (corresponds to lowercase "t" in Column E of Table 1; SEQ ID NO: 1).

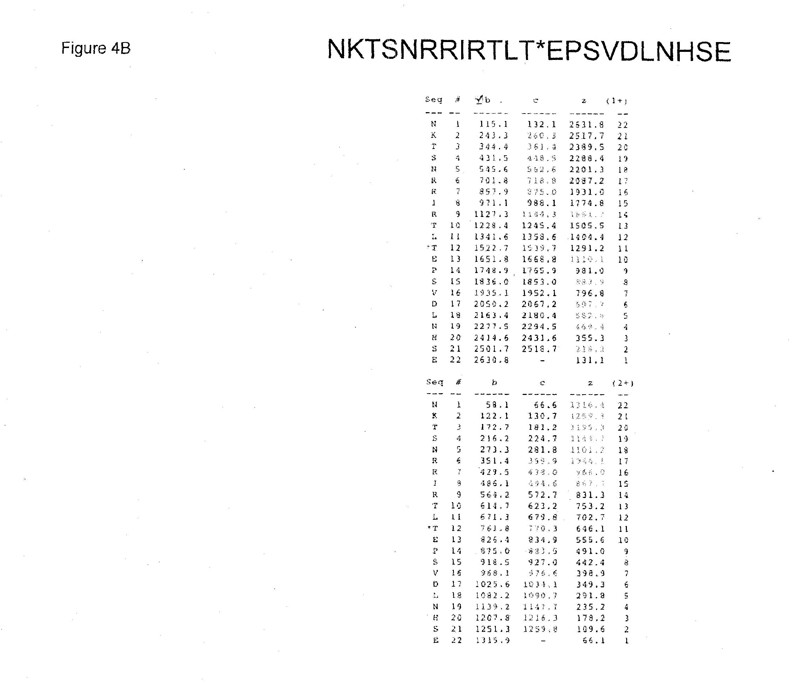

**FIGURE 4B** is the numerical data which correspond to the exemplary mass spectrograph of Figure 4A, depicting the detection of the phosphorylation of threonine 1135 in Rictor, as further described in Example 1 (red and blue indicate ions detected in MS/MS spectrum); T* indicates the phosphorylated Threonine (corresponds to lowercase "t" in Column E of Table 1; SEQ ID NO: 1).

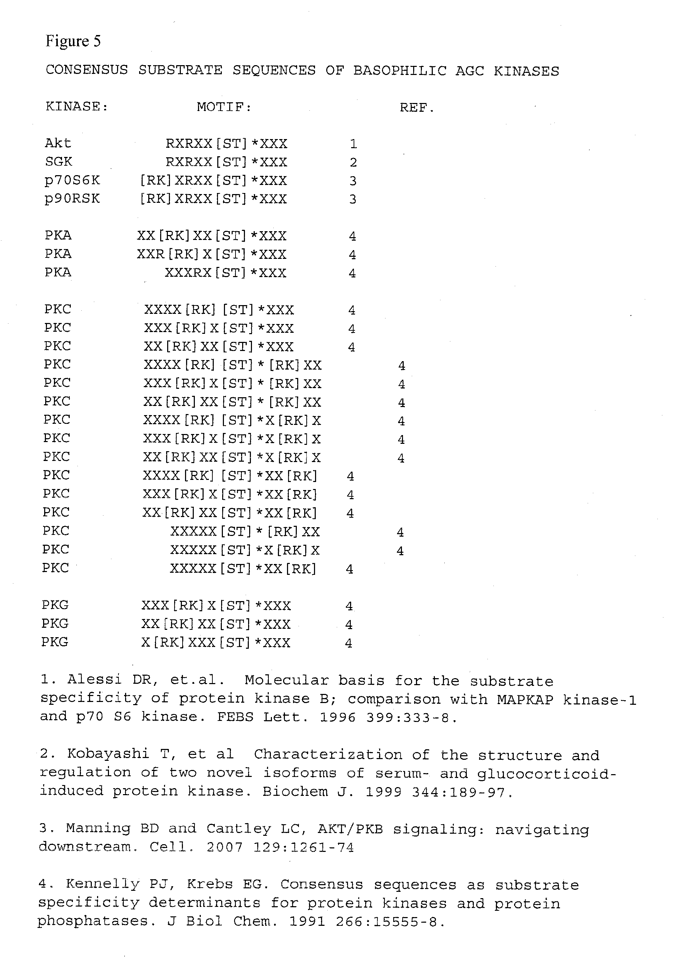

**FIGURE 5** is a table showing the various consensus substrate sequences of basophillic AGC kinases.

### DETAILED DESCRIPTION OF THE INVENTION

The inventors have discovered and disclosed herein novel serine and threonine phosphorylation sites in signaling proteins extracted from the cell line/tissue/patient sample listed in column G of Figure 2. The newly discovered phosphorylation sites significantly extend our knowledge of basophilic Ser/Thr kinases, substrates and of the proteins in which the novel sites occur. The disclosure herein of the novel phosphorylation sites and reagents including peptides and antibodies specific for the sites add important new tools for the elucidation of signaling pathways that are associate with a host of biological processes including cell division, growth, differentiation, develomental changes and disease. Their discovery in insulin signaling pathways cells provides and focuses further elucidation of many disease processes. And, the novel sites provide additional diagnostic and therapeutic targets.

### 1. Novel Phosphorylation Sites in Insulin signaling pathways

In one aspect, the invention provides 142 novel serine and/or threonine phosphorylation sites in signaling proteins from cellular extracts from insulin-responsive tissue samples (such as 3T3-L1; mouse liver; mouse Akt2(-/-) liver etc., as further described below in Examples), identified using the techniques described in "Immunoaffinity Isolation of Modified Peptides From Complex Mixtures," U.S. Patent Publication NO. 20030044848, Rush *et at.,* using Table 1 summarizes the identified novel phosphorylation sites.

These phosphorylation sites thus occur in proteins found in insulin signaling pathways. The sequences of the human homologues are publicly available in SwissProt database and their Accession numbers listed in Column B of Table 1. The novel sites occur in proteins such as: adaptor/scaffold proteins, enzyme/non-protein kinase/phoshpatase proteins, Ser/Thr (non-receptor) protein kinases, vesicle proteins, g proteins or regulator proteins, chromatin or DNA binding/repair/replication proteins, receptor/channel/transporter/cell surface proteins, RNA processing proteins, cytoskeletal proteins, transcriptional regulators and translation proteins. (*see* Column C of Table 1).

The novel phosphorylation sites of the invention were identified according to the methods described by Rush *el al.,* U.S. Patent Publication No. 20030044848, which are herein incorporated by reference in its entirety. Briefly, phosphorylation sites were isolated and characterized by immunoaffinity isolation and mass-spectrometric characterization (IAP) (Figure 1), using the following cellular extracts from insulin-responsive tissue samples: 3T3-L1; mouse liver; mouse Akt2(-/-) liver. In addition to the newly discovered phosphorylation sites (all having a phosphorylatable serine or threonine), many known phosphorylation sites were also identified.

The immunoaffinity/mass spectrometric technique described in Rush et al, *i.e.,* the "IAP" method, is described in detail in the Examples and briefly summarized below.

The IAP method generally comprises the following steps: (a) a proteinaceous preparation (e.g., a digested cell extract) comprising phosphopeptides from two or more different proteins is obtained from an organism; (b) the preparation is contacted with at least one immobilized motif-specific, context-independent antibody; (c) at least one phosphopeptide specifically bound by the immobilized antibody in step (b) is isolated; and (d) the modified peptide isolated in step (c) is characterized by mass spectrometry (MS) and/or tandem mass spectrometry (MS-MS). Subsequently, (e) a search program (e.g., Sequest) may be utilized to substantially match the spectra obtained for the isolated, modified peptide during the characterization of step (d) with the spectra for a known peptide sequence. A quantification step, e.g., using SILAC or AQUA, may also be used to quantify isolated peptides in order to compare peptide levels in a sample to a baseline.

In the IAP method as disclosed herein, a phospho-Akt substrate antibody (detecting RXRXXS/T motif) (commercially available from Cell Signaling Technology, Inc., Beverly, MA, Catalogue # 9614) may be used in the immunoaffinity step to isolate the widest possible number of phospho-serine and/or phospho-threonine containing peptides from the cell extracts.

As described in more detail in the Examples, lysates may be prepared from various carcinoma cell lines or tissue samples and digested with trypsin after treatment with DTT and iodoacetamide to alkylate cysteine residues. Before the immunoaffinity step, peptides may be pre-fractionated (e.g., by reversed-phase solid phase extraction using Sep-Pak C₁₈ columns) to separate peptides from other cellular components. The solid phase extraction cartridges may then be eluted (e.g., with acetonitrile). Each lyophilized peptide fraction can be redissolved and treated with a phospho-Akt substrate antibody (detecting RXRXXS/T motif) (commercially available from Cell Signaling Technology, Inc., Beverly, MA, Catalogue # 9614) immobilized on protein Agarose. Immunoaffinity-purified peptides can be eluted and a portion of this fraction may be concentrated (e.g., with Stage or Zip tips) and analyzed by LC-MS/MS (e.g., using a ThermoFinnigan LCQ Deca XP Plus ion trap mass spectrometer or LTQ). MS/MS spectra can be evaluated using, e.g., the program Sequest with the NCBI human protein database.

The novel phosphorylation sites identified are summarized in Table1/Figure 2. Column A lists the parent (signaling) protein in which the phosphorylation site occurs. Column D identifies the serine and/or threonine residue at which phosphorylation occurs (each number refers to the amino acid residue position of the serine and/or threonine in the parent human protein, according to the published sequence retrieved by the SwissProt accession number). Column E shows flanking sequences of the identified serine and/or threonine residues (which are the sequences of trypsin-digested peptides). Figure 2 also shows the particular type of cancer (see Column G) and cell line(s) (see Column F) in which a particular phosphorylation site was discovered.

**Table 1. Novel Tyrosine, Serine and Threonine Phosphorylation Sites.**

| | A | B | C | D | E | H |
|---|---|---|---|---|---|---|
| 1 | Protein Name | Accession No. | Protein Type | Phospho-Residue | Phosphorylation Site Sequence | SEQ ID NO |
| 2 | Rictor | NP_689969.2 | Adaptor/scaffold | T1135 | NRRIRTLtEPSVDFN | SEO ID NO: 1 |
| 3 | ZO2 | NP_004808.2 | Adaptor/scaffold | S220 | RDRSRGRsLERGLDH | SEQ ID NO: 2 |
| 4 | APPL2 | NP_060641.2 | Adaptor/scaffold | S508 | SMAVKTDsTTEVIYE | SEQ ID NO: 3 |
| 5 | ATG6 | NP_003757.1 | Adaptor/scaffold | S90 | IPPARMMsTESANSF | SEQ ID NO: 4 |
| 6 | Rictor | NP_689969.2 | Adaptor/scaffold | T1133 | TSNRRIRtLTEPSVD | SEQ ID NO: 5 |
| 7 | ZO2 | NP_004808.2 | Adaptor/scaffold | S216 | HARTRDRsRGRSLER | SEQ ID NO: 6 |
| 8 | PARD3 | NP_062565.2 | Adaptor/scaffold | S1178 | EDRRRTYsFEQPWPN | SEQ ID NO: 7 |
| 9 | AKAP13 | NP_006729.4 | Adaptor/scaffold | T2471 | SLPRRAEtFGGFDSH | SEQ ID NO: 8 |
| 10 | MACF1 | NP_149033.2 | Adaptor/scaffold | S2602 | QDIARQKsSLEATRE | SEQ ID NO: 9 |
| 11 | Tks5 | NP_055446.2 | Adaptor/scaffold | S988 | LRGVRRNsSFSTARS | SEQ ID NO: 10 |
| 12 | CARD14 | NP_077015.1 | Adaptor/scaffold | S276 | EENEKLRsLTFSLAE | SEQ ID NO: 11 |
| 13 | midasin | NP_055426.1 | Adaptor/scaffold | T5123 | RVHKRLRtVDTDSHA | SEQ ID NO: 12 |
| 14 | CD2AP | NP_036252.1 | Adaptor/scaffold | T231 | SVKLRTRtSSSETEE | SEQ ID NO: 13 |
| 15 16 | FNBP1L | NP_060207.2 | Adaptor/scaffold | S430 | GRGDRRHsSDINHLV | SEQ ID NO: 14 |
| | IRS-2 | NP_003740.2 | Adaptor/scaffold | S1148 | QGGRRRHsSETFSST | SEQ ID NO: 15 |
| 17 | P130Cas | NP_055382.2 | Adaptor/scaffold | S428 | PAEGKRLsASSTGST | SEQ ID NO: 16 |
| 18 | AKAP2 | NP_009134.1 | Adaptor/scaffold | S951 | SRKORTLsMIEEEIR | SEQ ID NO: 17 |
| 19 | FRS2 | AAH21562.1 | Adaptor/scaffold | S503 | SRKTRHNsTDLPDLPMLA | SEQ ID NO: 18 |
| 20 | JMJD2C | NP_055876.2 | Enzyme, misc. | S1027 | RKRQRVLsSRFKNEY | SEQ ID NO: 19 |
| 21 | aldolasa A | NP_000025.1 | Enzyme, misc. | S45 | SIAKRLOsIGTENTE | SEQ ID NO: 20 |
| 22 | glucokinase | NP_000153.1 | Kinase (non-protein) | T49 | DRGLRLEtHEEASVK | SEQ ID NO: 21 |
| 23 | PIPK l-gamma | NP_036530.1 | Kinase (non-protein) | T553 | QPRYRRRtQSSGQDG | SEQ ID NO: 22 |
| 24 | PTPN14 | NP_005392.2 | Phosphatase | T670 | LPMARRNtLREQGPP | SEQ ID NO: 23 |
| 25 | PPIG | NF_004783.2 | Enzyme, misc. | S376 | AQRMRVSsGERWIKG | SEQ ID NO: 24 |
| 26 | SENP2 | NP_067640.2 | Protease | S333 | SARLRLGsGSNGLLR | SEQ ID NO: 25 |
| 27 | DDX50 | NP_076950.1 | Enzyme, misc. | S113 | KKSKRVSsLDTSTHK | SEQ ID NO: 26 |
| 28 | TOP3A | NP_004609.1 | Enzyme, misc. | T356 | RIAEKLYtQGYISYP | SEQ ID NO: 27 |
| 29 | GFAT2 | NP_005101.1 | Enzyme, misc. | S244 | TRMKRLDsSACLHAV | SEQ ID NO: 28 |
| 30 | NEDD4L | NP_056092.2 | Enzyme, misc. | S340 | EPSSRLRsCSVTDAV | SEO ID NO: 29 |
| 31 | DAPK2 | NP_055141.2 | Protein kinase, Ser/Thr (non-receptor) | T369 | HPRRRSStS | SEQ ID NO: 30 |
| 32 | QIK | NP_056006.1 | Protein kinase, Ser/Thr (non-receptor) | T484 | RSGQRRHtLSEVTNQ | SEQ ID NO: 31 |
| 33 | QSK | NP_079440.2 | Protein kinase, Ser/Thr (non-receptor) | T411 | YLSMRRHtVGVADPR | SEQ ID NO: 32 |
| 34 | DAPK2 | NP_055141.2 | Protein kinase, Ser/Thr (non-receptor) | S367 | ALHPRRRsSTS | SEQ ID NO: 33 |
| 35 | DAPK2 | NP_055141.2 | Protein kinase, Ser/Thr (non-receptor) | S368 | LHPRRRSsTS | SEQ ID NO: 34 |
| 36 | QIK | NP_056006.1 | Protein kinase, Ser/Thr (non-receptor) | T359 | GRQRRPStIAEQTVA | SEQ ID NO: 35 |
| 37 | PKD3 | NP_005804.1 | Protein kinase, Ser/Thr (non-receptor) | S252 | EPSKRIPsWSGRPIW | SEQ ID NO: 36 |
| 38 | PFTAIRE1 | NP_036527.1 | Protein kinase, Ser/Thr (non-receptor) | S60 | VRVQRTQsTFDPFEK | SEQ ID NO: 37 |
| 39 | KHS1 | NP_006566.2 | Protein kinase, Ser/Thr (non-receptor) | S433 | PQILRRQsSPSCGPV | SEQ ID NO: 38 |
| 40 | MRCKb | NP_006026.3 | Protein kinase, Ser/Thr (non-receptor) | S707 | ELVRREAsHVLEVKN | SEO ID NO: 39 |
| 41 | PRP4 | NP_003904.3 | Protein kinase, Ser/Thr (non-receptor) | S166 | RSSTRSSsTKGKLEL | SEQ ID NO: 40 |
| 42 | PFTAIRE1 | NP_036527.1 | Protein kinase, Ser/Thr (non-receptor) | S77 | NQVKRVHsENNACIN | SEQ ID NO: 41 |
| 43 | Ndrg1 | NP_006087.2 | Vesicle protein | S344 | LDGTRSRsHTSEGTR | SEQ ID NO: 42 |
| 44 | Ndrg1 | NP_006087.2 | Vesicle protein | S354 | SEGTRSRsHTSEGTR | SEQ ID NO: 43 |
| 45 | Rab11FIP1 | NP_079427.3 | Vesicle protein | S280 | MSHKRTAsTDLKQLN | SEQ ID NO: 44 |
| 46 | EXOC4 | NP_068579.3 | Vesicle protein | S32 | ISVIRTLsTSDDVED | SEQ ID NO: 45 |
| 47 | Ndrg1 | NP_006087.2 | Vesicle protein | S364 | SEGTRSRsHTSEGAH | SEQ ID NO: 46 |
| 48 | GBP1 | NP_002044.1 | G protein or regulator | T532 | QEHLKQLtEKMENDR | SEQ ID NO: 47 |
| 49 | ARHGEF11 | NP_055599.1 | G protein or regulator | S35 | PSHHRQPsDASETTG | SEQ ID NO: 48 |
| 50 | ARHGAP21 | NP_065875.2 | G protein or regulator | T1802 | KSIRRRHtLGGHRDA | SEO ID NO: 49 |
| 51 | RapGEF1 | NP_005303.2 | G protein or regulator | T1071 | RNITRRKtDREEKT | SEQ ID NO_{:} 50 |
| 52 | SRGAP2 | NP_056141.2 | G protein or regulator | S493 | CSLARRSsTVRKQDS | SEQ ID NO: 51 |
| 53 | Rab3IL1 | NP_037533.2 | G protein or regulator | S66 | LDVLRLRsSSMEIRE | SEQ ID NO: 52 |
| 54 | CHO9 | NP_079410.4 | Chromatin, DNA-binding, DNA repair or DNA replication protein | S519 | KQRKKVEsESKQEKA | SEQ ID NO: 53 |
| 55 | HIST1H2BA | NP_733759.1 | Chromatin, DNA-binding, DNA repair or DNA replication protein | T10 | EVSSKGAtISKKGFK | SEQ ID NO: 54 |
| 56 | TREX2 | NP_542432.2 | Chromatin, DNA-binding, DNA repair or DNA replication protein | T71 | MCPERPFtAKASEIT | SEQ ID NO: 55 |
| 57 | C14orf43 | NP_919254.2 | Chromatin, DNA-binding, DNA repair or DNA replication protein | S572 | VIVTRRRsTRIPGTD | SEQ ID NO: 56 |
| 58 | NIPBL | NP_056199.2 | Chromatin, DNA-binding, DNA repair or DNA replication protein | S1077 | KMNKRKRsTVNEKPK | SEQ ID NO: 57 |
| 59 | ATRX | NP_000480.2 | Chromatin, DNA-binding, DNA repair or DNA replication protein | S1141 | LRERRNLsSKRNTKE | SEQ ID NO: 58 |
| 60 | KIF21A | NP_060111.2 | Cytoskeletal protein | T1146 | KARRRTTtQMELLYA | SEQ ID NO: 59 |
| 61 | CYLN2 | NP_003379.3 | Cytoskeletal protein | S551 | RLRERLLsASKEHQR | SEQ ID NO: 60 |
| 62 | KIF21A | NP_060111.2 | Cytoskeletal protein | T1144 | KNKARRRtTTQMELL | SEQ ID NO: 61 |
| 63 | GM130 | NP_004477.2 | Cytoskeletal protein | S261 | GELERALsAVSTQQK | SEQ ID NO: 62 |
| 64 | ACTR10 | NP_060947.1 | Cytoskeletal protein | S414 | PLMKRAFsTEK | SEQ ID NO: 63 |
| 65 | EMAP | NP_00100870 7.1 | Cytoskeletal protein | T177 | SRGNRNRtGSTSSSS | SEQ ID NO: 64 |
| 66 | EMAP | NP_00100870 7.1 | Cytoskeletal protein | S166 | SPGGRREsNGDSRGN | SEQ ID NO: 65 |
| 67 | KIF21A | NP_060111.2 | Cytoskeletal protein | T1145 | NKARRRTtTQMEILY | SEQ ID NO: 66 |
| 68 | tubulin, alpha, ubiquitous | NP_006073.2 | Cytoskeletal protein | T82 | IDEVRTGtYRQLFHP | SEQ ID NO: 67 |
| 69 | tubulin, alpha-6 | NP_116093.1 | Cytoskeletal protein | T82 | IDEVRTGtYRQLFHP | SEQ ID NO: 68 |
| 70 | DST iso2 | NP_001714.1 | Cytoskeletal protein | T2134 | KTLNKFLtKATSIAG | SEQ ID NO: 69 |
| 71 | NUP93 | NP_055484.2 | Receptor, channel, transporter or cell surface protein | T49 | RLRSRTLtRTSQETA | SEQ ID NO: 70 |
| 72 | TPCN1 | NP_060371.2 | Receptor, channel, transporter or cell surface protein | S812 | PPGSRQRsQTVT | SEQ ID NO: 71 |
| 73 | LRP4 | NP_002325.2 | Receptor, channel, transporter or cell surface protein | T1688 | SSTTRTRtSLEEVEG | SEQ ID NO: 72 |
| 74 | SLC4A4 | NP_003750.1 | Receptor, channel, transporter or cell surface protein | S1025 | KPSDRERsPTFLERH | SEQ ID NO: 73 |
| 75 | SLC7A8 | NP_036376.2 | Receptor, channel, transporter or cell surface protein | T11 | GARHRNNtEKKHPGG | SEQ ID NO: 74 |
| 76 | ABCB4 | NP_000434.1 | Receptor, channel, transporter or cell surface protein | T687 | SRLFRHStQKNLKNS | SEQ ID NO: 75 |
| 77 | OCA2 | NP_000266.2 | Receptor, channel, transporter or cell surface protein | T592 | LLARRLHtFHRQISQ | SEQ ID NO: 76 |
| 78 | PLM | NP_005022.2 | Receptor, channel, transporter or cell surface protein | T89 | SSIRRLStRRR | SEQ ID NO: 77 |
| 79 | CMTM8 | NP_849199.2 | Receptor, channel, transporter or cell surface protein | S9 | EEPQRARsHTVTTTA | SEQ ID NO: 78 |
| 80 | BRWD2 | NP_060587.8 | Receptor, channel, transporter or cell surface protein | S607 | CTLLREMsKNFPTIT | SEQ ID NO: 79 |
| 81 | FABP1 | NP_001434.1 | Receptor, channel, transporter or cell surface protein | T93 | EGDNKLVtTFKNIKS | SEQ ID NO: 80 |
| 82 | PCIF1 | NP_071387.1 | RNA processing | S116 | KPRKRQLsEEQPSGN | SEQ ID NO: 81 |
| 83 | SFRS4 | NP_005617.2 | RNA processing | S446 | ETRSRSRsNSKSKPN | SEQ ID NO: 82 |
| 84 | FLJ10330 | NP_060531.1 | RNA processing | S389 | RERSRERsKEQRSRG | SEQ ID NO: 83 |
| 85 | SRm300 | NP_057417.3 | RNA processing | S1709 | RLSRRSRsASSSPET | SEQ ID NO: 84 |
| 86 | SRm300 | NP_057417.3 | RNA processing | S1539 | PLGQRSRsGSSQELD | SEQ ID NO: 85 |
| 87 | BAT2 | NP_542417.2 | RNA processing | T1083 | PPAPRGRtASETRSE | SEQ ID NO: 86 |
| 88 | CPSF6 | NP_008938.1 | RNA processing | S494 | SGSRRERsRERDHSR | SEQ ID NO: 87 |
| 89 | FXR1 | NP_005078.2 | RNA processing | T605 | PGEEKINtLKEENTQ | SEQ ID NO: 88 |
| 90 | NCBP1 | NP_002477.1 | RNA processing | T21 | QPHKRRKtSDANETE | SEQ ID NO: 89 |
| 91 | eIF4B | NP_001408.2 | Translation | S418 | ETQERERsRTGSESS | SEQ ID NO: 90 |
| 92 | eIF4B | NP_001408.2 | Translation | S489 | NAWVKRSsNPPARSQ | SEQ ID NO: 91 |
| 93 | eIF4B | NP_001408.2 | Translation | S442 | RNARRREsEKSLENE | SEQ ID NO: 92 |
| 94 | TAF13 | NP_005636.1 | Translation | S33 | GKRKRLFsKELRCMM | SEQ ID NO: 93 |
| 95 | eIF5 | NP_001960.2 | Translation | T227 | SDHAKVLtLSDDLER | SEQ ID NO: 94 |
| 96 | eIF3-theta | NP_003741.1 | Translation | T574 | RILARRQTlEERKER | SEQ ID NO: 95 |
| 97 | RPL32 | NP_000985.1 | Translation | S131 | NPNARLRsEENE | SEQ ID NO: 96 |
| 98 | PLAA | NP_00102685 9.1 | Activator protein | S318 | KAFEKELsHATIDSK | SEQ ID NO: 97 |
| 99 | HSP70 | NP_005336.2 | Chaperone | T265 | RAVRRLRtACERAKR | SEQ ID NO: 98 |
| 100 | MYPT1 | NP_002471.1 | Phosphatase | S507 | TIPRRLAsTSDIEEK | SEQ ID NO: 99 |
| 101 | PDCD4 | NP_055271.2 | Apoptosis | S68 | RRLRKNSsRDSGRGD | SEQ ID NO: 100 |
| 102 | HECTD1 | NP_056197.2 | Ubiquitin conjugating system | S2113 | VERTRTTsSVRRDDP | SEQ ID NO: 101 |
| 103 | HECTD1 | NP_056197.2 | Ubiquitin conjugating system | S357 | PGLRRLDsSGERSHR | SEQ ID NO: 102 |
| 104 | MTUS1 | NP_00100192 4.1 | Mitochondrial protein | S760 | PQRIRRVsSSGKPTS | SEQ ID NO: 103 |
| 105 | FCP1 | NP_004706.3 | Transcriptional regulator | S839 | SRRKRQPsMSETMPL | SEQ ID NO: 104 |
| 106 | AZI1 | NP_00100981 1.2 | Calcium-binding protein | S114 | SGKKRPAsLSTAPSE | SEQ ID NO: 105 |
| 107 | MCEF | NP_055238.1 | Transcriptional regulator | S694 | FFRQRMFsPMEEKEL | SEQ ID NO: 106 |
| 108 | ASH1L | NP_060959.2 | Transcriptional regulator | S1226 | GQKKRRHsFEHVSLI | SEQ ID NO: 107 |
| 109 | FMIP | NP_00100287 7.1 | Unknown function | T328 | TTKRRRPtLGVQLDD | SEQ ID NO: 108 |
| 110 | MTUS1 | NP_00100192 4.1 | Mitochondrial protein | S1203 | MALSRQLsTEQAVLQ | SEO ID NO: 109 |
| 111 | PROX1 | NP_002754.2 | Transcriptional regulator | S79 | KLLKRANsYEDAMMP | SEQ ID NO: 110 |
| 112 | ZWINT | NP_008988.2 | Cell cycle regulation | T211 | DKLQRYQtFLQLLYT | SEQ ID NO: 111 |
| 113 | RDBP | NP_002895.3 | Transcriptional regulator | T91 | SGFKRSRtLEGKLKD | SEO ID NO 112 |
| 114 | TCF12 | NP_003196.1 | Transcriptional regulator | T557 | KVSSRGRtSSTNEDE | SEQ ID NO: 113 |
| 115 | Stard9 | XP_00112929 0.1 | Unknown function | S2330 | QKEtRVSsLNKVSSQ | SEQ ID NO: 114 |
| 116 | R3HDM2 | AAH41857.1 | Unknown function | S20 | TSSSRQSsTDSELKS | SEQ ID NO: 115 |
| 117 | MUM1L1 | NP_689636.2 | Unknown function | S258 | PKALKEEsEDTCLET | SEQ ID NO: 115 |
| 118 | DDX17 | NP_006377.2 | Transcriptional regulator | S599 | KDGGRRDsASYRDRS | SEQ ID NO: 117 |
| 119 | DISP2 | NP_277045.1 | Unknown function | S1173 | QPLARRRsPSFDTST | SEQ ID NO: 118 |
| 120 | DSCR2 | NP_003711.1 | Endoplasmic reticulum or golgi | T54 | VRLLRRQtKTSLEVS | SEQ ID NO: 119 |
| 121 | FLJ30092 | XP_497354.2 | Unknown function | T600 | AAALRKAtKWAQSGL | SEQ ID NO: 120 |
| 122 | HSC70 | NP_006588.1 | Chaperone | T265 | RAVRRLRtACERAKR | SEQ ID NO: 121 |
| 123 | HSPA2 | NP_068814.2 | Chaperone | T268 | RAVRRLRtACERAKR | SEQ ID NO: 122 |
| 124 | KIAA0226 | NP_055502.1 | Unknown function | S388 | SSVLRRSsFSEGGTL | SEQ ID NO: 123 |
| 125 | KIAA1604 | NP_065994.1 | Unknown function | S829 | KRGERRNsFSENEKH | SEQ ID NO: 124 |
| 126 | MCC | NP_002378.1 | Cell cycle regulation | S179 | VVCGRKKsSCSLSVA | SEQ ID NO: 125 |
| 127 | MCEF | NP_055238.1 | Transcriptional regulator | T834 | EHGSRKRtISQSSSL | SEQ ID NO: 126 |
| 128 | NuMA-1 | NP_006176.2 | Cell cycle regulation | S1991 | RQQRKRVsLEPHQGP | SEQ ID NO: 127 |
| 129 | NUMB | NP_003735.3 | Tumor suppressor | S427 | AGHRRTPsEADRWLE | SEQ ID NO: 128 |
| 130 | Stard9 | XP_00112929 0.1 | Unknown function | S2343 | SQPEKRVsFSLEEDS | SEQ ID NO: 129 |
| 131 | FRMD4A | NP_060497.3 | Unknown function | S640 | SSHKRFPsTGSCAEA | SEQ ID NO: 130 |
| 132 | NOBOX | NP_00107388 2.1 | Not assigned | T277 | QIRKKTRtLYRSDQL | SEQ ID NO: 131 |
| 133 | pleckstrin | NP_002655.2 | Lipid binding protein | T114 | CKFARKStRRSIRLP | SEQ ID NO: 132 |
| 134 | FLJ38348 | EAX00411.1 | Unknown function | S54 | NLKNRQKsLKEEEQE | SEQ ID NO: 133 |
| 135 | UACA | NP_00100822 5.1 | Apoptosis | S1063 | NKQLKDLsQKYTEVK | SEQ ID NO: 134 |
| 136 | PDZD2 | NP_835260.2 | Secreted protein | S2169 | FSMAKLAsSSSSLQT | SEQ ID NO: 135 |
| 137 | SEMA3E | NP_036563.1 | Secreted protein | T465 | GIVLKVItIYNQEME | SEQ ID NO:136 |
| 138 | C5orf5 | NP_057687.2 | Unknown function | S474 | HLDLKNVsDGDKWEE | SEQ ID NO: 137 |
| 139 | CDH6 | NP_004923.1 | Adhesion or extracellular matrix protein | S470 | INNPKQSsRVPLYIK | SEQ ID NO: 138 |
| 140 | FAM98A | NP_056290.3 | Unknown function | T517 | FGQGRHYtS | SEQ ID NO:139 |
| 141 | LMBRD2 | NP_00100752 8.1 | Unknown function | S610 | YGHNREDsTRNRNIH | SEQ ID NO: 140 |
| 142 | SNIP1 | NP_078976.2 | Inhibitor protein | S128 | DRQHREPsEQEHRRA | SEQ ID NO: 141 |
| 143 | TACC2 | NP_008928.1 | Cell cycle regulation | T809 | ALYSRIGtAEVEKPA | SEQ ID NO: 142 |

One of skill in the art will appreciate that, in many instances the utility of the instant invention is best understood in conjunction with an appreciation of the many biological roles and significance of the various target signaling proteins/polypeptides of the invention. The foregoing is illustrated in the following paragraphs summarizing the knowledge in the art relevant to a few non-limiting representative peptides containing selected phosphorylation sites according to the invention.

Rictor, phosphorylated at Thrl 135 and 1133, is among the proteins listed in this patent. Rictor, a novel regulatory binding partner of the kinase mTOR, is an essential component of mTOR complex 2 (mTORC2), a kinase complex that phosphorylates the pro-survival kinase Akt at Ser473. mTORC2 is essential in early development. Rictor is required for the hydrophobic motif phosphorylation of Akt/PKB and PKCalpha, but not S6K1. Insulin signaling to FOXO3, but not to TSC2 or GSK3beta, requires rictor (Dev Cell. 2006 11:859-71). The rictor-mTOR complex modulates the phosphorylation of Protein Kinase C alpha (PKCalpha) and the actin cytoskeleton (Curr Biol. 2004 Jul 14:1296-302). The phosphorylation of Akt Ser473 by the mTOR/rictor complex is required for migration of metastatic MT2 breast cancer cells (Cancer Res. 2007 67:5293-9). Rictor has potential diagnostic and/or therapeutic implications for pathologies including childhood solid tumors and rhabdomyosarcoma (Mol Cancer Ther. 2007 6:1620-8), malignant glioma (J Clin Oncol. 2005 23:2411-22), and tumor invasion and metastasis (Cancer Res. 2007 67:5293-9). Rictor-mTOR may serve as a drug target in tumors that have lost the expression of PTEN (Science. 2005 307:1098-101). (PhosphoSite®, Cell Signaling Technology (Danvers, MA), Human PSD™, Biobase Corporation, (Beverly, MA)).

NDRG1, phosphorylated at Ser344, is among the proteins listed in this patent. NDRG1, N-myc downstream regulated gene 1, is a metastasis suppressor protein involved in growth arrest and cell differentiation. It is highly expressed in adult skeletal muscle and brain. It is induced by a variety of agents including p53, vitamin D, retinoic acid, phorbol esters, androgenic and estrogenic hormones, phosphatase and tensin homologue deleted on chromosome 10 (PTEN), nickel compounds, elevated intracellular calcium, DNA methylation and histone deacetylation inhibiting agents, DNA damage, and decreased glucose concentration. NRDG1 plays a role in cellular stress, p53-mediated apoptosis, the mitotic spindle checkpoint, and cell differentiation and proliferation. NDRG1 is unregulated by differentiation signals in various cancer cell lines, and suppresses tumor metastasis. It is strongly upregulated under hypoxic conditions, a condition that is prevalent in solid tumors. Hypoxia-inducible factor- (HIF-1α), p53, and N-Myc regulate the transcription of NDRG1. NDRG1 interacts with SIRT1/p53 signaling to attenuate hypoxic injury in human trophoblasts. Like the protein AS160, which is regulated by Akt in the insulin response (J Biol Chem. 2003 278:14599-602), NDRG1 is involved in Rab signaling. Rab proteins are small G proteins required for membrane trafficking. NDRG1 is a ubiquitous Rab4a effector protein that modulates angiogenesis and is involved in vesicular recycling of E-cadherin and transferrin. NDRG 1 knockdown delays the recycling rate of transferrin, while its overexpression increases the rate of transferrin recycling. Interacts with SIRT1/p53 signaling to attenuate hypoxic injury in human trophoblasts. It plays a specific role in the molecular cause of Charcot-Marie-Tooth type 4D disease and is a marker of tumor progression and enhancer of cellular differentiation (Carcinogenesis. 2007 Oct 4; [Epub ahead of print]). Mutations cause hereditary motor and sensory neuropathies. NDRG1 has potential diagnostic and/or therapeutic implications for multiple types of solid tumors (Carcinogenesis. 2007 Oct 4 [Epub ahead of print]), hepatocellular carcinoma (Mod Pathol. 2007 20:76-83), esophageal squamous cell carcinoma (Dis Esophagus. 2006 19:454-8), peripheral demyelinating neuropathies (Am J Hum Genet 2000 67:47-58), mast cell function and allergic responses (J Immunol. 2007 178:7042-53). (PhosphoSite®, Cell Signaling Technology (Danvers, MA), Human PSD™, Biobase Corporation, (Beverly, MA)).

DAPK2, phosphorylated at Thr369, is among the proteins listed in this patent. DAPK2 (death-associated protein kinase 2), an ubiquitous member of the DAP kinase subfamily of serine-threonine kinases, is activated by Ca(2+)/calmodulin and induces apoptosis in a calcium-calmodulin dependent manner. DAPK2 acts as a tumor suppressor by inhibiting cell adhesion/migration and promoting apoptosis. DAPK2 mediates membrane blebbing and the formation of autophagic vesicles. DAPK2 contains an N-terminal protein kinase domain followed by a conserved caimodulin-binding domain. Overexpression induces apoptosis. DAPK2 has potential diagnostic and/or therapeutic implications for pathologies and processes including autophagy, breast cancer (Cancer Res. 2006 66:5934-40), and myetopoiesis and myeloid leukemia (J Leukoc Biol. 2007 81:1599-608). (PhosphoSite®, Cell Signaling Technology (Danvers, MA), Human PSD™, Biobase Corporation, (Beverly, MA)),

JMJD2C, phosphorylated at Ser1027, is among the proteins listed in this patent. JMJD2C, is a histone demethylase that plays a central role in the histone code. It is implicated in the epigenetic reprogramming during early embryogenesis. It is preferentially expressed in undifferentiated embryonic stem (ES) cells. JMJD2C, along with JMJD1A, regulates self-renewal in ES cells. JMJD2C is a transcriptional corepressor that may play a role in cell cycle regulation. It specifically demethylates trimethylated Lys9 and Lys36 of histone H3 while it has no activity on mono- and dimethylated residues. Alternative splicing produces two isoforms of the human protein. This protein has potential diagnostic and/or therapeutic implications based on association with the esophageal neoplasms (Cancer Res 2000 60:4735-9). (PhosphoSite®, Cell Signaling Technology (Danvers, MA), Human PSD™, Biobase Corporation, (Beverly, MA)).

Z02, phosphorylated at Ser220, is among the proteins listed in this patent. ZO2 (zona occludens 2) is a SAFB binding protein involved in cell-cell adhesion and the establishment and maintenance of tight junctions. Z02 is not only located in adherens junctions on the cytoplasmic side of the plasma membrane but is also nuclear in migratory endothelial cells, epithelial cell cultures, and during environmental stress. Five alternatively-spliced isoforms have been described. Isoform A1 is abundant in the heart and brain whereas isoform C1 is expressed at high level in the kidney, pancreas, heart and placenta. In brain and skeletal muscle, only isoform A1 is detectable. Isoform C1 is found in normal as well as in most neoplastic tissues while isoform A1 is present almost exclusively in normal tissue. ZO2 is associated with familial hypercholanemia and breast and pancreatic ductal adenocarcinomas. This protein has potential diagnostic and/or therapeutic implications based on association with the following diseases: colonic neoplasms, prostatic neoplasms, breast neoplasms (Biochim Biophys Acta 2000 1493:319-24). (PhosphoSite®, Cell Signaling Technology (Danvers, MA), Human PSD™. Biobase Corporation, (Beverly, MA)).

QIK, phosphorylated at Thr484, is among the proteins listed in this patent. QIK is a serine/threonine kinase of the CAMKL family and related to AMPK. It is specifically expressed in adipose tissues and its known substrates include TORC2 and IRS 1. Like AMPK, QIK is phosphorylated and activated by LKB 1. It is part of a molecular complex including TORC2 and calcineurin that regulates the effects of circulating glucose and gut hormones during feeding on TORC2-mediated gene expression. In response to increased insulin levels, Akt2 phosphorylates and activates QIK which in turn phosphorylates TORC2. Phosphorylated TORC2 is translocated to the cytoplasm where it ubiquitinylated and degraded. QIK phosphorylates Ser794 of IRS 1 in insulin-stimulated adipocytes, potentially modulating the efficiency of insulin signal transduction. Inhibits CREB activity by phosphorylating and repressing the CREB-specific coactivators, CRTC1-3. QIK has potential diagnostic and/or therapeutic implications for cellular processes and pathologies including diabetes, insulin receptor biology, energy and lipid metabolism, cellular growth, and metabolic diseases. (PhosphoSite®, Cell Signaling Technology (Danvers, MA), Human PSD™, Biobase Corporation, (Beverly, MA)).

QSK, phosphorylated at Thr411, is among the proteins listed in this patent. QSK is a serine/threonine kinase of the CAMKL family and related to AMPK. Like AMPK, QSK is phosphorylated and activated by LKB 1. When it is phosphorylated on Thr271, it is bound and activated by 14-3-3 zeta. QSK binds to and is activated by 14-3-3 zeta when phosphorylated on Thr-163. Binding of 14-3-3 to QSK enhanced its catalytic activity towards the TORC2 protein, and was required for the localization of QSK to punctate structures within the cytoplasm. Alternative splicing produces three isoforms of human QSK. (PhosphoSite®, Cell Signaling Technology (Danvers, MA), Human PSD™, Biobase Corporation, (Beverly, MA)).

Aldolase A, phosphorylated at Ser45, is among the proteins listed in this patent. Aldolase A (fructose-bisphosphate aldolase) is a glycolytic enzyme that catalyzes the reversible conversion of fructose-1,6-bisphosphate to glyceraldehyde 3-phosphate and dihydroxyacetone phosphate. Vertebrates have 3 aldolase isozymes, which are regulated differentially during development. The developing embryo produces aldolase A, which is produced in even greater amounts in adult muscle where it can be as much as 5% of total cellular protein. In adult liver, kidney and intestine, aldolase A expression is repressed and aldolase B is produced. In brain and other nervous tissue, aldolase A and C are expressed about equally. In transformed liver cells, aldolase A replaces aldolase B (Omim #103850). Deficiencies in aldolase A manifest as hemolytic anemia and metabolic myopathy. Aldolase A has potential diagnostic and/or therapeutic implications for cellular processes and pathologies including hemolytic anemia (Biochem J 2004 380:51-6.), and myopathies (New Eng. J. Med. 334: 1100-1104, 1996.). (PhosphoSite®, Cell Signaling Technology (Danvers, MA), Human PSD™, Biobase Corporation, (Beverly, MA)).

PLAA, phosphorylated at Ser318, is among the proteins listed in this patent. PLAA (phospholipase A2 activating protein) activates phospholipase A2, which produces eicosanoids and prostaglandin E(2) in immune and inflammatory responses. PLAA is a specific activator of PLA2 in chondrocytes, and suggests that it mediates the membrane effect of 1,25-dihydroxyvitamin D3 (the active form of vitamin D). Vitamin D analogs sensitize breast cancer cells to TNFalpha and suggesting that PLA2 might be involved in vitamin D-mediated caspase-independent cell death (Mol Cell Endocrinol. 2001 172:69-78). Vitamin D causes rapid increases in protein kinase C alpha (PKCα) activity (a basophilic kinase). Many physiological responses to steroid hormones are PKC-dependent, providing an alternate method for the steroids to modulate gene expression other than by traditional steroid hormone receptor-mediated pathways (Steroids. 2004 69: 591-597). Topical administration of vitamin D enhances the suppressive capacity of CD4(+)CD25(+) cells from the draining lymph nodes (J Immunol. 2007 179:6273-83). PLAA has potential diagnostic and/or therapeutic implications for inflammatory conditions (J Biol Chem. 2001 276:5467-75), immunosuppression (J Immunol. 2007 179:6273-83), and cytoplasmic hormone signaling in breast cancer (Mol Cell Endocrinol. 2001 172:69-78). (PhosphoSite®, Cell Signaling Technology (Danvers, MA), Human PSD™, Biobase Corporation, (Beverly, MA)).

APPL2, phosphorylated at Ser508, is among the proteins listed in this patent. APPL2 is a Rab5 effector protein that resides on a subpopulation of endosomes. Required for the regulation of cell proliferation in response to extracellular signals mediated by an early endosomal compartment. APPL2 links Rab5 to nuclear signal transduction. Its function requires Rab5 binding. Translocated into the nucleus upon release from endosomal membranes following internalization of EGF. APPL2 binds to subunits of the nucleosome remodeling and deacetylase (NuRD) complex, an abundant and widely expressed deacetylase complex. The NURD complex contains both histone deacetylation and chromatin remodeling ATPase activities. Contains a PH domain and a phosphotyrosine interaction domain (PID) domain that has a structure similar to the insulin receptor substrate-1 PTB domain. APPL2 is very high similar to APPL, which is an adaptor protein that binds to AKT2 and PI3 kinase catalytic subunit p 110alpha (PIK3CA) and may recruit these proteins to the membrane. (PhosphoSite®, Cell Signaling Technology (Danvers, MA), Human PSD™, Biobase Corporation, (Beverly, MA)).

ATG6, phosphorylated at Ser90, is among the proteins listed in this patent. ATG6 (Beclin 1) is part of a lipid kinase complex and has the properties of a tumor suppressor. Recent studies suggest that it plays a central role in coordinating the cytoprotective function of autophagy and in opposing the cellular death process of apoptosis. Autophagy is a recycling process that allows cells to survive periods of nutrient limitation; however, it has a wider physiological role, participating in development and aging, and also in protection against pathogen invasion, cancer and certain neurodegenerative diseases. ATG6 is a key autophagic protein that has been used to define and investigate the process of autophagy (Cell Res. 2007 17:839-49). ATG6 interacts with Bcl-2. PKC delta, a basophilic kinase, is novel inhibitors of autophagy in pancreatic cancer cells (Autophagy. 2007 3:480-3). ATG6 inhibits tumor growth in colon cancer cell lines (Anticancer Res. 2007 27(3B): 1453-7). Mutation in the corresponding gene is associated with several cancers. This protein has potential diagnostic and/or therapeutic implications based on its association with pancreatic cancer, colon cancer, ovarian neoplasms, prostatic neoplasms, and breast neoplasms (J Clin Invest 2003 112:1809-20). (PhosphoSite®, Cell Signaling Technology (Danvers, MA), Human PSD™, Biobase Corporation, (Beverly, MA)).

eIF4B, phosphorylated at Ser 418, is among the proteins listed in this patent. eIF4B (eukaryotic translation initiation factor 4B) is a translation initiation factor that is required for the binding of mRNA to ribosomes. It forms a complex with EIF4-F and EIF4-A. eIF4B binds near the 5'-terminal cap of mRNA in the presence of EIF-4F and ATP. Promotes the ATPase activity and the ATP-dependent RNA unwinding activity of both EIF4-A and EIF4-F.eIF4B is downstream of the mTOR pathway and its level of phosphorylation was inhibited in glioblastoma cells following administration of N(1), N(11)-Diethylnorspermine (DENSPM) is a spermine analog and prototype anti-cancer drug that depletes cellular polyamine, increases cellular oxidative stress through the generation of H(2)O(2) and induces the death of multiple types of cancer cells (Cancer Biol Ther. 2007 Jul 27;6(10)). eIF4B has potential diagnostic and/or therapeutic implications for cellular processes and pathologies including glioblastoma and melanoma (Int J Cancer 1997 May 2;71(3):396-401.). (PhosphoSite®, Cell Signaling Technology (Danvers, MA), Human PSD™, Biobase Corporation, (Beverly, MA)).

Glucokinase, phosphorylated at Thr49, is among the proteins listed in this patent. Glucokinase is a glycolytic enzyme that converts glucose to glucose-6-phosphate in the first and rate-limiting step of glucose metabolism. It is critical for the glucose-sensing cell phenotype, and acts in insulin secretion and hepatic intermediary metabolism. By catalyzing the phosphorylation of glucose to glucose-6-phosphate, glucose is trapped inside the cell. Glucokinase has a lower affinity for glucose than the three other isozymes of hexokinase, allowing other organs such as the brain and muscles to have first call on glucose when its supply is limited. Unlike other hexokinases, glucokinase is not inhibited by glucose-6-phosphate. Glucokinase is found in the outer membrane compartment of mitochondria. May bind VDAC, suppressing mitochondrial function. Glucokinase transcription is induced by insulin, perhaps via the activation of Stat 5B. Mutant glucokinase causes a rare form of diabetes and may also play a role in type 2 diabetes. Three splice variant isoforms of human glucokinase have been described. Glucokinase has potential diagnostic and/or therapeutic implications for processes and pathologies including type 2 diabetes mellitus and insulin resistance (Biochem Biophys Res Commun 1996 221614-8.), and metabolic diseases of the liver. (PhosphoSite®, Cell Signaling Technology (Danvers, MA), Human PSD™, Biobase Corporation, (Beverly, MA)).

HSP70, phosphorylated at Thr265, is among the proteins listed in this patent. HSP70 (heat shock 70) kDa protein 1A, an HSP70 family chaperone that modulates stress responses. It is a critical chaperone protein that has a high affinity for unfolded polypeptide chains. It binds extended peptide segments with a net hydrophobic character exposed by polypeptides during translation and membrane translocation, or following stress-induced damage. In cooperation with other chaperones, hsp70 stabilizes preexistent proteins against aggregation and mediates the folding of newly translated polypeptides in the cytosol as well as within organelles. Mitochondrial HSP70 is crucial to the import process: mutant forms of HSP70 fail to import precursor proteins. It has an anti-apoptotic function in sympathetic neurons and mediates this effect primarily by suppressing c-Jun transcriptional signalling. Interacts with tau protein and mediates proper folding of`tau. It can promote the degradation of tau protein. Triptolide, a potential therapeutic agent for progression/metastasis of pancreatic cancer, causes pancreatic cancer cell death by inducing apoptosis, an effect mediated by the inhibition of HSP70. A genetic polymorphism of HSP70 is associated with ankylosing spondylitis, celiac disease, and rheumatoid arthritis; altered expression is associated with lung cancer and diabetes. This protein has potential diagnostic and/or therapeutic implications based on association with ovarian neoplasms (Biochem Pharmacol 1999 58:69-76). (PhosphoSite®, Cell Signaling Technology (Danvers, MA), Human PSD™, Biobase Corporation, (Beverly, MA)).

PIPKI-gamma, phosphorylated at Thr553, is among the proteins listed in this patent. PIPK I-gamma is a member of the type I phosphatidylinositol-4-phosphate 5-kinase family of enzymes. It localizes in synapses and focal adhesion plaques, and binds the FERM domain of talin through its C-terminus. PIPKI-gamma serves as both a scaffold that links E-cadherin to clathrin adaptor protein (AP) complexes and the trafficking machinery, and a regulator of trafficking events via the spatial generation of phosphatidylinositol-4,5-bisphosphate (J Cell Biol. 2007 6:343-53). It is critical to the endocytosis of synaptic vesicle proteins. The cytoskeletal protein talin binds to PIPKI-gamma, activating the enzyme and promoting the local production of phosphatidylinositol 4,5 bisphosphate, which regulates focal adhesion dynamics as well as clathrin-mediated endocytosis in neuronal cells (J Biol Chem. 2005 280:8381-6). Assembly of E-cadherin-based adherens junctions (AJ) are obligatory for establishment of polarized epithelia and plays a key role in repressing the invasiveness of many carcinomas. PIPKI-gamma directly binds to E-cadherin and modulates E-cadherin trafficking. PIPKI-gamma also interacts with the µ subunits of clathrin adaptor protein (AP) complexes and acts as a signalling scaffold that links AP complexes to E-cadherin. Depletion of PIPKI-gamma or disruption of PIPKI-gamma binding to either E-cadherin or AP complexes results in defects in E-cadherin transport and blocks AJ assembly. An E-cadherin germline mutation that loses PIPKI-gamma binding and shows disrupted basolateral membrane targeting no longer forms AJs and leads to hereditary gastric cancers. A defect PIPKI-gamma causes lethal congenital arthrogryposis (Am J Hum Genet. 2007 81:530-9). Inhibiting the activity of PIPKI-gamma can inhibit or prevent cell migration-mediated condition or disease (United States Patent 20060257848). Defects in PIPKI-gamma cause type 3 (LCCS3) (Am. J. Hum. Genet. 81: 530-539, 2007). PIPKI-gamma has potential diagnostic and/or therapeutic implications for processes and pathologies including endocytosis, lethal contractural syndromes, and gastric cancer (Journal of Cell Biology, Vol. 176, No. 3, 343-353). (PhosphoSite®, Cell Signaling Technology (Danvers, MA), Human PSD™, Biobase Corporation,

MYPT1, phosphorylated at Ser507, is among the proteins listed in this patent. MYPT1 (myosin phosphatase target subunit 1) is a regulatory subunit of protein phosphatase 1. Myosin phosphatase regulates the interaction of actin and myosin downstream of the small G protein Rho. Four splice-variant isoforms have been described. (PhosphoSite®, Cell Signaling Technology (Danvers, MA), Human PSD™, Biobase Corporation, (Beverly, MA)).

NDRG1, phosphorylated at Ser354, is among the proteins listed in this patent. NDRG1, N-myc downstream regulated gene 1, is a metastasis suppressor protein involved in growth arrest and cell differentiation. It is highly expressed in adult skeletal muscle and brain. It is induced by a variety of agents including p53, vitamin D, retinoic acid, phorbol esters, androgenic and estrogenic hormones, phosphatase and tensin homologue deleted on chromosome 10 (PTEN), nickel compounds, elevated intracellular calcium, DNA methylation and histone deacetylation inhibiting agents, DNA damage, and decreased glucose concentration. NRDG1 plays a role in cellular stress, p53-mediated apoptosis, the mitotic spindle checkpoint, and cell differentiation and proliferation. NDRG1 is upregulated by differentiation signals in various cancer cell lines, and suppresses tumor metastasis. It is strongly upregulated under hypoxic conditions, a condition that is prevalent in solid tumors. Hypoxia-inducible factor- (HIF-1α), p53, and N-Myc regulate the transcription of NDRG1. NDRG1 interacts with SIRT1/p53 signaling to attenuate hypoxic iniurv in human trophoblasts. Like the protein AS160, which is regulated by Akt in the insulin response (J Biol Chem. 2003 278:14599-602), NDRG1 is involved in Rab signaling. Rab proteins are small G proteins required for membrane trafficking. NDRG1 is a ubiquitous Rab4a effector protein that modulates angiogenesis and is involved in vesicular recycling of E-cadherin and transferrin. NDRG1 knockdown delays the recycling rate of transferrin, while its overexpression increases the rate of transferrin recycling. Interacts with SIRT1/p53 signaling to attenuate hypoxic injury in human trophoblasts. It plays a specific role in the molecular cause of Charcot-Marie-Tooth type 4D disease and is a marker of tumor progression and enhancer of cellular differentiation (Carcinogenesis. 2007 Oct 4; [Epub ahead of print]). Mutations cause hereditary motor and sensory neuropathies. NDRG 1 has potential diagnostic and/or therapeutic implications for multiple types of solid tumors (Carcinogenesis. 2007 Oct 4 [Epub ahead of print]), hepatocellular carcinoma (Mod Pathol. 2007 20:76-83), esophageal squamous cell carcinoma (Dis Esophagus. 2006 19:454-8

PDCD4, phosphorylated at Ser68, is among the proteins listed in this patent. PDCD4 (programmed cell death 4 protein) is upregulated in bladder and breast carcinoma tissues. It is localized to the nucleus in proliferating cells that seems to possess a tumor suppressor activity. It directly interacts with the RNA helicase eIF4A and inhibits protein synthesis by interfering with the assembly of the cap-dependent translation initiation complex. PDCD4 suppresses carbonic anhydrase type II protein expression in carcinoid cell lines. Since tumor cells require a high bicarbonate flux for their growth, carbonic anhydrase suppression results in growth inhibition. Expression of this gene is modulated by cytokines in natural killer and T cells. The gene product is thought to play a role in apoptosis but the specific role has not yet been determined. Two differentially spliced isoforms have been identified. PDCD4 has potential diagnostic and/or therapeutic implications for cellular processes and pathologies including bladder cancer, breast cancer and carcinoid. (Phosphosite®, Cell Signaling Technology (Danvers, MA), Human PSD™, Biobase Corporation, (Beverly, MA)).

HECTD1, phosphorylated at Ser2113, is among the proteins listed in this patent. HECTD 1 is ubiquitin-protein ligase required for development of the head mesenchyme and neural tube closure. Accepts ubiquitin from an E2 ubiquitin-conjugating enzyme in the form of a thioester and then directly transfers the ubiquitin to targeted substrates. It is a member of the Sad 1 or UNC-like C-terminal containing family, contains three ankyrin repeats, two HEAT repeats, a HECT domain, and a Mib or herc2 domain, has moderate similarity to C. elegans C34D4.14, which plays a role in the response to hypoxia(PhosphoSite®, Cell Signaling Technology (Danvers, MA), Human PSD™, Biobase Corporation, (Beverly, MA)).

PTPN14, phosphorylated at Thr670, is among the proteins listed in this patent. PTPN14 (protein tyrosine phosphatase non-receptor type 14) is a non-receptor phospho-tyrosine protein phosphatase that regulates cell motility and cell-cell adhesion. PTPN14 is mutated in a small percentage of human cancers including colorectal cancers and a smaller fraction of hang, breast, and gastric cancers. May play a role in liver metastases and tumor invasion in pancreatic cancer. Contains 1 FERM domain. PTPN14 has potential diagnostic and/or therapeutic implications for cellular processes and pathologies including colorectal, lung, breast, liver, pancreatic and gastric cancers. (PhosphoSite®, Cell Signaling Technology (Danvers, MA), Human PSD™, Biobase Corporation, (Beverly, MA)).

NUP93, phosphorylated at Thr49, is among the proteins listed in this patent. NUP93 (Nucleoporin 93) is a nuclear pore protein required for correct nuclear pore assembly. The nuclear pore complex, comprised of approximately 30 nucleoporins, mediates the exchange of macromolecules across the nuclear envelope. (PhosphoSite®, Cell Signaling Technology (Danvers, MA), Human PSD™, Biobase Corporation, (Beverly, MA)).

GBP1, phosphorylated at Thr532, is among the proteins listed in this patent. GBP1 (Guanylate binding protein 1) is an interferon-inducible G protein involved in interferon-gamma (IFNG) mediated antiviral responses and is induced in inflammatory skin diseases. GBP1 possesses a high GTP hydrolysis activity. GBP1, -2, and -3 are the most abundant cellular proteins induced in response to IFNG, tumor necrosis factor-alpha (TNF-alpha), and interleukin-1beta (IL-1beta). (PhosphoSite®, Cell Signaling Technology (Danvers, MA), Human FSD™, Biobase Corporation, (Beverly, MA)).

CHD9, phosphorylated at Ser519, is among the proteins listed in this patent. CHD9 (chromodomain helicase DNA binding protein 9) is a transcriptional coactivator for PPARA and possibly other nuclear receptors. CHD9 is proposed to be an ATP-dependent chromatin remodeling protein. Has DNA-dependent ATPase activity and binds to A/T-rich DNA. CHD9 associates with A/T-rich regulatory regions in promoters of genes that participate in the differentiation of progenitors during osteogenesis. Interacts with PPARA. Probably interacts with ESR1 and NR1I3. Alternative splicing produces three splice-variant isoforms of the human protein. (PhosphoSite®, Cell Signaling Technology (Danvers, MA), Human PSD^{™}, Biobase Corporation, (Beverly, MA)).

ATRX, phosphorylated at Ser1141, is among the proteins listed in this patent. ATRX, X-linked nuclear protein, functions in ATP-dependent chromatin remodeling in a complex with DAXX, may function in DNA repair, recombination, and mitotic segregation; alteration of gene is associated with alpha thalassemia-mental retardation syndrome. This protein has potential diagnostic and/or therapeutic implications based on association with the following diseases: X-Linked Mental Retardation (Am J Hum Genet 1996 Jun;58(6):1185-91.). (PhosphoSite®, Cell Signaling Technology (Danvers, MA), Human PSD^{™}, Biobase Corporation, (Beverly, MA)).

HSC70, phosphorylated at Thr265, is among the proteins listed in this patent. HSC70, Heat shock 70kD protein 8, constitutively expressed member of heat shock HSP70 family of molecular chaperones, marker for hypertrophic cardiomyopathy, Alzheimer disease, and rheumatoid arthritis; deletion correlates with sporadic breast carcinoma. This protein has potential diagnostic and/or therapeutic implications based on association with the following diseases: Alzheimer Disease (Biochem Biophys Res Commun 2001 Jan 12;280(1):249-58.). (PhosphoSite®, Cell Signaling Technology (Danvers, MA), Human PSD™, Biobase Corporation, (Beverly, MA)).

Tks5, phosphorylated at Ser988, is among the proteins listed in this patent. Tks5, Protein with strong similarity to SH3 multiple domains 1 (mouse Sh3md1); which binds proteins and phosphoinositide and may act in signaling by tyrosine kinases, contains five variant SH3 and five Src homology 3 (SH3) domains and a phox protein (PX) domain. (PhosphoSite®, Cell Signaling Technology (Danvers, MA), Human PSD™, Biobase Corporation, (Beverly, MA)).

The invention also provides peptides comprising a novel phosphorylation site of the invention. In one particular embodiment, the peptides comprise any one of the amino acid sequences as set forth in SEQ ID NOs: 1-142, which are trypsin-digested peptide fragments of the parent proteins. Alternatively, a parent signaling protein listed in Table 1 may be digested with another protease, and the sequence of a peptide fragment comprising a phosphorylation site can be obtained in a similar way. Suitable proteases include, but are not limited to, serine proteases *(e.g.* hepsin), metallo proteases *(e.g.* PUMP1), chymotrypsin, cathepsin, pepsin, thermolysin, carboxypeptidases, etc.

The invention also provides proteins and peptides that are mutated to eliminate a novel phosphorylation site of the invention. Such proteins and peptides are particular useful as research tools to understand complex signaling transduction pathways of insulin signaling, for example, to identify new upstream kmase(s) or phosphatase(s) or other proteins that regulate the activity of a signaling protein; to identify downstream effector molecules that interact with a signaling protein, etc.

Various methods that are well known in the art can be used to eliminate a phosphorylation site. For example, the phosphorylatable serine and/or threonine may be mutated into a non-phosphorylatable residue, such as phenylalanine. A "phosphorylatable" amino acid refers to an amino acid that is capable of being modified by addition of a phosphate group (any includes both phosphorylated form and unphosphorylated form). Alternatively, the serine and/or threonine may be deleted. Residues other than the serine and/or threonine may also be modified (e.g., delete or mutated) if such modification inhibits the phosphorylation of the serine and/or threonine residue. For example, residues flanking the serine and/or threonine may be deleted or mutated, so that a kinase cannot recognize/phosphorylate the mutated protein or the peptide. Standard mutagenesis and molecular cloning techniques can be used to create amino acid substitutions or deletions.

### 2. Modulators of the Phosphorylation Sites

In another aspect, the invention provides a modulator that modulates serine and/or threonine phosphorylation at a novel phosphorylation site of the invention, including small molecules, peptides comprising a novel phosphorylation site, and binding molecules that specifically bind at a novel phosphorylation site, including but not limited to antibodies or antigen-binding fragments thereof.

Modulators of a phosphorylation site include any molecules that directly or indirectly counteract, reduce, antagonize or inhibit serine and/or threonine phosphorylation of the site. The modulators may compete or block the binding of the phosphorylation site to its upstream kinase(s) or phosphatase(s), or to its downstream signaling transduction molecule(s).

The modulators may directly interact with a phosphorylation site. The modulator may also be a molecule that does not directly interact with a phosphorylation site. For example, the modulators can be dominant negative mutants, *i.e.,* proteins and peptides that are mutated to eliminate the phosphorylation site. Such mutated proteins or peptides could retain the binding ability to a downstream signaling molecule but lose the ability to trigger downstream signaling transduction of the wild type parent signaling protein.

The modulators include small molecules that modulate the serine and/or threonine phosphorylation at a novel phosphorylation site of the invention. Chemical agents, referred to in the art as "small molecule" compounds are typically organic, non-peptide molecules, having a molecular weight less than 10,000, less than 5,000, less than 1,000, or less than 500 daltons. This class of modulators includes chemically synthesized molecules, for instance, compounds from combinatorial chemical libraries. Synthetic compounds may be rationally designed or identified based on known or inferred properties of a phosphorylation site of the invention or may be identified by screening compound libraries. Alternative appropriate modulators of this class are natural products, particularly secondary metabolites from organisms such as plants or fungi, which can also be identified by screening compound libraries. Methods for generating and obtaining compounds are well known in the art (Schreiber SL, Science 151: 1964-1969(2000); Radmann J. and Gunther J., Science 151: 1947-1948 (2000)).

The modulators also include peptidomimetics, small protein-like chains designed to mimic peptides. Peptidomimetics may be analogues of a peptide comprising a phosphorylation site of the invention. Peptidomimetics may also be analogues of a modified peptide that are mutated to eliminate a phosphorylation site of the invention. Peptidomimetics (both peptide and non-peptidyl analogues) may have improved properties (e.g., decreased proteolysis, increased retention or increased bioavailability). Peptidomimetics generally have improved oral availability, which makes them especially suited to treatment of disorders in a human or animal.

In certain embodiments, the modulators are peptides comprising a novel phosphorylation site of the invention. In certain embodiments, the modulators are antibodies or antigen-binding fragments thereof that specifically bind at a novel phosphorylation site of the invention.

### 3. Heavy-Isotope Labeled Peptides (AQUA Peptides).

In another aspect, the invention provides peptides comprising a novel phosphorylation site of the invention. In a particular embodiment, the invention provides Heavy-Isotype Labeled Peptides (AQUA peptides) comprising a novel phosphorylation site. Such peptides are useful to generate phosphorylation site-specific antibodies for a novel phosphorylation site. Such peptides are also useful as potential diagnostic tools for screening for insulin-signaling related, or as potential therapeutic agents for treating insulin-signaling related diseases.

The peptides may be of any length, typically six to fifteen amino acids. The novel serine and/or threonine phosphorylation site can occur at any position in the peptide; if the peptide will be used as an immnogen, it preferably is from seven to twenty amino acids in length. In some embodiments, the peptide is labeled with a detectable marker.

"Heavy-isotope labeled peptide" (used interchangeably with AQUA peptide) refers to a peptide comprising at least one heavy-isotope label, as described in WO/03016861, "Absolute Quantification of Proteins and Modified Forms Thereof by Multistage Mass Spectrometry" (Gygi *et al.*) (the teachings of which are hereby incorporated herein by reference, in their entirety). The amino acid sequence of an AQUA peptide is identical to the sequence of a proteolytic fragment of the parent protein in which the novel phosphorylation site occurs. AQUA peptides of the invention are highly useful for detecting, quantitating or modulating a phosphorylation site of the invention (both in phosphorylated and unphosphorylated forms) in a biological sample.

A peptide of the invention, including an AQUA peptides comprises any novel phosphorylation site. Preferably, the peptide or AQUA peptide comprises a novel phosphorylation site of a protein in Table 1 that is an adaptor/scaffold proteins, enzyme/non-protein kinase/phoshpatase proteins, Ser/Thr (non-receptor) protein kinases, vesicle proteins, g proteins or regulator proteins, chromatin or DNA binding/repair/replication proteins, receptor/channel/transporter/cell surface proteins, RNA processing proteins, cytoskeletal proteins, transcriptional regulators and translation proteins.

Particularly preferred peptides and AQUA peptides are these comprising a novel serine and/or threonine phosphorylation site (shown as a lower case "s" or "t" (respectively) within the sequences listed in Table 1) selected from the group consisting of SEQ ID NOs: 1 (Rictor); 2 (ZO2); 3 (APPL2); 4 (ATG6);5 (Rictor); 10 (Tks5); 19 (JMJD2C); 20 (adolase A); 21 (glucokinase); 22 (PIPK I-gamma); 23 (PTPN14); 30 (DAPK2); 31 (QIK); 32 (QSK); 42 (Ndrg1); 43 (Ndrg1); 47 (GPB1); 48 (ARHGEF11); 53 (CHD9); 58 (ATRX); 70 (NUP93), 90 (elF4B); 97 (PLAA); 98 (HSP70); 99 (MYPT1); 100 (PDCD4); 101 (HECTD1); 121 (HSC70).

In some embodiments, the peptide or AQUA peptide comprises the amino acid sequence shown in any one of the above listed SEQ ID NOs. In some embodiments, the peptide or AQUA peptide consists of the amino acid sequence in said SEQ ID NOs. In some embodiments, the peptide or AQUA peptide comprises a fragment of the amino acid sequence in said SEQ ID NOs., wherein the fragment is six to twenty amino acid long and includes the phosphorylatable serine and/or threonine. In some embodiments, the peptide or AQUA peptide consists of a fragment of the amino acid sequence in said SEQ ID NOs., wherein the fragment is six to twenty amino acid long and includes the phosphorylatable serine and/or threonine.

In certain embodiments, the peptide or AQUA peptide comprises any one of SEQ ID NOs: 1-142, which are trypsin-digested peptide fragments of the parent proteins.

It is understood that parent protein listed in Table 1 may be digested with any suitable protease (e.g., serine proteases (*e.g.* trypsin, hepsin), metallo proteases (e.g. PUMP1), chymotrypsin, cathepsin, pepsin, thermolysin, carboxypeptidases, etc), and the resulting peptide sequence comprising a phosphorylated site of the invention may differ from that of trypsin-digested fragments (as set forth in Column E), depending the cleavage site of a particular enzyme. An AQUA peptide for a particular a parent protein sequence should be chosen based on the amino acid sequence of the parent protein and the particular protease for digestion; that is, the AQUA peptide should match the amino acid sequence of a proteolytic fragment of the parent protein in which the novel phosphorylation site occurs.

An AQUA peptide is preferably at least about 6 amino acids long. The preferred ranged is about 7 to 15 amino acids.

The AQUA method detects and quantifies a target protein in a sample by introducing a known quantity of at least one heavy-isotope labeled peptide standard (which has a unique signature detectable by LC-SRM chromatography) into a digested biological sample. By comparing to the peptide standard, one may readily determines the quantity of a peptide having the same sequence and protein modification(s) in the biological sample. Briefly, the AQUA methodology has two stages:(1) peptide internal standard selection and validation; method development; and (2) implementation using validated peptide internal standards to detect and quantify a target protein in a sample. The method is a powerful technique for detecting and quantifying a given peptide/protein within a complex biological mixture, such as a cell lysate, and may be used, *e.g.,* to quantify change in protein phosphorylation, as a result of drug treatment, or to quantify a protein in different biological states.

Generally, to develop a suitable internal standard, a particular peptide (or modified peptide) within a target protein sequence is chosen based on its amino acid sequence and a particular protease for digestion. The peptide is then generated by solid-phase peptide synthesis such that one residue is replaced with that same residue containing stable isotopes (¹³C, ¹⁵N). The result is a peptide that is chemically identical to its native counterpart formed by proteolysis, but is easily distinguishable by MS via a mass shift. A newly synthesized AQUA internal standard peptide is then evaluated by LC-MS/MS. This process provides qualitative information about peptide retention by reverse-phase chromatography, ionization efficiency, and fragmentation via collision-induced dissociation. Informative and abundant fragment ions for sets of native and internal standard peptides are chosen and then specifically monitored in rapid succession as a function of chromatographic retention to form a selected reaction monitoring (LC-SRM) method based on the unique profile of the peptide standard.

The second stage of the AQUA strategy is its implementation to measure the amount of a protein or the modified form of the protein from complex mixtures. Whole cell lysates are typically fractionated by SDS-PAGE gel electrophoresis, and regions of the gel consistent with protein migration are excised. This process is followed by in-gel proteolysis in the presence of the AQUA peptides and LC-SRM analysis. (*See* Gerber *et al. supra.*) AQUA peptides are spiked in to the complex peptide mixture obtained by digestion of the whole cell lysate with a proteolytic enzyme and subjected to immunoaffinity purification as described above. The retention time and fragmentation pattern of the native peptide formed by digestion (*e.g.,* trypsinization) is identical to that of the AQUA internal standard peptide determined previously; thus, LC-MS/MS analysis using an SRM experiment results in the highly specific and sensitive measurement of both internal standard and analyte directly from extremely complex peptide mixtures. Because an absolute amount of the AQUA peptide is added (e.g. 250 fmol), the ratio of the areas under the curve can be used to determine the precise expression levels of a protein or phosphorylated form of a protein in the original cell lysate. In addition, the internal standard is present during in-gel digestion as native peptides are formed, such that peptide extraction efficiency from gel pieces, absolute losses during sample handling (including vacuum centrifugation), and variability during introduction into the LC-MS system do not affect the determined ratio of native and AQUA peptide abundances.

An AQUA peptide standard may be developed for a known phosphorylation site previously identified by the IAP-LC-MS/MS method within a target protein. One AQUA peptide incorporating the phosphorylated form of the site, and a second AQUA peptide incorporating the unphosphorylated form of site may be developed. In this way, the two standards may be used to detect and quantify both the phosphorylated and unphosphorylated forms of the site in a biological sample.

Peptide internal standards may also be generated by examining the primary amino acid sequence of a protein and determining the boundaries of peptides produced by protease cleavage. Alternatively, a protein may actually be digested with a protease and a particular peptide fragment produced can then sequenced. Suitable proteases include, but are not limited to, serine proteases (e.g. trypsin, hepsin), metallo proteases (e.g. PUMP1), chymotrypsin, cathepsin, pepsin, thermolysin, carboxypeptidases, etc.

A peptide sequence within a target protein is selected according to one or more criteria to optimize the use of the peptide as an internal standard. Preferably, the size of the peptide is selected to minimize the chances that the peptide sequence will be repeated elsewhere in other non-target proteins. Thus, a peptide is preferably at least about 6 amino acids. The size of the peptide is also optimized to maximize ionization frequency. Thus, peptides longer than about 20 amino acids are not preferred. The preferred ranged is about 7 to 15 amino acids. A peptide sequence is also selected that is not likely to be chemically reactive during mass spectrometry, thus sequences comprising cysteine, tryptophan, or methionine are avoided.

A peptide sequence that is outside a phosphorylation site may be selected as internal standard to determine the quantity of all forms of the target protein. Alternatively, a peptide encompassing a phosphorylated site may be selected as internal standard to detect and quantify only the phosphorylated form of the target protein. Peptide standards for both phosphorylated form and unphosphorylated form can be used together, to determine the extent of phosphorylation in a particular sample.

The peptide is labeled using one or more labeled amino acids (*i.e.* the label is an actual part of the peptide) or less preferably, labels may be attached after synthesis according to standard methods. Preferably, the label is a mass-altering label selected based on the following considerations: The mass should be unique to shift fragment masses produced by MS analysis to regions of the spectrum with low background; the ion mass signature component is the portion of the labeling moiety that preferably exhibits a unique ion mass signature in MS analysis; the sum of the masses of the constituent atoms of the label is preferably uniquely different than the fragments of all the possible amino acids. As a result, the labeled amino acids and peptides are readily distinguished from unlabeled ones by the ion/mass pattern in the resulting mass spectrum. Preferably, the ion mass signature component imparts a mass to a protein fragment that does not match the residue mass for any of the 20 natural amino acids.

The label should be robust under the fragmentation conditions of MS and not undergo unfavorable fragmentation. Labeling chemistry should be efficient under a range of conditions, particularly denaturing conditions, and the labeled tag preferably remains soluble in the MS buffer system of choice. The label preferably does not suppress the ionization efficiency of the protein and is not chemically reactive. The label may contain a mixture of two or more isotopically distinct species to generate a unique mass spectrometric pattern at each labeled fragment position. Stable isotopes, such as ¹³C, ¹⁵N, ¹⁷O, ¹⁸O, or ³⁴S, are among preferred labels. Pairs of peptide internal standards that incorporate a different isotope label may also be prepared. Preferred amino acid residues into which a heavy isotope label may be incorporated include leucine, proline, valine, and phenylalanine.

Peptide internal standards are characterized according to their mass-to-charge (m/z) ratio, and preferably, also according to their retention time on a chromatographic column (e.g. an HPLC column). Internal standards that co-elute with unlabeled peptides of identical sequence are selected as optimal internal standards. The internal standard is then analyzed by fragmenting the peptide by any suitable means, for example by collision-induced dissociation (CID) using, e.g., argon or helium as a collision gas. The fragments are then analyzed, for example by multi-stage mass spectrometry (MSⁿ) to obtain a fragment ion spectrum, to obtain a peptide fragmentation signature. Preferably, peptide fragments have significant differences in m/z ratios to enable peaks corresponding to each fragment to be well separated, and a signature that is unique for the target peptide is obtained. If a suitable fragment signature is not obtained at the first stage, additional stages of MS are performed until a unique signature is obtained.

Fragment ions in the MS/MS and MS³ spectra are typically highly specific for the peptide of interest, and, in conjunction with LC methods, allow a highly selective means of detecting and quantifying a target peptide/protein in a complex protein mixture, such as a cell lysate, containing many thousands or tens of thousands of proteins. Any biological sample potentially containing a target protein/peptide of interest may be assayed. Crude or partially purified cell extracts are preferably used. Generally, the sample has at least 0.01 mg of protein, typically a concentration of 0.1-10 mg/mL, and may be adjusted to a desired buffer concentration and pH.

A known amount of a labeled peptide internal standard, preferably about 10 femtomoles, corresponding to a target protein to be detected/quantified is then added to a biological sample, such as a cell lysate. The spiked sample is then digested with one or more protease(s) for a suitable time period to allow digestion. A separation is then performed (e.g., by HPLC, reverse-phase HPLC, capillary electrophoresis, ion exchange chromatography, etc.) to isolate the labeled internal standard and its corresponding target peptide from other peptides in the sample. Microcapillary LC is a preferred method.

Each isolated peptide is then examined by monitoring of a selected reaction in the MS. This involves using the prior knowledge gained by the characterization of the peptide internal standard and then requiring the MS to continuously monitor a specific ion in the MS/MS or MSⁿ spectrum for both the peptide of interest and the internal standard. After elution, the area under the curve (AUC) for both peptide standard and target peptide peaks arc calculated. The ratio of the two areas provides the absolute quantification that can be normalized for the number of cells used in the analysis and the protein's molecular weight, to provide the precise number of copies of the protein per cell. Further details of the AQUA methodology are described in Gygi *et al.,* and *Gerber et al. supra.*

Accordingly, AQUA internal peptide standards (heavy-isotope labeled peptides) may be produced, as described above, for any of the 142 novel phosphorylation sites of the invention (see Table 1/Figure 2). For example, peptide standards for a given phosphorylation site (e.g., an AQUA peptide having the sequence NRRIRTLtyEPSVDFN (SEQ ID NO: 1), wherein "t" corresponds to phosphorylatable threonine 1135 of Rictor) may be produced for both the phosphorylated and unphosphorylated forms of the sequence. Such standards may be used to detect and quantify both phosphorylated form and unphosphorylated form of the parent signaling protein (e.g., Rictor) in a biological sample.

Heavy-isotope labeled equivalents of a phosphorylation site of the invention, both in phosphorylated and unphosphorylated form, can be readily synthesized and their unique MS and LC-SRM signature determined, so that the peptides are validated as AQUA peptides and ready for use in quantification.

The novel phosphorylation sites of the invention are particularly well suited for development of corresponding AQUA peptides, since the IAP method by which they were identified (see Part A above and Example 1) inherently confirmed that such peptides are in fact produced by enzymatic digestion (e.g., trypsinization) and are in fact suitably fractionated/ionized in MS/MS. Thus, heavy-isotope labeled equivalents of these peptides (both in phosphorylated and unphosphorylated form) can be readily synthesized and their unique MS and LC-SRM signature determined, so that the peptides are validated as AQUA peptides and ready for use in quantification experiments.

Accordingly, the invention provides heavy-isotope labeled peptides (AQUA peptides) that may be used for detecting, quantitating, or modulating any of the phosphorylation sites of the invention (Table 1). For example, an AQUA peptide having the sequence SMAVKTIDsTTEVIYE (SEQ ID NO: 3), wherein s (Ser 508) is phosphoserine, and wherein V = labeled valine (e.g., ¹⁴C)) is provided for the quantification of phosphorylated (or unphosphorylated) form of APPL2 (an adaptor/scaffold protein) in a biological sample.

Example 4 is provided to further illustrate the construction and use, by standard methods described above, of exemplary AQUA peptides provided by the invention. For example, AQUA peptides corresponding to both the phosphorylated and unphosphorylated forms of SEQ ID NO: 3 (a trypsin-digested fragment of APPL2, with a Ser 508 phosphorylation site) may be used to quantify the amount of phosphorylated APPL2 in a biological sample, e.g., a sample before or after treatment with a therapeutic agent.

Peptides and AQUA peptides provided by the invention will be highly useful in the further study of signal transduction anomalies underlying insulin-signaling related disease (including, among many others, cancer and diabetes) and pathways. Peptides and AQUA peptides of the invention may also be used for identifying diagnostic/bio-markers of insulin-signaling diseases (including, among many others, diabetes and cancer), identifying new potential drug targets, and/or monitoring the effects of test therapeutic agents on signaling proteins and pathways.

### 4. Phosphorylation Site-Specific Antibodies

In another aspect, the invention discloses phosphorylation site-specific binding molecules that specifically bind at a novel serine and/or threonine phosphorylation site of the invention, and that distinguish between the phosphorylated and unphosphorylated forms. In one embodiment, the binding molecule is an antibody or an antigen-binding fragment thereof. The antibody may specifically bind to an amino acid sequence comprising a phosphorylation site identified in Table 1.

In some embodiments, the antibody or antigen-binding fragment thereof specifically binds the phosphorylated site. In other embodiments, the antibody or antigen-binding fragment thereof specially binds the unphosphorylated site. An antibody or antigen-binding fragment thereof specially binds an amino acid sequence comprising a novel serine and/or threonine phosphorylation site in Table 1 when it does not significantly bind any other site in the parent protein and does not significantly bind a protein other than the parent protein. An antibody of the invention is sometimes referred to herein as a"phospho-specific" antibody.

An antibody or antigen-binding fragment thereof specially binds an antigen when the dissociation constant is ≤ 1mM, preferably ≤ 100nM, and more preferably ≤ 10nM.

In some embodiments, the antibody or antigen-binding fragment of the invention binds an amino acid sequence that comprises a novel phosphorylation site of a protein in Table 1 that is adaptor/scaffold proteins, enzyme/non-protein kinase/phoshpatase proteins, Ser/Thr (non-receptor) protein kinases, vesicle proteins, g proteins or regulator proteins, chromatin or DNA binding/repair/replication proteins, receptor/channel/transporter/cell surface proteins, RNA processing proteins, cytoskeletal proteins, transcriptional regulators and translation proteins.

In particularly preferred embodiments, an antibody or antigen-binding fragment thereof of the invention specially binds an amino acid sequence comprising a novel serine and/or threonine phosphorylation site shown as a lower case "s" or "t" (respectively) in a sequence listed in Table 1 selected from the group consisting of SEQ ID NOS: 1 (Rictor); 2 (ZO2); 3 (APPL2); 4 (ATG6);5 (Rictor); 10 (Tks5); 19 (JMJD2C); 20 (adolase A); 21 (glucokinase); 22 (PIPK I-gamma); 23 (PTPN14); 30 (DAPK2); 31 (QIK); 32 (QSK); 42 (Ndrg1); 43 (Ndrg1); 47 (GPB1); 48 (ARHGEF11); 53 (CHD9); 58 (ATRX); 70 (NUP93), 90 (e1F4B); 97 (PLAA); 98 (HSP70); 99 (MYPT1); 100 (PDCD4); 101 (HECTD1); 121 (HSC70).

In some embodiments, an antibody or antigen-binding fragment thereof of the invention specifically binds an amino acid sequence comprising any one of the above listed SEQ ID NOs. In some embodiments, an antibody or antigen-binding fragment thereof of the invention especially binds an amino acid sequence comprises a fragment of one of said SEQ ID NOs., wherein the fragment is four to twenty amino acid long and includes the phosphorylatable serine and/or threonine.

In certain embodiments, an antibody or antigen-binding fragment thereof of the invention specially binds an amino acid sequence that comprises a peptide produced by proteolysis of the parent protein with a protease wherein said peptide comprises a novel serine and/or threonine phosphorylation site of the invention. In some embodiments, the peptides are produced from trypsin digestion of the parent protein. The parent protein comprising the novel serine and/or threonine phosphorylation site can be from any species, preferably from a mammal including but not limited to non-human primates, rabbits, mice, rats, goats, cows, sheep, and guinea pigs. In some embodiments, the parent protein is a human protein and the antibody binds an epitope comprising the novel serine and/or threonine phosphorylation site shown by a lower case "s" or "t" in Column E of Table 1. Such peptides include any one of SEQ ID NOs: 1-142.

An antibody of the invention can be an intact, four immunoglobulin chain antibody comprising two heavy chains and two light chains. The heavy chain of the antibody can be of any isotype including IgM, IgG, IgE, IgG, IgA or IgD or sub-isotype including IgG1, IgG2, IgG3, IgG4, IgE1, IgE2, etc. The light chain can be a kappa light chain or a lambda light chain.

Also within the invention are antibody molecules with fewer than 4 chains, including single chain antibodies, Camelid antibodies and the like and components of the antibody, including a heavy chain or a light chain. The term "antibody" (or "antibodies") refers to all types of immunoglobulins. The term "an antigen-binding fragment of an antibody" refers to any portion of an antibody that retains specific binding of the intact antibody. An exemplary antigen-binding fragment of an antibody is the heavy chain and/or light chain CDR. or the heavy and/or light chain variable region. The term "does not bind," when appeared in context of an antibody's binding to one phospho-form (e.g., phosphorylated form) of a sequence, means that the antibody does not substantially react with the other phospho-form (e.g., non-phosphorylated form) of the same sequence. One of skill in the art will appreciate that the expression may be applicable in those instances when (1) a phospho-specific antibody either does not apparently bind to the non-phospho form of the antigen as ascertained in commonly used experimental detection systems (Western blotting, IHC, Immunofluorescence, etc.); (2) where there is some reactivity with the surrounding amino acid sequence, but that the phosphorylated residue is an immunodominant feature of the reaction. In cases such as these, there is an apparent difference in affinities for the two sequences. Dilutional analyses of such antibodies indicates that the antibodies apparent affinity for the phosphorylated form is at least 10-100 fold higher than for the non-phosphorylated form; or where (3) the phospho-specific antibody reacts no more than an appropriate control antibody would react under identical experimental conditions. A control antibody preparation might be, for instance, purified immunoglobulin from a pre-immune animal of the same species, an isotype- and species-matched monoclonal antibody. Tests using control antibodies to demonstrate specificity are recognized by one of skill in the art as appropriate and definitive.

In some embodiments an immunoglobulin chain may comprise in order from 5' to 3', a variable region and a constant region. The variable region may comprise three complementarity determining regions (CDRs), with interspersed framework (FR) regions for a structure FR1, CDR1, FR2, CDR2, FR3, CDR3 and FR4. Also within the invention are heavy or light chain variable regions, framework regions and CDRs. An antibody of the invention may comprise a heavy chain constant region that comprises some or all of a CH1 region, hinge, CH2 and CH3 region.

An antibody of the invention may have an binding affinity (K_{D}) of 1x 10⁻⁷ M or less. In other embodiments, the antibody binds with a K_{D} of 1 x10⁻⁸ M, 1 x 10⁻⁹ M, 1 x 10⁻¹⁰ M, 1 x 10⁻¹¹ M, 1 x 10⁻¹² M or less. In certain embodiments, the K_{D} is 1 pM to 500 pM, between 500 pM to 1 µM, between 1 µM to 100 nM, or between 100 mM to 10 nM.

Antibodies of the invention can be derived from any species of animal, preferably a mammal. Non-limiting exemplary natural antibodies include antibodies derived from human, chicken, goats, and rodents (e.g., rats, mice, hamsters and rabbits), including transgenic rodents genetically engineered to produce human antibodies (see, e.g., Lonberg et al., WO93/12227; U.S. Pat. No. 5,545,806; and Kucherlapati, et al., WO91/10741; U.S. Pat. No. 6,150,584, which are herein incorporated by reference in their entirety). Natural antibodies are the antibodies produced by a host animal. "Genetically altered antibodies" refer to antibodies wherein the amino acid sequence has been varied from that of a native antibody. Because of the relevance of recombinant DNA techniques to this application, one need not be confined to the sequences of amino acids found in natural antibodies; antibodies can be redesigned to obtain desired characteristics. The possible variations are many and range from the changing of just one or a few amino acids to the complete redesign of, for example, the variable or constant region. Changes in the constant region will, in general, be made in order to improve or alter characteristics, such as complement fixation, interaction with membranes and other effector functions. Changes in the variable region will be made in order to improve the antigen binding characteristics.

The antibodies of the invention include antibodies of any isotype including IgM, IgG, IgD, IgA and IgE, and any sub-isotype, including IgG1, IgG2a, IgG2b, IgG3 and IgG4, IgE1, IgE2 etc.. The light chains of the antibodies can either be kappa light chains or lambda light chains.

Antibodies disclosed in the invention may be polyclonal or monoclonal. As used herein, the term "epitope" refers to the smallest portion of a protein capable of selectively binding to the antigen binding site of an antibody.
It is well accepted by those skilled in the art that the minimal size of a protein epitope capable of selectively binding to the antigen binding site of an antibody is about five or six to seven amino acids.

Other antibodies specifically contemplated are oligoclonal antibodies. As used herein, the phrase "oligoelonal antibodies" refers to a predetermined mixture of distinct monoclonal antibodies. *See, e.g.,* PCT publication WO 95/20401; U.S. Patent Nos. 5,789,208 and 6,335,163. In one embodiment, oligoclonal antibodies consisting of a predetermined mixture of antibodies against one or more epitopes are generated in a single cell. In other embodiments, oligoclonal antibodies comprise a plurality of heavy chains capable of pairing with a common light chain to generate antibodies with multiple specificities (e.g., PCT publication WO 04/009618). Oligoclonal antibodies are particularly useful when it is desired to target multiple epitopes on a single target molecule. In view of the assays and epitopes disclosed herein, those skilled in the art can generate or select antibodies or mixtures of antibodies that are applicable for an intended purpose and desired need.

Recombinant antibodies against the phosphorylation sites identified in the invention are also included in the present application. These recombinant antibodies have the same amino acid sequence as the natural antibodies or have altered amino acid sequences of the natural antibodies in the present application. They can be made in any expression systems including both prokaryotic and eukaryotic expression systems or using phage display methods (see, e.g., Dower et al., WO91/17271 and McCafferty et al., WO92/01047; U.S. Pat. No. 5,969,108, which are herein incorporated by reference in their entirety).

Antibodies can be engineered in numerous ways. They can be made as single-chain antibodies (including small modular immunopharmaceuticals or SMIPs™), Fab and F(ab')₂ fragments, etc. Antibodies can be humanized, chimerized, deimmunized, or fully human. Numerous publications set forth the many types of antibodies and the methods of engineering such antibodies. For example, see U.S. Patent Nos. 6,355,245; 6,180,370; 5,693,762; 6,407,213; 6,548,640; 5,565,332; 5,225,539; 6,103,889; and 5,260,203.

The genetically altered antibodies should be functionally equivalent to the above-mentioned natural antibodies. In certain embodiments, modified antibodies provide improved stability or/and therapeutic efficacy. Examples of modified antibodies include those with conservative substitutions of amino acid residues, and one or more deletions or additions of amino acids that do not significantly deleteriously alter the antigen binding utility. Substitutions can range from changing or modifying one or more amino acid residues to complete redesign of a region as long as the therapeutic utility is maintained. Antibodies of this application can be modified post-translationally (e.g., acetylation, and/or phosphorylation) or can be modified synthetically (e.g., the attachment of a labeling group).

Antibodies with engineered or variant constant or Fc regions can be useful in modulating effector functions, such as, for example, antigen-dependent cytotoxicity (ADCC) and complement-dependent cytotoxicity (CDC). Such antibodies with engineered or variant constant or Fc regions may be useful in instances where a parent singling protein (Table 1) is expressed in normal tissue; variant antibodies without effector function in these instances may elicit the desired therapeutic response while not damaging normal tissue. Accordingly, certain aspects and methods of the present disclosure relate to antibodies with altered effector functions that comprise one or more amino acid substitutions, insertions, and/or deletions.

In certain embodiments, genetically altered antibodies are chimeric antibodies and humanized antibodies.

The chimeric antibody is an antibody having portions derived from different antibodies. For example, a chimeric antibody may have a variable region and a constant region derived from two different antibodies. The donor antibodies may be from different species. In certain embodiments, the variable region of a chimeric antibody is non-human, e.g., murine, and the constant region is human.

The genetically altered antibodies used in the invention include CDR grafted humanized antibodies. In one embodiment, the humanized antibody comprises heavy and/or light chain CDRs of a non-human donor immunoglobulin and heavy chain and light chain frameworks and constant regions of a human acceptor immunoglobulin. The method of making humanized antibody is disclosed in U.S. Pat. Nos: 5,530,101; 5,585,089; 5,693,761; 5,693,762; and 6,180,370 each of which is incorporated herein by reference in its entirety.

Antigen-binding fragments of the antibodies of the invention, which retain the binding specificity of the intact antibody, are also included in the invention. Examples of these antigen-binding fragments include, but are not limited to, partial or full heavy chains or light chains, variable regions, or CDR regions of any phosphorylation site-specific antibodies described herein.

In one embodiment of the application, the antibody fragments are truncated chains (truncated at the carboxyl end). In certain embodiments, these truncated chains possess one or more immunoglobulin activities (e.g., complement fixation activity). Examples of truncated chains include, but are not limited to, Fab fragments (consisting of the VL, VH, CL and CH1 domains); Fd fragments (consisting of the VH and CH1 domains); Fv fragments (consisting of VL and VH domains of a single chain of an antibody); dAb fragments (consisting of a VH domain); isolated CDR regions; (Fab')₂ fragments, bivalent fragments (comprising two Fab fragments linked by a disulphide bridge at the hinge region). The truncated chains can be produced by conventional biochemical techniques, such as enzyme cleavage, or recombinant DNA techniques, each of which is known in the art. These polypeptide fragments may be produced by proteolytic cleavage of intact antibodies by methods well known in the art, or by inserting stop codons at the desired locations in the vectors using site-directed mutagenesis, such as after CH1 to produce Fab fragments or after the hinge region to produce (Fab')₂ fragments. Single chain antibodies may be produced by joining VL- and VH-coding regions with a DNA that encodes a peptide linker connecting the VL and VH protein fragments

Papain digestion of antibodies produces two identical antigen-binding fragments, called "Fab" fragments, each with a single antigen-binding site, and a residual "Fc" fragment, whose name reflects its ability to crystallize readily. Pepsin treatment of an antibody yields an F(ab')₂ fragment that has two antigen-combining sites and is still capable of cross-linking antigen.

"Fv" usually refers to the minimum antibody fragment that contains a complete antigen-recognition and -binding site. This region consists of a dimer of one heavy- and one light-chain variable domain in tight, noncovalent association. It is in this configuration that the three CDRs of each variable domain interact to define an antigen-binding site on the surface of the V_{H}-V_{L} dimer. Collectively, the CDRs confer antigen-binding specificity to the antibody. However, even a single variable domain (or half of an Fv comprising three CDRs specific for an antigen) has the ability to recognize and bind antigen, although likely at a lower affinity than the entire binding site.

Thus, in certain embodiments, the antibodies of the application may comprise 1, 2, 3, 4, 5, 6, or more CDRs that recognize the phosphorylation sites identified in Column E of Table 1.

The Fab fragment also contains the constant domain of the light chain and the first constant domain (CH1) of the heavy chain. Fab' fragments differ from Fab fragments by the addition of a few residues at the carboxy terminus of the heavy chain CH1 domain including one or more cysteines from the antibody hinge region. Fab'-SH is the designation herein for Fab' in which the cysteine residue(s) of the constant domains bear a free thiol group. F(ab')₂ antibody fragments originally were produced as pairs of Fab' fragments that have hinge cysteines between them. Other chemical couplings of antibody fragments are also known.

"Single-chain Fv" or "scFv" antibody fragments comprise the V_{H} and V_{L} domains of an antibody, wherein these domains are present in a single polypeptide chain. In certain embodiments, the Fv polypeptide further comprises a polypeptide linker between the V_{H} and V_{L} domains that enables the scFv to form the desired structure for antigen binding. For a review of scFv see Pluckthun in The Pharmacology of Monoclonal Antibodies, vol. 113, Rosenburg and Moore, eds. (Springer-Verlag: New York, 1994), pp. 269-315.

SMIPs are a class of single-chain peptides engineered to include a target binding region and effector domain (CH2 and CH3 domains). *See,* e.g., U,S. Patent Application Publication No. 20050238646. The target binding region may be derived from the variable region or CDRs of an antibody, e.g., a phosphorylation site-specific antibody of the application. Alternatively, the target binding region is derived from a protein that binds a phosphorylation site.

Bispecific antibodies may be monoclonal, human or humanized antibodies that have binding specificities for at least two different antigens. In the present case, one of the binding specificities is for the phosphorylation site, the other one is for any other antigen, such as for example, a cell-surface protein or receptor or receptor subunit. Alternatively, a therapeutic agent may be placed on one arm. The therapeutic agent can be a drug, toxin, enzyme, DNA, radionuclide, etc.

In some embodiments, the antigen-binding fragment can be a diabody. The term "diabody" refers to small antibody fragments with two antigen-binding sites, which fragments comprise a heavy-chain variable domain (V_{H}) connected to a light-chain variable domain (V_{L}) in the same polypeptide chain (V_{H}-V_{L}). By using a linker that is too short to allow pairing between the two domains on the same chain, the domains are forced to pair with the complementary domains of another chain and create two antigen-binding sites. Diabodies are described more fully in, for example, EP 404,097; WO 93/11161; and Hollinger et al., Proc. Natl. Acad. Sci. USA, 90: 6444-6448 (1993).

Camelid antibodies refer to a unique type of antibodies that are devoid of light chain, initially discovered from animals of the camelid family. The heavy chains of these so-called heavy-chain antibodies bind their antigen by one single domain, the variable domain of the heavy immunoglobulin chain, referred to as VHH. VHHs show homology with the variable domain of heavy chains of the human VHIII family. The VHHs obtained from an immunized camel, dromedary, or llama have a number of advantages, such as effective production in microorganisms such as Saccharomyces cerevisiae.

In certain embodiments, single chain antibodies, and chimeric, humanized or primatized (CDR-grafted) antibodies, as well as chimeric or CDR-grafted single chain antibodies, comprising portions derived from different species, are also encompassed by the present disclosure as antigen-binding fragments of an antibody. The various portions of these antibodies can be joined together chemically by conventional techniques, or can be prepared as a contiguous protein using genetic engineering techniques. For example, nucleic acids encoding a chimeric or humanized chain can be expressed to produce a contiguous protein. See, e.g., U.S. Pat. Nos. 4,816,567 and 6,331,415; U.S. Pat. No. 4,816,397; European Patent No. 0,120,694; WO 86/01533; European Patent No. 0,194,276 B1; U.S. Pat. No. 5,225,539; and European Patent No. 0,239,400 B1. See also, Newnan et al., BioTechnology, 10: 1455-1460 (1992), regarding primatized antibody. See, e.g., Ladner et al., U.S. Pat. No. 4,946,778; and Bird et al., Science, 242: 423-426 (1988)), regarding single chain antibodies.

In addition, functional fragments of antibodies, including fragments of chimeric, humanized, primatized or single chain antibodies, can also be produced. Functional fragments of the subject antibodies retain at least one binding function and/or modulation function of the full-length antibody from which they are derived.

Since the immunoglobulin-related genes contain separate functional regions, each having one or more distinct biological activities, the genes of the antibody fragments may be fused to functional regions from other genes (e.g., enzymes, U.S. Pat. No. 5,004,692, which is incorporated by reference in its entirety) to produce fusion proteins or conjugates having novel properties.

Non-immunoglobulin binding polypeptides are also contemplated. For example, CDRs from an antibody disclosed herein may be inserted into a suitable non-immunoglobulin scaffold to create a non-immunoglobulin binding polypeptide. Suitable candidate scaffold structures may be derived from, for example, members of fibronectin type III and cadherin superfamilies.

Also contemplated are other equivalent non-antibody molecules, such as protein binding domains or aptamers, which bind, in a phospho-specific manner, to an amino acid sequence comprising a novel phosphorylation site of the invention. *See*, e.g., Neuberger et al., Nature 312: 604 (1984). Aptamers are oligonucleic acid or peptide molecules that bind a specific target molecule. DNA or RNA aptamers are typically short oligonucleotides, engineered through repeated rounds of selection to bind to a molecular target. Peptide aptamers typically consist of a variable peptide loop attached at both ends to a protein scaffold. This double structural constraint generally increases the binding affinity of the peptide aptamer to levels comparable to an antibody (nanomolar range).

The invention also discloses the use of the phosphorylation site-specific antibodies with immunotoxins. Conjugates that are immunotoxins including antibodies have been widely described in the art. The toxins may be coupled to the antibodies by conventional coupling techniques or immunotoxins containing protein toxin portions can be produced as fusion proteins. In certain embodiments, antibody conjugates may comprise stable linkers and may release cytotoxic agents inside cells (see U.S. Patent Nos. 6,867,007 and 6,884,869). The conjugates of the present application can be used in a corresponding way to obtain such immunotoxins. Illustrative of such immunotoxins are those described by Byers et al., Seminars Cell Biol 2:59-70 (1991) and by Fanger et al., Immunol Today 12:51-54 (1991). Exemplary immunotoxins include radiotherapeutic agents, ribosome-inactivating proteins (RIPs), chemotherapeutic agents, toxic peptides, or toxic proteins.

The phosphorylation site-specific antibodies disclosed in the invention may be used singly or in combination. The antibodies may also be used in an array format for high throughput uses. An antibody microarray is a collection of immobolized antibodies, typically spotted and fixed on a solid surface (such as glass, plastic and silicon chip).

In another aspect, the antibodies of the invention modulate at least one, or all, biological activities of a parent protein identified in Column A of Table 1. The biological activities of a parent protein identified in Column A of Table 1 include: 1) ligand binding activities (for instance, these neutralizing antibodies may be capable of competing with or completely blocking the binding of a parent signaling protein to at least one, or all, of its ligands; 2) signaling transduction activities, such as receptor dimerization, or serine and/or threonine phosphorylation; and 3) cellular responses induced by a parent signaling protein, such as oncogenic activities (e.g., cancer cell proliferation mediated by a parent signaling protein), and/or angiogenic activities.

In certain embodiments, the antibodies of the invention may have at least one activity selected from the group consisting of: 1) stimulating metabolic processes in cellular responses to insulin 2) mimicking the cellular responses to insulin, 3) providing co-stimulatory signals that are capable of reversing or relieving insulin hypo-responsiveness 4) regulating cellular responses to insulin 5) discovering markers for normal and abnormal insulin responsiveness 6) acting as a diagnostic marker.

In certain embodiments, the phosphorylation site specific antibodies disclosed in the invention are especially indicated for diagnostic and therapeutic applications as described herein. Accordingly, the antibodies may be used in therapies, including combination therapies, in the diagnosis and prognosis of disease, as well as in the monitoring of disease progression. The invention, thus, further includes compositions comprising one or more embodiments of an antibody or an antigen binding portion of the invention as described herein. The composition may further comprise a pharmaceutically acceptable carrier. The composition may comprise two or more antibodies or antigen-binding portions, each with specificity for a different novel serine and/or threonine phosphorylation site of the invention or two or more different antibodies or antigen-binding portions all of which are specific for the same novel serine and/or threonine phosphorylation site of the invention. A composition of the invention may comprise one or more antibodies or antigen-binding portions of the invention and one or more additional reagents, diagnostic agents or therapeutic agents.

The present application provides for the polynucleotide molecules encoding the antibodies and antibody fragments and their analogs described herein. Because of the degeneracy of the genetic code, a variety of nucleic acid sequences encode each antibody amino acid sequence. The desired nucleic acid sequences can be produced by de novo solid-phase DNA synthesis or by PCR mutagenesis of an earlier prepared variant of the desired polynucleotide. In one embodiment, the codons that are used comprise those that are typical for human or mouse (see, e.g., Nakamura, Y., Nucleic Acids Res. 28: 292 (2000)).

The invention also provides immortalized cell lines that produce an antibody of the invention. For example, hybridoma clones, constructed as described above, that produce monoclonal antibodies to the targeted signaling protein phosphorylation sties disclosed herein are also provided. Similarly, the invention includes recombinant cells producing an antibody of the invention, which cells may be constructed by well known techniques; for example the antigen combining site of the monoclonal antibody can be cloned by PCR and single-chain antibodies produced as phage-displayed recombinant antibodies or soluble antibodies in E. *coli* (*see,* e.g., ANTIBODY ENGINEERING PROTOCOLS, 1995, Humana Press, Sudhir Paul editor.)

### 5. Methods of Making Phosphorylation site-Specific Antibodies

In another aspect, the invention provides a method for making phosphorylation site-specific antibodies.

Polyclonal antibodies of the invention may be produced according to standard techniques by immunizing a suitable animal (e.g., rabbit, goat, etc.) with an antigen comprising a novel serine and/or threonine phosphorylation site of the invention. (*i.e.* a phosphorylation site shown in Table 1) in either the phosphorylated or unphosphorylated state, depending upon the desired specificity of the antibody, collecting immune serum from the animal, and separating the polyclonal antibodies from the immune serum, in accordance with known procedures and screening and isolating a polyclonal antibody specific for the novel serine and/or threonine phosphorylation site of interest as further described below. Methods for immunizing non-human animals such as mice, rats, sheep, goats, pigs, cattle and horses are well known in the art. See, e.g., Harlow and Lane, Antibodies: A Laboratory Manual, New York: Cold Spring Harbor Press, 1990.

The immunogen may be the full length protein or a peptide comprising the novel serine and/or threonine phosphorylation site of interest. In some embodiments the immunogen is a peptide of from 7 to 20 amino acids in length, preferably about 8 to 17 amino acids in length. In some embodiments, the peptide antigen desirably will comprise about 3 to 8 amino acids on each side of the phosphorylatable serine and/or threonine. In yet other embodiments, the peptide antigen desirably will comprise four or more amino acids flanking each side of the phosphorylatable amino acid and encompassing it. Peptide antigens suitable for producing antibodies of the invention may be designed, constructed and employed in accordance with well-known techniques. *See,* e.g., Antibodies: A Laboratory Manual, Chapter 5, p. 75-76, Harlow & Lane Eds., Cold Spring Harbor Laboratory (1988); Czemik, Methods In Enzymology, 201: 264-283 (1991); Merrifield, J. Am. Chem. Soc. 85: 21-49 (1962)).

Suitable peptide antigens may comprise all or partial sequence of a trypsin-digested fragment as set forth in Column E of Table 1/Figure 2. Suitable peptide antigens may also comprise all or partial sequence of a peptide fragment produced by another protease digestion.

Preferred immunogens are those that comprise a novel phosphorylation site of a protein in Table 1 that is an adaptor/scaffold proteins, enzyme/non-protein kinase/phoshpatase proteins, Ser/Thr (non-receptor) protein kinases, vesicle proteins, g proteins or regulator proteins, chromatin or DNA binding/repair/replication proteins, receptor/channel/transporter/cell surface proteins, RNA processing proteins, cytoskeletal proteins, transcriptional regulators and translation proteins. In some embodiments, the peptide immunogen is an AQUA peptide, for example, any one of SEQ ID NOS: 1-142.

Particularly preferred immunogens are peptides comprising any one of the novel serine and/or threonine phosphorylation site shown as a lower case "s" or "t" the sequences listed in Table 1 selected from the group consisting of SEQ ID NOS: 1 (Rictor); 2 (ZO2); 3 (APPL2); 4 (ATG6);5 (Rictor); 10 (Tks5); 19 (JMJD2C); 20 (adolase A); 21 (glucokinase); 22 (PIPK I-gamma); 23 (PTPN14); 30 (DAPK2); 31 (QIK); 32 (QSK); 42 (Ndrg1); 43 (Ndrg1); 47 (GPB1); 48 (ARHGEF11); 53 (CHD9); 58 (ATRX); 70 (NUP93), 90 (eIF4B); 97 (PLAA); 98 (HSP70); 99(MYPT1); 100 (PDCD4); 101 (HECTD1); 121 (HSC70).

In some embodiments the immunogen is administered with an adjuvant. Suitable adjuvants will be well known to those of skill in the art. Exemplary adjuvants include complete or incomplete Freund's adjuvant, RIBI (muramyl dipeptides) or ISCOM (immunostimulating complexes).

For example, a peptide antigen comprising the novel adaptor/scaffold protein phosphorylation site in SEQ ID NO: 10 shown by the lower case "s" in Table 1 may be used to produce antibodies that specifically bind the novel serine phosphorylation site.

When the above-described methods are used for producing polyclonal antibodies, following immunization, the polyclonal antibodies which secreted into the bloodstream can be recovered using known techniques. Purified forms of these antibodies can, of course, be readily prepared by standard purification techniques, such as for example, affinity chromatography with Protein A, anti-immunoglobulin, or the antigen itself. In any case, in order to monitor the success of immunization, the antibody levels with respect to the antigen in serum will be monitored using standard techniques such as ELISA, RIA and the like.

Monoclonal antibodies of the invention may be produced by any of a number of means that are well-known in the art. In some embodiments, antibody-producing B cells are isolated from an animal immunized with a peptide antigen as described above. The B cells may be from the spleen, lymph nodes or peripheral blood. Individual B cells are isolated and screened as described below to identify cells producing an antibody specific for the novel serine and/or threonine phosphorylation site of interest. Identified cells are then cultured to produce a monoclonal antibody of the invention.

Alternatively, a monoclonal phosphorylation site-specific antibody of the invention may be produced using standard hybridoma technology, in a hybridoma cell line according to the well-known technique of Kohler and Milstein. See Nature 265: 495-97 (1975); Kohler and Milstein, Eur, J. Immunol 6: 511 (1976); *see also,* Current Protocols in Molecular Biology, Ausubel et *al.* Eds. (1989). Monoclonal antibodies so produced are highly specific, and improve the selectivity and specificity of diagnostic assay methods provided by the invention. For example, a solution containing the appropriate antigen may be injected into a mouse or other species and, after a sufficient time (in keeping with conventional techniques), the animal is sacrificed and spleen cells obtained. The spleen cells are then immortalized by any of a number of standard means. Methods of immortalizing cells include, but are not limited to, transfecting them with oncogenes, infecting them with an oncogenic virus and cultivating them under conditions that select for immortalized cells, subjecting them to carcinogenic or mutating compounds, fusing them with an immortalized cell, e.g., a myeloma cell, and inactivating a tumor suppressor gene. See, e.g., Harlow and Lane, supra. If fusion with myeloma cells is used, the myeloma cells preferably do not secrete immunoglobulin polypeptides (a non-secretory cell line). Typically the antibody producing cell and the immortalized cell (such as but not limited to myeloma cells) with which it is fused are from the same species. Rabbit fusion hybridomas, for example, may be produced as described in U.S Patent No. 5,675,063, C. Knight, Issued October 7, 1997. The immortalized antibody producing cells, such as hybridoma cells, are then grown in a suitable selection media, such as hypoxanthine-aminopterin-thymidine (HAT), and the supernatant screened for monoclonal antibodies having the desired specificity, as described below. The secreted antibody may be recovered from tissue culture supernatant by conventional methods such as precipitation, ion exchange or affinity chromatography, or the like.

The invention also encompasses antibody-producing cells and cell lines, such as hybridomas, as described above.

Polyclonal or monoclonal antibodies may also be obtained through in vitro immunization. For example, phage display techniques can be used to provide libraries containing a repertoire of antibodies with varying affinities for a particular antigen. Techniques for the identification of high affinity human antibodies from such libraries are described by Griffiths et al., (1994) EMBO J., 13:3245-3260 ; Nissim et al., ibid, pp. 692-698 and by Griffiths et al., ibid, 12:725-734, which are incorporated by reference.

The antibodies may be produced recombinantly using methods well known in the art for example, according to the methods disclosed in U.S. Pat. No. 4,349,893 (Reading) or U.S. Pat. No. 4,816,567 (Cabilly *et al.*) The antibodies may also be chemically constructed by specific antibodies made according to the method disclosed in U.S. Pat. No. 4,676,980 (Segel *et al.*)

Once a desired phosphorylation site-specific antibody is identified, polynucleotides encoding the antibody, such as heavy, light chains or both (or single chains in the case of a single chain antibody) or portions thereof such as those encoding the variable region, may be cloned and isolated from antibody-producing cells using means that are well known in the art. For example, the antigen combining site of the monoclonal antibody can be cloned by PCR and single-chain antibodies produced as phage-displayed recombinant antibodies or soluble antibodies in *E. coli (see,* e.g., Antibody Engineering Protocols, 1995, Humana Press, Sudhir Paul editor.)

Accordingly, in a further aspect, the invention provides such nucleic acids encoding the heavy chain, the light chain, a variable region, a framework region or a CDR of an antibody of the invention. In some embodiments, the nucleic acids are operably linked to expression control sequences. The invention, thus, also provides vectors and expression control sequences useful for the recombinant expression of an antibody or antigen-binding portion thereof of the invention. Those of skill in the art will be able to choose vectors and expression systems that are suitable for the host cell in which the antibody or antigen-binding portion is to be expressed.

Monoclonal antibodies of the invention may be produced recombinantly by expressing the encoding nucleic acids in a suitable host cell under suitable conditions. Accordingly, the invention further provides host cells comprising the nucleic acids and vectors described above.

Monoclonal Fab fragments may also be produced in *Escherichia coli* by recombinant techniques known to those skilled in the art. *See,* e.g., W. Huse, Science 246: 1275-81 (1989); Mullinax et al., Proc, Nat'l Acad. Sci. 87: 8095 (1990)_{.}

If monoclonal antibodies of a single desired isotype are preferred for a particular application, particular isotypes can be prepared directly, by selecting from the initial fusion, or prepared secondarily, from a parental hybridoma secreting a monoclonal antibody of different isotype by using the sib selection technique to isolate class-switch variants (Steplewski, et al., Proc. Nat'l. Acad. Sci., 82: 8653 (1985); Spira et al., J. Immunol. Methods, 74: 307 (1984)). Alternatively, the isotype of a monoclonal antibody with desirable propertied can be changed using antibody engineering techniques that are well-known in the art.

Phosphorylation site-specific antibodies of the invention, whether polyclonal or monoclonal, may be screened for epitope and phospho-specificity according to standard techniques. *See,* e.g., Czernik et al., Methods in Enzymology, 201: 264-283 (1991). For example, the antibodies may be screened against the phosphorylated and/or unphosphosphorylated peptide library by ELISA to ensure specificity for both the desired antigen (*i.e.* that epitope including a phosphorylation site of the invention and for reactivity only with the phosphorylated (or unphosphorylated) form of the antigen. Peptide competition assays may be carried out to confirm lack of reactivity with other phospho-epitopes on the parent protein. The antibodies may also be tested by Western blotting against cell preparations containing the parent signaling protein, e.g., cell lines over-expressing the parent protein, to confirm reactivity with the desired phosphorylated epitope/target.

Specificity against the desired phosphorylated epitope may also be examined by constructing mutants lacking phosphorylatable residues at positions outside the desired epitope that are known to be phosphorylated, or by mutating the desired phospho-epitope and confirming lack of reactivity. Phosphorylation site-specific antibodies of the invention may exhibit some limited cross-reactivity to related epitopes in non-target proteins. This is not unexpected as most antibodies exhibit some degree of cross-reactivity, and antipeptide antibodies will often cross-react with epitopes having high homology to the immunizing peptide. *See,* e.g., *Czernik, supra.* Cross-reactivity with non-target proteins is readily characterized by Western blotting alongside markers of known molecular weight. Amino acid sequences of cross-reacting proteins may be examined to identify phosphorylation sites with flanking sequences that are highly homologous to that of a phosphorylation site of the invention.

In certain cases, polyclonal antisera may exhibit some undesirable general cross-reactivity to phosphoserine and/or threonine itself, which may be removed by further purification of antisera, e.g., over a phosphotyramine column. Antibodies of the invention specifically bind their target protein (i.e. a protein listed in Column A of Table 1) only when phosphorylated (or only when not phosphorylated, as the case may be) at the site disclosed in corresponding Columns D/E, and do not (substantially) bind to the other form (as compared to the form for which the antibody is specific).

Antibodies may be further characterized via immunohistochemical (IHC) staining using normal and diseased tissues to examine phosphorylation and activation state and level of a phosphorylation site in diseased tissue. IHC may be carried out according to well-known techniques. *See,* e.g., Antibodies: A Laboratory Manual, Chapter 10, Harlow & Lane Eds., Cold Spring Harbor Laboratory (1988). Briefly, paraffin-embedded tissue (e.g., tumor tissue) is prepared for immunohistochemical staining by deparaffinizing tissue sections with xylene followed by ethanol; hydrating in water then PBS; unmasking antigen by heating slide in sodium citrate buffer; incubating sections in hydrogen peroxide; blocking in blocking solution; incubating slide in primary antibody and secondary antibody; and finally detecting using ABC avidin/biotin method according to manufacturer's instructions.

Antibodies may be further characterized by flow cytometry carried out according to standard methods. *See* Chow et al., Cytometry (Communications in Clinical Cytometry) 46:72-78 (2001). Briefly and by way of example, the following protocol for cytometric analysis may be employed: samples may be centrifuged on Ficoll gradients to remove lysed erythrocytes and cell debris. Adherring cells may be scrapped off plates and washed with PBS. Cells may then be fixed with 2% paraformaldehyde for 10 minutes at 37 °C followed by permeabilization in 90% methanol for 30 minutes on ice. Cells may then be stained with the primary phosphorylation site-specific antibody of the invention (which detects a parent signaling protein enumerated in Table 1), washed and labeled with a fluorescent-labeled secondary antibody. Additional fluorochrome-conjugated marker antibodies (e.g., CD45, CD34) may also be added at this time to aid in the subsequent identification of specific hematopoietic cell types. The cells would then be analyzed on a flow cytometer (e.g. a Beckman Coulter FC500) according to the specific protocols of the instrument used.

Antibodies of the invention may also be advantageously conjugated to fluorescent dyes (e.g. Alexa488, PE) for use in multi-parametric analyses along with other signal transduction (phospho-CrkL, phospho-Erk 1/2) and/or cell marker (CD34) antibodies.

Phosphorylation site-specific antibodies of the invention may specifically bind to a signaling protein or polypeptide listed in Table 1 only when phosphorylated at the specified serine and/or threonine residue, but are not limited only to binding to the listed signaling proteins of human species, *per se.* The invention includes antibodies that also bind conserved and highly homologous or identical phosphorylation sites in respective signaling proteins from other species (e.g., mouse, rat, monkey, yeast), in addition to binding the phosphorylation site of the human homologue. The term "homologous" refers to two or more sequences or subsequences that have at least about 85%, at least 90%, at least 95%, or higher nucleotide or amino acid residue identity, when compared and aligned for maximum correspondence, as measured using sequence comparison method (e.g., BLAST) and/or by visual inspection. Highly homologous or identical sites conserved in other species can readily be identified by standard sequence comparisons (such as BLAST).

Methods for making bispecific antibodies are within the purview of those skilled in the art. Traditionally, the recombinant production of bispecific antibodies is based on the co-expression of two immunoglobulin heavy-chain/light-chain pairs, where the two heavy chains have different specificities (Milstein and Cuello, Nature, 305:537-539 (1983)). Antibody variable domains with the desired binding specificities (antibody-antigen combining sites) can be fused to immunoglobulin constant domain sequences. In certain embodiments, the fusion is with an immunoglobulin heavy-chain constant domain, including at least part of the hinge, CH2, and CH3 regions. DNAs encoding the immunoglobulin heavy-chain fusions and, if desired, the immunoglobulin light chain, are inserted into separate expression vectors, and are co-transfected into a suitable host organism. For further details of illustrative currently known methods for generating bispecific antibodies see, for example, Suresh et al., Methods in Enzymology, 121:210 (1986); WO 96/27011; Brennan et al., Science 229:81 (1985); Shalaby et al., J. Exp. Med. 175:217-225 (1992); Kostelny et al., J. Immunol. 148(5): 147-1553 (1992); Hollinger et al., Proc. Natl. Acad. Sci. USA 90:6444-6448 (1993); Gruber et al., J. Immunol. 152:5368 (1994); and Tutt et al., J. Immunol. 147:60 (1991). Bispecific antibodies also include cross-linked or heteroconjugate antibodies. Heteroconjugate antibodies may be made using any convenient cross-linking methods. Suitable cross-linking agents are well known in the art, and are disclosed in U.S. Pat. No. 4,676,980, along with a number of cross-linking techniques.

Various techniques for making and isolating bispecific antibody fragments directly from recombinant cell culture have also been described. For example, bispecific antibodies have been produced using leucine zippers. Kostelny et al., J. Immunol., 148(5):1547-1553 (1992). The leucine zipper peptides from the Fos and Jun proteins may be linked to the Fab' portions of two different antibodies by gene fusion. The antibody homodimers may be reduced at the hinge region to form monomers and then re-oxidized to form the antibody heterodimers. This method can also be utilized for the production of antibody homodimers. A strategy for making bispecific antibody fragments by the use of single-chain Fv (scFv) dimers has also been reported. See Gruber et al., J. Immunol., 152:5368 (1994). Alternatively, the antibodies can be "linear antibodies" as described in Zapata et al. Protein Eng. 8(10):1057-1062 (1995). Briefly, these antibodies comprise a pair of tandem Fd segments (V_{H} -C_{H}1-C_{H}-C_{H}1) which form a pair of antigen binding regions. Linear antibodies can be bispecific or monospecific.

To produce the chimeric antibodies, the portions derived from two different species (e.g., human constant region and murine variable or binding region) can be joined together chemically by conventional techniques or can be prepared as single contiguous proteins using genetic engineering techniques. The DNA molecules encoding the proteins of both the light chain and heavy chain portions of the chimeric antibody can be expressed as contiguous proteins. The method of making chimeric antibodies is disclosed in U.S. Pat. No. 5,677,427; U.S. Pat. No. 6,120,767; and U.S. Pat. No. 6,329,508, each of which is incorporated by reference in its entirety.

Fully human antibodies may be produced by a variety of techniques. One example is trioma methodology. The basic approach and an exemplary cell fusion partner, SPAZ-4, for use in this approach have been described by Oestberg et al., Hybridoma 2:361-367 (1983); Oestberg, U.S. Pat. No. 4,634,664; and Engleman et al., U.S. Pat. No. 4,634,666 (each of which is incorporated by reference in its entirety);

Human antibodies can also be produced from non-human transgenic animals having transgenes encoding at least a segment of the human immunoglobulin locus. The production and properties of animals having these properties are described in detail by, see, e.g., Lonberg et al., WO93/12227; U.S. Pat. No. 5,545,806; and Kucherlapati, et al., WO91/10741; U.S. Pat. No. 6,150,584, which are herein incorporated by reference in their entirety.

Various recombinant antibody library technologies may also be utilized to produce fully human antibodies. For example, one approach is to screen a DNA library from human B cells according to the general protocol outlined by Huse et al., Science 246:1275-1281 (1989). The protocol described by Huse is rendered more efficient in combination with phage-display technology. See, e.g., Dower et al., WO 91/1727 and McCafferty et al., WO 92/01047; U.S. Pat. No. 5,969,108, (each of which is incorporated by reference in its entrety).

Eukaryotic ribosome can also be used as means to display a library of antibodies and isolate the binding human antibodies by screening against the target antigen, as described in Coia G, et al., J. Immunol. Methods 1: 254 (1-2):191-7 (2001); Hanes J. et al., Nat. Biotechnol. 18(12):1287-92 (2000); Proc. Natl. Acad. Sci. U. S. A. 95(24):14130-5 (1998); Proc. Natl. Acad. Sci. U. S. A. 94(10):4937-42 (1997), each which is incorporated by reference in its entirety.

The yeast system is also suitable for screening mammalian cell-surface or secreted proteins, such as antibodies. Antibody libraries may be displayed on the surface of yeast cells for the purpose of obtaining the human antibodies against a target antigen. This approach is described by Yeung, et al., Biotechnol. Prog. 18(2):212-20 (2002); Boeder, E. T., et al., Nat. Biotechnol. 15(6):553-7 (1997), each of which is herein incorporated by reference in its entirety. Alternatively, human antibody libraries may be expressed intracellularly and screened via the yeast two-hybrid system (WO0200729A2, which is incorporated by reference in its entirety).

Recombinant DNA techniques can be used to produce the recombinant phosphorylation site-specific antibodies described herein, as well as the chimeric or humanized phosphorylation site-specific antibodies, or any other genetically-altered antibodies and the fragments or conjugate thereof in any expression systems including both prokaryotic and eukaryotic expression systems, such as bacteria, yeast, insect cells, plant cells, mammalian cells (for example, NS0 cells).

Once produced, the whole antibodies, their dimers, individual light and heavy chains, or other immunoglobulin forms of the present application can be purified according to standard procedures of the art, including ammonium sulfate precipitation, affinity columns, column chromatography, gel electrophoresis and the like (see, generally, Scopes, R., Protein Purification (Springer-Verlag, N.Y., 1982)). Once purified, partially or to homogeneity as desired, the polypeptides may then be used therapeutically (including extracorporeally) or in developing and performing assay procedures, immunofluorescent staining, and the like. (See, generally, Immunological Methods, Vols. I and II (Lefkovits and Pernis, eds., Academic Press, NY, 1979 and 1981).

### 6. Therapeutic Uses

In a further aspect, the invention provides methods and compositions for therapeutic uses of the peptides or proteins comprising a phosphorylation site of the invention, and phosphorylation site-specific antibodies of the invention.

In one embodiment, the invention provides for a method of treating or preventing carcinoma in a subject, wherein the carcinoma is associated with the phosphorylation state of a novel phosphorylation site in Table 1, whether phosphorylated or dephosphorylated, comprising: administering to a subject in need thereof a therapeutically effective amount of a peptide comprising a novel phosphorylation site (Table 1) and/or an antibody or antigen-binding fragment thereof that specifically bind a novel phosphorylation site of the invention (Table 1). The antibodies maybe full-length antibodies, genetically engineered antibodies, antibody fragments, and antibody conjugates of the invention.

The term "subject" refers to a vertebrate, such as for example, a mammal, or a human. Although present application are primarily concerned with the treatment of human subjects, the disclosed methods may also be used for the treatment of other mammalian subjects such as dogs and cats for veterinary purposes.

In one aspect, the disclosure provides a method of treating insulin-signaling related disease (including, among many others, diabetes and cancer) in which a peptide or an antibody that reduces at least one biological activity of a targeted signaling protein is administered to a subject. For example, the peptide or the antibody administered may disrupt or modulate the interaction of the target signaling protein with its ligand. Alternatively, the peptide or the antibody may interfere with, thereby reducing, the down-stream signal transduction of the parent signaling protein. An antibody that specifically binds the novel serine and/or threonine phosphorylation site only when the serine and/or threonine is phosphorylated, and that does not substantially bind to the same sequence when the serine and/or threonine is not phosphorylated, thereby prevents downstream signal transduction triggered by a phospho-serine and/or threonine. Alternatively, an antibody that specifically binds the unphosphorylated target phosphorylation site reduces the phosphorylation at that site and thus reduces activation of the protein mediated by phosphorylation of that site. Similarly, an unphosphorylated peptide may compete with an endogenous phosphorylation site for the same target (e.g., kinases), thereby preventing or reducing the phosphorylation of the endogenous target protein. Alternatively, a peptide comprising a phosphorylated novel serine and/or threonine site of the invention but lacking the ability to trigger signal transduction may competitively inhibit interaction of the endogenous protein with the same down-stream ligand(s).

The antibodies of the invention may also be used to target cells for effector-mediated cell death. The antibody disclosed herein may be administered as a fusion molecule that includes a phosphorylation site-targeting portion joined to a cytotoxic moiety to directly kill cells. Alternatively, the antibody may directly kill the cells through complement-mediated or antibody-dependent cellular cytotoxicity.

Accordingly in one embodiment, the antibodies of the present disclosure may be used to deliver a variety of cytotoxic compounds. Any cytotoxic compound can be fused to the present antibodies. The fusion can be achieved chemically or genetically (e.g., via expression as a single, fused molecule). The cytotoxic compound can be a biological, such as a polypeptide, or a small molecule. As those skilled in the art will appreciate, for small molecules, chemical fusion is used, while for biological compounds, either chemical or genetic fusion can be used.

Non-limiting examples of cytotoxic compounds include therapeutic drugs, radiotherapeutic agents, ribosome-inactivating proteins (RIPs), chemotherapeutic agents, toxic peptides, toxic proteins, and mixtures thereof. The cytotoxic drugs can be intracellularly acting cytotoxic drugs, such as short-range radiation emitters, including, for example, short-range, high-energy α-emitters. Enzymatically active toxins and fragments thereof, including ribosome-inactivating proteins, are exemplified by saporin, luffin, momordins, ricin, trichosanthin, gelonin, abrin, etc. Procedures for preparing enzymatically active polypeptides of the immunotoxins are described in WO84/03508 and WO85/03508, which are hereby incorporated by reference. Certain cytotoxic moieties are derived from adriamycin, chlorambucil, daunomycin, methotrexate, neocarzinostatin, and platinum, for example.

Exemplary chemotherapeutic agents that may be attached to an antibody or antigen-binding fragment thereof include taxol, doxorubicin, verapamil, podophyllotoxin, procarbazine, mechlorethamine, cyclophosphamide, camptothecin, ifosfamide, melphalan, chlorambucil, bisulfan, nitrosurea, dactinomycin, daunorubicin, doxorubicin, bleomycin, plicomycin, mitomycin, etoposide (VP16), tamoxifen, transplatinum, 5-fluorouracil, vincristin, vinblastin, or methotrexate.

Procedures for conjugating the antibodies with the cytotoxic agents have been previously described and are within the purview of one skilled in the art.

Alternatively, the antibody can be coupled to high energy radiation emitters, for example, a radioisotope, such as ¹³¹I, a γ-emitter, which, when localized at the tumor site, results in a killing of several cell diameters. See, e.g., S. E. Order, "Analysis, Results, and Future Prospective of the Therapeutic Use of Radiolabeled Antibody in Cancer Therapy", Monoclonal Antibodies for Cancer Detection and Therapy, Baldwin et al. (eds.), pp. 303-316 (Academic Press 1985), which is hereby incorporated by reference. Other suitable radioisotopes include α-emitters, such as ²¹²Bi , ²¹³Bi, and ²¹¹At, and β-emitters, such as ¹⁸⁶Re and ⁹⁰Y.

Because many of the signaling proteins in which novel serine and/or threonine phosphorylation sites of the invention occur also are expressed in normal cells and tissues, it may also be advantageous to administer a phosphorylation site-specific antibody with a constant region modified to reduce or eliminate ADCC or CDC to limit damage to normal cells. For example, effector function of an antibodies may be reduced or eliminated by utilizing an IgG1 constant domain instead of an IgG2/4 fusion domain. Other ways of eliminating effector function can be envisioned such as, e.g., mutation of the sites known to interact with FcR or insertion of a peptide in the hinge region, thereby eliminating critical sites required for FcR interaction. Variant antibodies with reduced or no effector function also include variants as described previously herein.

The peptides and antibodies of the invention may be used in combination with other therapies or with other agents. Other agents include but are not limited to polypeptides, small molecules, chemicals, metals, organometallic compounds, inorganic compounds, nucleic acid molecules, oligonucleotides, aptamers, spiegelmers, antisense nucleic acids, locked nucleic acid (LNA) inhibitors, peptide nucleic acid (PNA) inhibitors, immunomodulatory agents, antigen-binding fragments, prodrugs, and peptidomimetic compounds. In certain embodiments, the antibodies and peptides of the invention may be used in combination with cancer therapies known to one of skill in the art.

In certain aspects, the present disclosure relates to combination treatments comprising a phosphorylation site-specific antibody described herein and immunomodulatory compounds, vaccines or chemotherapy. Illustrative examples of suitable immunomodulatory agents that may be used in such combination therapies include agents that block negative regulation of T cells or antigen presenting cells (e.g., anti-CTLA4 antibodies, anti-PD-L1 antibodies, anti-PDL-2 antibodies, anti-PD-1 antibodies and the like) or agents that enhance positive co-stimulation of T cells (e.g., anti-CD40 antibodies or anti 4-1BB antibodies) or agents that increase NK cell number or T-cell activity (e.g., inhibitors such as IMiDs, thalidomide, or thalidomide analogs). Furthermore, immunomodulatory therapy could include cancer vaccines such as dendritic cells loaded with tumor cells, proteins, peptides, RNA, or DNA derived from such cells, patient derived heat-shock proteins (hsp's) or general adjuvants stimulating the immune system at various levels such as CpG, Luivac®, Biostim®, Ribomunyl®, Imudon®, Bronchovaxom® or any other compound or other adjuvant activating receptors of the innate immune system (e.g., toll like receptor agonist, anti-CTLA-4 antibodies, etc.). Also, immunomodulatory therapy could include treatment with cytokines such as IL-2, GM-CSF and IFN-gamma.

Furthermore, combination of antibody therapy with chemotherapeutics could be particularly useful to reduce overall tumor burden, to limit angiogenesis, to enhance tumor accessibility, to enhance susceptibility to ADCC, to result in increased immune function by providing more tumor antigen, or to increase the expression of the T cell attractant LIGHT.

Pharmaceutical compounds that may be used for combinatory anti-tumor therapy include, merely to illustrate: aminoglutethimide, amsacrine, anastrozole, asparaginase, beg, bicalutamide, bleomycin, buserelin, busulfan, camptothecin, capecitabine, carboplatin, carmustine, chlorambucil, cisplatin, cladribine, clodronate, colchicine, cyclophosphamide, cyproterone, cytarabine, dacarbazine, dactinomycin, daunorubicin, dienestrol, diethylstilbestrol, docetaxel, doxorubicin, epirubicin, estradiol, estramustine, etoposide, exemestane, filgrastim, fludarabine, fludrocortisone, fluorouracil, fluoxymesterone, flutamide, gemcitabine, genistein, goserelin, hydroxyurea, idarubicin, ifosfamide, imatinib, interferon, irinotecan, letrozole, leucovorin, leuprolide, levamisole, lomustine, mechlorethamine, medroxyprogesterone, megestrol, melphalan, mercaptopurine, mesna, methotrexate, mitomycin, mitotane, mitoxantrone, nilutamide, nocodazole, octreotide, oxaliplatin, paclitaxel, pamidronate, pentostatin, plicamycin, porfimer, procarbazine, raltitrexed, rituximab, streptozocin, suramin, tamoxifen, temozolomide, teniposide, testosterone, thioguanine, thiotepa, titanocene dichloride, topotecan, trastuzumab, tretinoin, vinblastine, vincristine, vindesine, and vinorelbine.

These chemotherapeutic anti-tumor compounds may be categorized by their mechanism of action into groups, including, for example, the following classes of agents: anti-metabolites/anti-cancer agents, such as pyrimidine analogs (5-fluorouracil, floxuridine, capecitabine, gemcitabine and cytarabine) and purine analogs, folate inhibitors and related inhibitors (mercaptopurine, thioguanine, pentostatin and 2-chlorodeoxyadenosine (cladribine)); antiproliferative/antimitotic agents including natural products such as vinca alkaloids (vinblastine, vincristine, and vinorelbine), microtubule disruptors such as taxane (paclitaxel, docetaxel), vincristine, vinblastine, nocodazole, epothilones and navelbine, epidipodophyllotoxins (etoposide, teniposide), DNA damaging agents (actinomycin, amsacrine, anthracyclines, bleomycin, busulfan, camptothecin, carboplatin, chlorambucil, cisplatin, cyclophosphamide, cytoxan, dactinomycin, daunorubicin, doxorubicin, epirubicin, hexamethylmelamineoxaliplatin, iphosphamide, melphalan, mechlorethamine, mitomycin, mitoxantrone, nitrosourea, plicamycin, procarbazine, taxol, taxotere, teniposide, triethylenethiophosphoramide and etoposide (VP16)); antibiotics such as dactinomycin (actinomycin D), daunorubicin, doxorubicin (adriamycin), idarubicin, anthracyclines, mitoxantrone, bleomycins, plicamycin (mithramycin) and mitomycin; enzymes (L-asparaginase which systemically metabolizes L-asparagine and deprives cells which do not have the capacity to synthesize their own asparagine); antiplatelet agents; antiproliferative/antimitotic alkylating agents such as nitrogen mustards (mechlorethamine, cyclophosphamide and analogs, melphalan, chlorambucil), ethylenimines and methylmelamines (hexamethylmelamine and thiotepa), alkyl sulfonates-busulfan, nitrosoureas (carmustine (BCNU) and analogs, streptozocin), trazenes - dacarbazinine (DTIC); antiproliferative/antimitotic antimetabolites such as folic acid analogs (methotrexate); platinum coordination complexes (cisplatin, carboplatin), procarbazine, hydroxyurea, mitotane, aminoglutethimide; hormones, hormone analogs (estrogen, tamoxifen, goserelin, bicalutamide, nilutamide) and aromatase inhibitors (letrozole, anastrozole); anticoagulants (heparin, synthetic heparin salts and other inhibitors of thrombin); fibrinolytic agents (such as tissue plasminogen activator, streptokinase and urokinase), aspirin, dipyridamole, ticlopidine, clopidogrel, abciximab; antimigratory agents; antisecretory agents (breveldin); immunosuppressives (cyclosporine, tacrolimus (FK-506), sirolimus (rapamycin), azathioprine, mycophenolate mofetil); immunomodulatory agents (thalidomide and analogs thereof such as lenalidomide (Revlimid, CC-5013) and CC-4047 (Actimid)), cyclophosphamide; anti-angiogenic compounds (TNP-470, genistein) and growth factor inhibitors (vascular endothelial growth factor (VEGF) inhibitors, fibroblast growth factor (FGF) inhibitors); angiotensin receptor blocker; nitric oxide donors; anti-sense oligonucleotides; antibodies (trastuzumab); cell cycle inhibitors and differentiation inducers (tretinoin); mTOR inhibitors, topoisomerase inhibitors (doxorubicin (adriamycin), amsacrine, camptothecin, daunorubicin, dactinomycin, eniposide, epirubicin, etoposide, idarubicin and mitoxantrone, topotecan, irinotecan), corticosteroids (cortisone, dexamethasone, hydrocortisone, methylprednisolone, prednisone, and prenisolone); growth factor signal transduction kinase inhibitors; mitochondrial dysfunction inducers and caspase activators; and chromatin disruptors.

In certain embodiments, pharmaceutical compounds that may be used for combinatory anti-angiogenesis therapy include: (1) inhibitors of release of "angiogenic molecules," such as bFGF (basic fibroblast growth factor); (2) neutralizers of angiogenic molecules, such as anti-βbFGF antibodies; and (3) inhibitors of endothelial cell response to angiogenic stimuli, including collagenase inhibitor, basement membrane turnover inhibitors, angiostatic steroids, fungal-derived angiogenesis inhibitors, platelet factor 4, thrombospondin, arthritis drugs such as D-penicillamine and gold thiomalate, vitamin D₃ analogs, alpha-interferon, and the like. For additional proposed inhibitors of angiogenesis, see Blood et al., Biochim. Biophys. Acta, 1032:89-118 (1990), Moses et al., Science, 248:1408-1410 (1990), Ingber et al., Lab. Invest., 59:44-51 (1988), and U.S. Pat. Nos. 5,092,885, 5,112,946, 5,192,744, 5,202,352, and 6,573,256. In addition, there are a wide variety of compounds that can be used to inhibit angiogenesis, for example, peptides or agents that block the VEGF-mediated angiogenesis pathway, endostatin protein or derivatives, lysine binding fragments of angiostatin, melanin or melanin-promoting compounds, plasminogen fragments (e.g., Kringles 1-3 of plasminogen), troponin subunits, inhibitors of vitronectin αᵥβ₃, peptides derived from Saposin B, antibiotics or analogs (e.g., tetracycline or neomycin), dienogest-containing compositions, compounds comprising a MetAP-2 inhibitory core coupled to a peptide, the compound EM-138, chalcone and its analogs, and naaladase inhibitors. See, for example, U.S. Pat. Nos. 6,395,718, 6,462,075, 6,465,431, 6,475,784, 6,482,802, 6,482,810, 6,500,431, 6,500,924, 6,518,298, 6,521,439, 6,525,019, 6,538,103, 6,544,758, 6,544,947, 6,548,477, 6,559,126, and 6,569,845.

### 7. Diagnostic Uses

In a further aspect, the invention provides methods for detecting and quantitating phosphoyrlation at a novel serine and/or threonine phosphorylation site of the invention. For example, peptides, including AQUA peptides of the invention, and antibodies of the invention are useful in diagnostic and prognostic evaluation of insulin-signaling disease including (among many others) cancer and diabetes, wherein the disease is associated with the phosphorylation state of a novel phosphorylation site in Table 1, whether phosphorylated or dephosphorylated.

Methods of diagnosis can be performed *in vitro* using a biological sample (e.g., blood sample, lymph node biopsy or tissue) from a subject, or in vivo. The phosphorylation state or level at the serine and/or threonine residue identified in the corresponding row in Column D of Table 1 may be assessed. A change in the phosphorylation state or level at the phosphorylation site, as compared to a control, indicates that the subject is suffering from, or susceptible to, carcinoma.

In one embodiment, the phosphorylation state or level at a novel phosphorylation site is determined by an AQUA peptide comprising the phosphorylation site. The AQUA peptide may be phosphorylated or unphosphorylated at the specified scrine and/or threonine position.

In another embodiment, the phosphorylation state or level at a phosphorylation site is determined by an antibody or antigen-binding fragment thereof, wherein the antibody specifically binds the phosphorylation site. The antibody may be one that only binds to the phosphorylation site when the serine and/or threonine residue is phosphorylated, but does not bind to the same sequence when the serine and/or threonine is not phosphorylated; or vice versa.

In particular embodiments, the antibodies of the present application are attached to labeling moieties, such as a detectable marker. One or more detectable labels can be attached to the antibodies. Exemplary labeling moieties include radiopaque dyes, radiocontrast agents, fluorescent molecules, spin-labeled molecules, enzymes, or other labeling moieties of diagnostic value, particularly in radiologic or magnetic resonance imaging techniques.

A radiolabeled antibody in accordance with this disclosure can be used for in vitro diagnostic tests. The specific activity of an antibody, binding portion thereof, probe, or ligand, depends upon the half-life, the isotopic purity of the radioactive label, and how the label is incorporated into the biological agent. In immunoassay tests, the higher the specific activity, in general, the better the sensitivity. Radioisotopes useful as labels, e.g., for use in diagnostics, include iodine (¹³¹I or ¹²⁵I), indium (¹¹¹In), technetium (⁹⁹Tc), phosphorus (³²P), carbon (¹⁴C), and tritium (³H), or one of the therapeutic isotopes listed above.

Fluorophore and chromophore labeled biological agents can be prepared from standard moieties known in the art. Since antibodies and other proteins absorb light having wavelengths up to about 310 nm, the fluorescent moieties may be selected to have substantial absorption at wavelengths above 310 nm, such as for example, above 400 nm. A variety of suitable fluorescers and chromophores are described by Stryer, Science, 162:526 (1968) and Brand et al., Annual Review of Biochemistry, 41:843-868 (1972), which are hereby incorporated by reference. The antibodies can be labeled with fluorescent chromophore groups by conventional procedures such as those disclosed in U.S. Patent Nos. 3,940,475, 4,289,747, and 4,376,110, which are hereby incorporated by reference.

The control may be parallel samples providing a basis for comparison, for example, biological samples drawn from a healthy subject, or biological samples drawn from healthy tissues of the same subject. Alternatively, the control may be a pre-determined reference or threshold amount. If the subject is being treated with a therapeutic agent, and the progress of the treatment is monitored by detecting the serine and/or threonine phosphorylation state level at a phosphorylation site of the invention, a control may be derived from biological samples drawn from the subject prior to, or during the course of the treatment.

In certain embodiments, antibody conjugates for diagnostic use in the present application are intended for use in vitro, where the antibody is linked to a secondary binding ligand or to an enzyme (an enzyme tag) that will generate a colored product upon contact with a chromogenic substrate. Examples of suitable enzymes include urease, alkaline phosphatase, (horseradish) hydrogen peroxidase and glucose oxidase. In certain embodiments, secondary binding ligands are biotin and avidin or streptavidin compounds.

Antibodies of the invention may also be optimized for use in a flow cytometry (FC) assay to determine the activation/phosphorylation status of a target signaling protein in subjects before, during, and after treatment with a therapeutic agent targeted at inhibiting serine and/or threonine phosphorylation at the phosphorylation site disclosed herein. For example, bone marrow cells or peripheral blood cells from patients may be analyzed by flow cytometry for target signaling protein phosphorylation, as well as for markers identifying various hematopoietic cell types. In this manner, activation status of the malignant cells may be specifically characterized. Flow cytometry may be carried out according to standard methods. *See, e.g*., Chow et al., Cytometry (Communications in Clinical Cytometry) 46: 72-78 (2001).

Alternatively, antibodies of the invention may be used in immunohistochemical (IHC) staining to detect differences in signal transduction or protein activity using normal and diseased tissues. IHC may be carried out according to well-known techniques. *See, e.g.,* Antibodies A Laboratory Manual, supra.

Peptides and antibodies of the invention may be also be optimized for use in other elinically-suitable applications, for example bead-based multiplex-type assays, such as IGEN, Luminex^{™} and/or Bioplex^{™} assay formats, or otherwise optimized for antibody arrays formats, such as reversed-phase array applications (see, e.g. Paweletz et al., Oncogene 20(16): 1981-89 (2001)). Accordingly, in another embodiment, the invention provides a method for the multiplex detection of the phosphorylation state or level at two or more phosphorylation sites of the invention (Table 1) in a biological sample, the method comprising utilizing two or more antibodies or AQUA peptides of the invention. In one preferred embodiment, two to five antibodies or AQUA peptides of the invention are used. In another preferred embodiment, six to ten antibodies or AQUA peptides of the invention are used, while in another preferred embodiment eleven to twenty antibodies or AQUA peptides of the invention are used.

In certain embodiments the diagnostic methods of the application may be used in combination with other diagnostic tests.

The biological sample analyzed may be any sample that is suspected of having abnormal serine and/or threonine phosphorylation at a novel phosphorylation site of the invention, such as a homogenized neoplastic tissue sample.

### 8. Screening assays

In another aspect, the invention provides a method for identifying an agent that modulates serine and/or threonine phosphorylation at a novel phosphorylation site of the invention, comprising: a) contacting a candidate agent with a peptide or protein comprising a novel phosphorylation site of the invention; and b) determining the phosphorylation state or level at the novel phosphorylation site. A change in the phosphorylation level of the specified serine and/or threonine in the presence of the test agent, as compared to a control, indicates that the candidate agent potentially modulates serine and/or threonine phosphorylation at a novel phosphorylation site of the invention.

In one embodiment, the phosphorylation state or level at a novel phosphorylation site is determined by an AQUA peptide comprising the phosphorylation site. The AQUA peptide may be phosphorylated or unphosphorylated at the specified serine and/or threonine position.

In another embodiment, the phosphorylation state or level at a phosphorylation site is determined by an antibody or antigen-binding fragment thereof, wherein the antibody specifically binds the phosphorylation site. The antibody may be one that only binds to the phosphorylation site when the serine and/or threonine residue is,phosphorylated, but does not bind to the same sequence when the serine and/or threonine is not phosphorylated; or vice versa.

In particular embodiments, the antibodies of the present application are attached to labeling moieties, such as a detectable marker.

The control may be parallel samples providing a basis for comparison, for example, the phosphorylation level of the target protein or peptide in absence of the testing agent. Alternatively, the control may be a predetermined reference or threshold amount.

### 9. Immunoassays

In another aspect, the present application concerns immunoassays for binding, purifying, quantifying and otherwise generally detecting the phosphorylation state or level at a novel phosphorylation site of the invention.

Assays may be homogeneous assays or heterogeneous assays. In a homogeneous assay the immunological reaction usually involves a phosphorylation site-specific antibody of the invention, a labeled analyte, and the sample of interest. The signal arising from the label is modified, directly or indirectly, upon the binding of the antibody to the labeled analyte. Both the immunological reaction and detection of the extent thereof are carried out in a homogeneous solution. Immunochemical labels that may be used include free radicals, radioisotopes, fluorescent dyes, enzymes, bacteriophages, coenzymes, and so forth.

In a heterogeneous assay approach, the reagents are usually the specimen, a phosphorylation site-specific antibody of the invention, and suitable means for producing a detectable signal. Similar specimens as described above may be used. The antibody is generally immobilized on a support, such as a bead, plate or slide, and contacted with the specimen suspected of containing the antigen in a liquid phase. The support is then separated from the liquid phase and either the support phase or the liquid phase is examined for a detectable signal using means for producing such signal. The signal is related to the presence of the analyte in the specimen. Means for producing a detectable signal include the use of radioactive labels, fluorescent labels, enzyme labels, and so forth.

Phosphorylation site-specific antibodies disclosed herein may be conjugated to a solid support suitable for a diagnostic assay (e.g., beads, plates, slides or wells formed from materials such as latex or polystyrene) in accordance with known techniques, such as precipitation.

In certain embodiments, immunoassays are the various types of enzyme linked immunoadsorbent assays (ELISAs) and radioimmunoassays (RIA) known in the art. Immunohistochemical detection using tissue sections is also particularly useful. However, it will be readily appreciated that detection is not limited to such techniques, and Western blotting, dot and slot blotting, FACS analyses, and the like may also be used. The steps of various useful immunoassays have been described in the scientific literature, such as, e.g., Nakamura et al., in Enzyme Immunoassays: Heterogeneous and Homogeneous Systems, Chapter 27 (1987), incorporated herein by reference.

In general, the detection of immunocomplex formation is well known in the art and may be achieved through the application of numerous approaches. These methods are based upon the detection of radioactive, fluorescent, biological or enzymatic tags. Of course, one may find additional advantages through the use of a secondary binding ligand such as a second antibody or a biotin/avidin ligand binding arrangement, as is known in the art.

The antibody used in the detection may itself be conjugated to a detectable label, wherein one would then simply detect this label. The amount of the primary immune complexes in the composition would, thereby, be determined.

Alternatively, the first antibody that becomes bound within the primary immune complexes may be detected by means of a second binding ligand that has binding affinity for the antibody. In these cases, the second binding ligand may be linked to a detectable label. The second binding ligand is itself often an antibody, which may thus be termed a "secondary" antibody. The primary immune complexes are contacted with the labeled, secondary binding ligand, or antibody, under conditions effective and for a period of time sufficient to allow the formation of secondary immune complexes. The secondary immune complexes are washed extensively to remove any non-specifically bound labeled secondary antibodies or ligands, and the remaining label in the secondary immune complex is detected.

An enzyme linked immunoadsorbent assay (ELISA) is a type of binding assay. In one type of ELISA, phosphorylation site-specific antibodies disclosed herein are immobilized onto a selected surface exhibiting protein affinity, such as a well in a polystyrene microtiter plate. Then, a suspected neoplastic tissue sample is added to the wells. After binding and washing to remove non-specifically bound immune complexes, the bound target signaling protein may be detected.

In another type of ELISA, the neoplastic tissue samples are immobilized onto the well surface and then contacted with the phosphorylation site-specific antibodies disclosed herein. After binding and washing to remove non-specifically bound immune complexes, the bound phosphorylation site-specific antibodies are detected.

Irrespective of the format used, ELISAs have certain features in common, such as coating, incubating or binding, washing to remove non-specifically bound species, and detecting the bound immune complexes.

The radioimmunoassay (RIA) is an analytical technique which depends on the competition (affinity) of an antigen for antigen-binding sites on antibody molecules. Standard curves are constructed from data gathered from a series of samples each containing the same known concentration of labeled antigen, and various, but known, concentrations of unlabeled antigen. Antigens are labeled with a radioactive isotope tracer. The mixture is incubated in contact with an antibody. Then the free antigen is separated from the antibody and the antigen bound thereto. Then, by use of a suitable detector, such as a gamma or beta radiation detector, the percent of either the bound or free labeled antigen or both is determined. This procedure is repeated for a number of samples containing various known concentrations of unlabeled antigens and the results are plotted as a standard graph. The percent of bound tracer antigens is plotted as a function of the antigen concentration. Typically, as the total antigen concentration increases the relative amount of the tracer antigen bound to the antibody decreases. After the standard graph is prepared, it is thereafter used to determine the concentration of antigen in samples undergoing analysis.

In an analysis, the sample in which the concentration of antigen is to be determined is mixed with a known amount of tracer antigen. Tracer antigen is the same antigen known to be in the sample but which has been labeled with a suitable radioactive isotope. The sample with tracer is then incubated in contact with the antibody. Then it can be counted in a suitable detector which counts the free antigen remaining in the sample. The antigen bound to the antibody or immunoadsorbent may also be similarly counted. Then, from the standard curve, the concentration of antigen in the original sample is determined.

### 10. Pharmaceutical Formulations and Methods of Administration

Methods of administration of therapeutic agents, particularly peptide and antibody therapeutics, are well-known to those of skill in the art.

Peptides of the invention can be administered in the same manner as conventional peptide type pharmaceuticals. Preferably, peptides are administered parenterally, for example, intravenously, intramuscularly, intraperitoneally, or subcutaneously. When administered orally, peptides may be proteolytically hydrolyzed. Therefore, oral application may not be usually effective. However, peptides can be administered orally as a formulation wherein peptides are not easily hydrolyzed in a digestive tract, such as liposome-microcapsules. Peptides may be also administered in suppositories, sublingual tablets, or intranasal spray.

If administered parenterally, a preferred pharmaceutical composition is an aqueous solution that, in addition to a peptide of the invention as an active ingredient, may contain for example, buffers such as phosphate, acetate, etc., osmotic pressure-adjusting agents such as sodium chloride, sucrose, and sorbitol, etc., antioxidative or antioxygenic agents, such as ascorbic acid or tocopherol and preservatives, such as antibiotics. The parenterally administered composition also may be a solution readily usable or in a lyophilized form which is dissolved in sterile water before administration.

The pharmaceutical formulations, dosage forms, and uses described below generally apply to antibody-based therapeutic agents, but are also useful and can be modified, where necessary, for making and using therapeutic agents of the disclosure that are not antibodies.

To achieve the desired therapeutic effect, the phosphorylation site-specific antibodies or antigen-binding fragments thereof can be administered in a variety of unit dosage forms. The dose will vary according to the particular antibody. For example, different antibodies may have different masses and/or affinities, and thus require different dosage levels. Antibodies prepared as Fab or other fragments will also require differing dosages than the equivalent intact immunoglobulins, as they are of considerably smaller mass than intact immunoglobulins, and thus require lower dosages to reach the same molar levels in the patient's blood. The dose will also vary depending on the manner of administration, the particular symptoms of the patient being treated, the overall health, condition, size, and age of the patient, and the judgment of the prescribing physician. Dosage levels of the antibodies for human subjects are generally between about 1 mg per kg and about 100 mg per kg per patient per treatment, such as for example, between about 5 mg per kg and about 50 mg per kg per patient per treatment. In terms of plasma concentrations, the antibody concentrations may be in the range from about 25 µg/mL to about 500 µg/mL. However, greater amounts may be required for extreme cases and smaller amounts may be sufficient for milder cases.

Administration of an antibody will generally be performed by a parenteral route, typically via injection such as intra-articular or intravascular injection (e.g., intravenous infusion) or intramuscular injection. Other routes of administration, e.g., oral (p.o.), may be used if desired and practicable for the particular antibody to be administered. An antibody can also be administered in a variety of unit dosage forms and their dosages will also vary with the size, potency, and in vivo half-life of the particular antibody being administered. Doses of a phosphorylation site-specific antibody will also vary depending on the manner of administration, the particular symptoms of the patient being treated, the overall health, condition, size, and age of the patient, and the judgment of the prescribing physician.

The frequency of administration may also be adjusted according to various parameters. These include the clinical response, the plasma half-life of the antibody, and the levels of the antibody in a body fluid, such as, blood, plasma, serum, or synovial fluid. To guide adjustment of the frequency of administration, levels of the antibody in the body fluid may be monitored during the course of treatment.

Formulations particularly useful for antibody-based therapeutic agents are also described in U.S. Patent App. Publication Nos. 20030202972, 20040091490 and 20050158316. In certain embodiments, the liquid formulations of the application are substantially free of surfactant and/or inorganic salts. In another specific embodiment, the liquid formulations have a pH ranging from about 5.0 to about 7.0. In yet another specific embodiment, the liquid formulations comprise histidine at a concentration ranging from about 1 mM to about 100 mM. In still another specific embodiment, the liquid formulations comprise histidine at a concentration ranging from 1 mM to 100 mM. It is also contemplated that the liquid formulations may further comprise one or more excipients such as a saccharide, an amino acid (e.g., arginine, lysine, and methionine) and a polyol. Additional descriptions and methods of preparing and analyzing liquid formulations can be found, for example, in PCT publications WO 03/106644, WO 04/066957, and WO 04/091658.

Wetting agents, emulsifiers and lubricants, such as sodium lauryl sulfate and magnesium stearate, as well as coloring agents, release agents, coating agents, sweetening, flavoring and perfuming agents, preservatives and antioxidants can also be present in the pharmaceutical compositions of the application.

In certain embodiments, formulations of the subject antibodies are pyrogen-free formulations which are substantially free of endotoxins and/or related pyrogenic substances. Endotoxins include toxins that are confined inside microorganisms and are released when the microorganisms are broken down or die. Pyrogenic substances also include fever-inducing, thermostable substances (glycoproteins) from the outer membrane of bacteria and other microorganisms. Both of these substances can cause fever, hypotension and shock if administered to humans. Due to the potential harmful effects, it is advantageous to remove even low amounts of endotoxins from intravenously administered pharmaceutical drug solutions. The Food & Drug Administration ("FDA") has set an upper limit of 5 endotoxin units (EU) per dose per kilogram body weight in a single one hour period for intravenous drug applications (The United States Pharmacopeial Convention, Pharmacopeial Forum 26 (1):223 (2000)). When therapeutic proteins are administered in amounts of several hundred or thousand milligrams per kilogram body weight, as can be the case with monoclonal antibodies, it is advantageous to remove even trace amounts of endotoxin.

The amount of the formulation which will be therapeutically effective can be determined by standard clinical techniques. In addition, *in vitro* assays may optionally be used to help identity optimal dosage ranges. The precise dose to be used in the formulation will also depend on the route of administration, and the seriousness of the disease or disorder, and should be decided according to the judgment of the practitioner and each patient's circumstances. Effective doses may be extrapolated from dose-response curves derived from in vitro or animal model test systems. The dosage of the compositions to be administered can be determined by the skilled artisan without undue experimentation in conjunction with standard dose-response studies. Relevant circumstances to be considered in making those determinations include the condition or conditions to be treated, the choice of composition to be administered, the age, weight, and response of the individual patient, and the severity of the patient's symptoms. For example, the actual patient body weight may be used to calculate the dose of the formulations in milliliters (mL) to be administered. There may be no downward adjustment to "ideal" weight. In such a situation, an appropriate dose may be calculated by the following formula:

Dose (mL) = [patient weight (kg) x dose level (mg/kg)/ drug concentration (mg/mL)]

For the purpose of treatment of disease, the appropriate dosage of the compounds (for example, antibodies) will depend on the severity and course of disease, the patient's clinical history and response, the toxicity of the antibodies, and the discretion of the attending physician. The initial candidate dosage may be administered to a patient. The proper dosage and treatment regimen can be established by monitoring the progress of therapy using conventional techniques known to those of skill in the art.

The formulations of the application can be distributed as articles of manufacture comprising packaging material and a pharmaceutical agent which comprises, e.g., the antibody and a pharmaceutically acceptable carrier as appropriate to the mode of administration. The packaging material will include a label which indicates that the formulation is for use in the treatment of prostate cancer.

### 11. Kits

Antibodies and peptides (including AQUA peptides) of the invention may also be used within a kit for detecting the phosphorylation state or level at a novel phosphorylation site of the invention, comprising at least one of the following: an AQUA peptide comprising the phosphorylation site, or an antibody or an antigen-binding fragment thereof that binds to an amino acid sequence comprising the phosphorylation site. Such a kit may further comprise a packaged combination of reagents in predetermined amounts with instructions for performing the diagnostic assay. Where the antibody is labeled with an enzyme, the kit will include substrates and co-factors required by the enzyme. In addition, other additives may be included such as stabilizers, buffers and the like. The relative amounts of the various reagents may be varied widely to provide for concentrations in solution of the reagents that substantially optimize the sensitivity of the assay. Particularly, the reagents may be provided as dry powders, usually lyophilized, including excipients that, on dissolution, will provide a reagent solution having the appropriate concentration.

The following Examples are provided only to further illustrate the invention, and are not intended to limit its scope, except as provided in the claims appended hereto. The invention encompasses modifications and variations of the methods taught herein which would be obvious to one of ordinary skill in the art.

### EXAMPLE 1

### Isolation of Phospho-serine and Phospho-threonine Containing Peptides From Cellular Extracts of Insulin-Responsive Tissue Samples and Identification of Novel Phosphorylation Sites.

In order to discover novel serine and/or threonine phosphorylation sites in insulin-signaling related pathways, IAP isolation techniques were used to identify phosphoserine and/or threonine-containing peptides in cell extracts from cellular extracts from insulin-responsive tissue samples identified in Column G of Table 1 including 3T3-L1; mouse liver; mouse Akt2(-/-) liver Tryptic phosphoserine and/or threonine-containing peptides were purified and analyzed from extracts of each of the cell lines mentioned above, as follows. Cells were cultured in DMEM medium or RPMI 1640 medium supplemented with 10% fetal bovine serum and penicillin/streptomycin.

Suspension cells were harvested by low speed centrifugation. After complete aspiration of medium, cells were resuspended in 1 mL lysis buffer per 1.25 x 10⁸ cells (20 mM HEPES pH 8.0, 9 M urea, 1 mM sodium vanadate, supplemented or not with 2.5 mM sodium pyro-phosphate, 1 mM β-glycerol-phosphate) and sonicated.

Adherent cells at about 80% confluency were starved in medium without serum overnight and stimulated, with ligand depending on the cell type or not stimulated. After complete aspiration of medium from the plates, cells were scraped off the plate in 10 ml lysis buffer per 2x10⁸ cells (20 mM HEPES pH 8.0, 9 M urea, 1 mM sodium vanadate, supplemented with 2.5 mM sodium pyraphasphate, 1 mM β-glycerol-phosphate) and sonicated.

Frozen tissue samples were cut to small pieces, homogenize in lysis buffer (20 mM HEPES pH 8.0, 9 M Urea, 1 mN sodium vanadate, supplemented with 2.5 mM sodium pyrophosphate, 1 mM b-glycerol-phasphate. 1 ml lysis buffer for 100 mg of frozen tissue) using a polytron for 2 times of 20 see. each time. Homogenate is then briefly sonicated.

Sonicated cell lysates were cleared by centrifugation at 20,000 x g, and proteins were reduced with DTT at a final concentration of 4.1 mM and alkylated with iodoacetamide at 8.3 mM. For digestion with trypsin, protein extracts were diluted in 20 mM HEPES pH 8.0 to a final concentration of 2 M urea and soluble TLCK-trypsin (Worthington) was added at 10-20 µg/mL. Digestion was performed for 1-2 days at room temperature.

Trifluoroacetic acid (TFA) was added to protein digests to a final concentration of 1%, precipitate was removed by centrifugation, and digests were loaded onto Sep-Pak C₁₈ columns (Waters) equilibrated with 0.1% TFA. A column volume of 0.7-1.0 ml was used per 2x10⁸ cells. Columns were washed with 15 volumes of 0.1% TFA, followed by 4 volumes of 5% acetonitrile (MeCN) in 0.1 % TFA. Peptide fraction I was obtained by eluting columns with 2 volumes each of 8, 12, and 15% MeCN in 0.1% TFA and combining the eluates. Fractions II and III were a combination of eluates after eluting columns with 18, 22, 25% MeCN in 0.1% TFA and with 30, 35,40% MEON in 0.1% TFA, respectively. All peptide fractions were lyophilized.

Peptides from each fraction corresponding to 2 x 10⁸ cells were dissolved in 1 ml of IAP buffer (20 mM Tris/HCl or 50 mM MOPS pH 7.2, 10 mM sodium phosphate, 50 mM NaCl) and insoluble matter (mainly in peptide fractions III) was removed by centrifugation. IAP was performed on each peptide fraction separately. The phosphoserine and/or threonine monoclonal antibody P-Tyr-100 (Cell Signaling Technology, Inc., catalog number 9411) was coupled at 4 mg/ml beads to protein G (Roche), respectively. Immobilized antibody (15 µl, 60 µg) was added as 1:1 slurry in IAP buffer to 1 ml of each peptide fraction, and the mixture was incubated overnight at 4° C with gentle rotation. The immobilized antibody beads were washed three times with 1 ml IAP buffer and twice with I ml water, all at 4° C. Peptides were eluted from beads by incubation with 75 µl of 0.1% TFA at room temperature for 10 minutes.

Alternatively, one single peptide fraction was obtained from Sep-Pak C18 columns by elution with 2 volumes each of 10%, 15%, 20 %, 25 %, 30 %, 35 % and 40 % acetonitirile in 0.1% TFA and combination of all eluates. IAP on this peptide fraction was performed as follows: After

lyophilization, peptide was dissolved in 1.4 ml IAP buffer (MOPS pH 7.2,

10 mM sodium phosphate, 50 mM NaCl) and insoluble matter was removed by centrifugation. Immobilized antibody (40 µl, 160 µg) was added as 1:1 slurry in IAP buffer, and the mixture was incubated overnight at 4° C with gentle shaking. The immobilized antibody beads were washed three times with 1 ml IAP buffer and twice with 1 ml water, all at 4° C. Peptides were eluted from beads by incubation with 55 µl of 0.15% TFA at room temperature for 10 min (eluate 1), followed by a wash of the beads (eluate 2) with 45 µl of 0.15% TFA. Both eluates were combined.

### Analysis by LC-MS/MS Mass Spectrometry.

40 µl or more of IAP eluate were purified by 0.2 µl Stage Tips or ZipTips. Peptides were eluted from the microcolumns with 1 µl of 40% MeCN, 0.1% TFA (fractions I and II) or 1 µl of 60% MeCN, 0.1% TFA (fraction III) into 7.6-9.0 µl of 0.4% acetic acid/0.005% heptafluorobutyric acid. For single fraction analysis, 1 µl of 60% MeCN, 0.1% TFA, was used for elution from the microcolumns. This sample was loaded onto a 10 cm x 75 µm PicoFrit capillary column (New Objective) packed with Magic C18 AQ reversed-phase resin (Michrom Bioresources) using a Famos autosampler with an inert sample injection valve (Dionex). The column was then developed with a 45-min linear gradient of acetonitrile delivered at 200 nl/min (Ultimate, Dionex), and tandem mass spectra were collected in a data-dependent manner with an LTQ ion trap mass spectrometer essentially as described by Gygi *et al*., *supra*.

### Database Analysis & Assignments.

MS/MS spectra were evaluated using TurboSequest in the Sequest Browser package (v. 27, rev. 12) supplied as part of BioWorks 3.0 (ThermoFinnigan). Individual MS/MS spectra were extracted from the raw data file using the Sequest Browser program CreateDta, with the following settings: bottom MW, 700; top MW, 4,500; minimum number of ions, 20 (40 for LTQ); minimum TIC, 4 x 10⁵ (2 x 10³ for LTQ); and precursor charge state, unspecified. Spectra were extracted from the beginning of the raw data file before sample injection to the end of the eluting gradient. The IonQuest and VuDta programs were not used to further select MS/MS spectra for Sequest analysis. MS/MS spectra were evaluated with the following TurboSequest parameters: peptide mass tolerance, 2.5; fragment ion tolerance, 0.0 (1.0 for LTQ); maximum number of differential amino acids per modification, 4; mass type parent, average; mass type fragment, average; maximum number of internal cleavage sites, 10; neutral losses of water and ammonia from b and y ions were considered in the correlation analysis. Proteolytic enzyme was specified except for spectra collected from elastase digests.

Searches were performed against the then current NCBI human protein database. Cysteine carboxamidomethylation was specified as a static modification, and phosphorylation was allowed as a variable modification on serine and/or threonine residues. It was determined that restricting phosphorylation to serine and/or threonine residues had little effect on the number of phosphorylation sites assigned.

In proteomics research, it is desirable to validate protein identifications based solely on the observation of a single peptide in one experimental result, in order to indicate that the protein is, in fact, present in a sample. This has led to the development of statistical methods for validating peptide assignments, which are not yet universally accepted, and guidelines for the publication of protein and peptide identification results (*see* Carr et al., Mol. Cell Proteomics 3: 531-533 (2004)), which were followed in this Example. However, because the immunoaffinity strategy separates phosphorylated peptides from unphosphorylated peptides, observing just one phosphopeptide from a protein is a common result, since many phosphorylated proteins have only one serine and/or threonine-phosphorylatcd site. For this reason, it is appropriate to use additional criteria to validate phosphopeptide assignments. Assignments are likely to be correct if any of these additional criteria are met: (i) the same phosphopeptide sequence is assigned to co-eluting ions with different charge states, since the MS/MS spectrum changes markedly with charge state; (ii) the phosphorylation site is found in more than one peptide sequence context due to sequence overlaps from incomplete proteolysis or use of proteases other than trypsin; (iii) the phosphorylation site is found in more than one peptide sequence context due to homologous but not identical protein isoforms; (iv) the phosphorylation site is found in more than one peptide sequence context due to homologous but not identical proteins among species; and (v) phosphorylation sites validated by MS/MS analysis of synthetic phosphopeptides corresponding to assigned sequences, since the ion trap mass spectrometer produces highly reproducible MS/MS spectra. The last criterion is routinely used to confirm novel site assignments of particular interest.

All spectra and all sequence assignments made by Sequest were imported into a relational database. The following Sequest scoring thresholds were used to select phosphopeptide assignments that are likely to be correct: RSp < 6, XCorr ≥ 2.2, and DeltaCN > 0.099. Further, the sequence assignments could be accepted or rejected with respect to accuracy by using the following conservative, two-step process.

In the first step, a subset of high-scoring sequence assignments should be selected by filtering for XCorr values of at least 1.5 for a charge state of+1, 2.2 for +2, and 3.3 for +3, allowing a maximum RSp value of 10. Assignments in this subset should be rejected if any of the following criteria are satisfied: (i) the spectrum contains at least one major peak (at least 10% as intense as the most intense ion in the spectrum) that can not be mapped to the assigned sequence as an a, *b,* or *y* ion, as an ion arising from neutral-loss of water or ammonia from a b or y ion, or as a multiply protonated ion; (ii) the spectrum does not contain a series of *b* or y ions equivalent to at least six uninterrupted residues; or (iii) the sequence is not observed at least five times in all the studies conducted (except for overlapping sequences due to incomplete proteolysis or use of proteases other than trypsin).

In the second step, assignments with below-threshold scores should be accepted if the low-scoring spectrum shows a high degree of similarity to a high-scoring spectrum collected in another study, which simulates a true reference library-searching strategy.

### EXAMPLE 2

### Production of Phosphorylation site-Specific Polyclonal Antibodies

Polyclonal antibodies that specifically bind a novel phosphorylation site of the invention (Table I/Figure 2) only when the serine and/or threonine residue is phosphorylated (and does not bind to the same sequence when the serine and/or threonine is not phosphorylated), and vice versa, are produced according to standard methods by first constructing a synthetic peptide antigen comprising the phosphorylation site and then immunizing an animal to raise antibodies against the antigen, as further described below. Production of exemplary polyclonal antibodies is provided below.

### A. Rictor (threonine 1135).

A 15 amino acid phospho-peptide antigen, NRRIRTLt*EPSVDFN (SEQ NO:1; t*= phosphothreonine), which comprises the phosphorylation site derived from human Rictor (an adaptor/scaffold protein, Thr 1135 being the phosphorylatable residue), plus cysteine on the C-terminal for coupling, is constructed according to standard synthesis techniques using, e.g., a Rairun/Protem Technologies, Inc., Symphony peptide synthesizer. See ANTIBODIES: A LABORATORY MANUAL, *supra.;* Merrifield, *supra.* This peptide is then coupled to KLH and used to immunize animals to produce (and subsequently screen) phosphorylation site-specific polyclonal antibodies as described in Immunization/Screening below.

### B. Z02 (serine 220).

A 15 amino acid phospho-peptide antigen, RDRSRGRs*LERGLDH (SEQ NO:2; s*= phosphoserine), which comprises the phosphorylation site derived from human ZO2 (an adaptor/scaffold protein, Ser 220 being the phosphorylatable residue), plus cysteine on the C-terminal for coupling, is constructed according to standard synthesis techniques using, e.g., a Rainin/Protein Technologies, Inc., Symphony peptide synthesizer. *See* ANTIBODIES: A LABORATORY MANUAL, *supra.;* Merrifield, *supra.* This peptide is then coupled to KLH and used to immunize animals to produce (and subsequently screen) phosphorylation site-specific polyclonal antibodies as described in Immunization/Screening below.

### C. APPL2 (serine 508).

A 15 amino acid phospho-peptide antigen, SMAVKTDs*TTEVIYE (SEQ NO: 3; s*= phosphoserine), which comprises the phosphorylation site derived from human APPL2 (an adaptor/scaffold protein, Ser 508 being the phosphorylatable residue), plus cysteine on the C-terminal for coupling, is constructed according to standard synthesis techniques using, e.g., a Rainin/Protein Technologies, Inc., Symphony peptide synthesizer. *See* ANTIBODIES: A LABORATORY MANUAL, *supra.;* Merrifield, *supra.* This peptide is then coupled to KLH and used to immunize animals to produce (and subsequently screen) phosphorylation site-specific polyclonal antibodies as described in Immunization/Screening below.

### Immunization/Screening.

A synthetic phospho-peptide antigen as described in A-C above is coupled to KLH, and rabbits are injected intradermally (ID) on the back with antigen in complete Freunds adjuvant (500 µg antigen per rabbit). The rabbits are boosted with same antigen in incomplete Freund adjuvant (250 µg antigen per rabbit) every three weeks. After the fifth boost, bleeds are collected. The sera are purified by Protein A-affinity chromatography by standard methods (*see* ANTIBODIES: A LABORATORY MANUAL, Cold Spring Harbor, *supra.*). The eluted immunoglobulins are further loaded onto an unphosphorylated synthetic peptide antigen-resin Knotes column to pull out antibodies that bind the unphosphorylated form of the phosphorylation sites. The flow through fraction is collected and applied onto a phospho-synthetic peptide antigen-resin column to isolate antibodies that bind the phosphorylated form of the phosphorylation sites. After washing the column extensively, the bound antibodies (i.e. antibodies that bind the phosphorylated peptides described in A-C above, but do not bind the unphosphorylated form of the peptides) are eluted and kept in antibody storage buffer.

The isolated antibody is then tested for phospho-specificity using Western blot assay using an appropriate cell line that expresses (or overexpresses) target phospho-protein (*i.e,* phosphorylated Rictor, Zo2 or APPL2), found in, for example, 3T3-L1 or mouse liver cells. Cells are cultured in DMEM or RPMI supplemented with 10% FCS. Cell are collected, washed with PBS and directly lysed in cell lysis buffer. The protein concentration of cell lysates is then measured. The loading buffer is added into cell lysate and the mixture is boiled at 100 °C for 5 minutes. 20 µl (10 µg protein) of sample is then added onto 7.5% SDS-PAGE gel.

A standard Western blot may be performed according to the Immunoblotting Protocol set out in the CELL SIGNALING TECHNOLOGY, INC. 2003-04 Catalogue, p. 390. The isolated phosphorylation site-specific antibody is used at dilution 1:1000. Phospho-specificity of the antibody will be shown by binding of only the phosphorylated form of the target amino acid sequence. Isolated phosphorylation site-specific polyclonal antibody does not (substantially) recognize the same target sequence when not phosphorylated at the specified serine and/or threonine position (e.g., the antibody does not bind to Z02 in the non-stimulated cells, when serine 220 is not phosphorylated).

In order to confirm the specificity of the isolated antibody, different cell lysates containing various phosphorylated signaling proteins other than the target protein are prepared. The Western blot assay is performed again using these cell lysates. The phosphorylation site-specific polyclonal antibody isolated as described above is used (1:1000 dilution) to test reactivity with the different phosphorylated non-target proteins. The phosphorylation site-specific antibody does not significantly cross-react with other phosphorylated signaling proteins that do not have the described phosphorylation site, although occasionally slight binding to a highly homologous sequence on another protein may be observed. In such case the antibody may be further purified using affinity chromatography, or the specific immunoreactivity cloned by rabbit hybridoma technology.

### EXAMPLE 3

### Production of Phosphorylation site-spceific Monoclonal Antibodies

Monoclonal antibodies that specifically bind a novel phosphorylation site of the invention (Table 1) only when the serine and/or threonine residue is phosphorylated (and does not bind to the same sequence when the serine and/or threonine is not phosphorylated) are produced according to standard methods by first constructing a synthetic peptide antigen comprising the phosphorylation site and then immunizing an animal to raise antibodies against the antigen, and harvesting spleen cells from such animals to produce fusion hybridomas, as further described below. Production of exemplary monoclonal antibodies is provided below.

### A. ATG6 (serine 90).

A 15 amino acid phospho-peptide antigen, IPPARMMs*TESANSF (SEQ ID NO: 4; s*= phosphoserine), which comprises the phosphorylation site derived from human ATG6 (an adaptor/scaffold protein, Ser 90 being the phosphorylatable residue), plus cysteine on the C-terminal for coupling, is constructed according to standard synthesis techniques using, *e.g.,* a Rainin/Protein Technologies, Inc., Symphony peptide synthesizer. See ANTIBODIES: A LABORATORY MANUAL, *supra.;* Merrifield, *supra.* This peptide is then coupled to KLH and used to immunize animals and harvest spleen cells for generation (and subsequent screening) of phosphorylation site-specific monoclonal antibodies as described in Immunization/Fusion/Screening below.

### B. Tks5 (serine 988).

A 15 amino acid phospho-peptide antigen, LRGVRRNs*SFSTARS (SEQ ID NO: 10; s*= phosphoserine), which comprises the phosphorylation site derived from human NDE1 (an adaptor/scaffold protein, Ser 988 being the phosphorylatable residue), plus cysteine on the C-terminal for coupling, is constructed according to standard synthesis techniques using, *e.g.,* a Rainin/Protein Technologies, Inc., Symphony peptide synthesizer. *See* ANTIBODIES: A LABORATORY MANUAL, *supra.;* Merrifield, *supra.* This peptide is then coupled to KLH and used to immunize animals and harvest spleen cells for generation (and subsequent screening) of phosphorylation site-specific monoclonal antibodies as described in Immunization/Fusion/Screening below

### C. JMJD2C (serine 1027).

A 15 amino acid phospho-peptide antigen, RKRQRVLs*SRFKNEY (SEQ ID NO: 19; s*= phosphoserine), which comprises the phosphorylation site derived from human JMJD2C (an enzyme protein, Ser 1027 being the phosphorylatable residue), plus cysteine on the C-terminal for coupling, is constructed according to standard synthesis techniques using, *e.g.,* a Rainin/Protein Technologies, Inc., Symphony peptide synthesizer. See ANTIBODIES: A LABORATORY MANUAL, *supra.;* Merrifield, *supra.* This peptide is then coupled to KLH and used to immunize animals and harvest spleen cells for generation (and subsequent screening) of phosphorylation site-specific monoclonal antibodies as described in Immunization/Fusion/Scrcening below

### Immunization/Fusion/Screening.

A synthetic phospho-peptide antigen as described in A-C above is coupled to KLH, and BALB/C mice are injected intradermally (ID) on the back with antigen in complete Freunds adjuvant (e.g., 50 µg antigen per mouse). The mice are boosted with same antigen in incomplete Freund adjuvant (e.g. 25 µg antigen per mouse) every three weeks. After the fifth boost, the animals are sacrificed and spleens are harvested.

Harvested spleen cells are fused to SP2/0 mouse myeloma fusion partner cells according to the standard protocol of Kohler and Milstein (1975). Colonies originating from the fusion are screened by ELISA for reactivity to the phospho-peptide and non-phospho-peptide forms of the antigen and by Western blot analysis (as described in Example 1 above). Colonies found to be positive by ELISA to the phospho-peptide while negative to the non-phospho-peptide are further characterized by Western blot analysis. Colonies found to be positive by Western blot analysis are subcloned by limited dilution. Mouse ascites are produced from a single clone obtained from subcloning, and tested for phospho-specificity (against the ATG6, Tks5 or JMJD2C) phospho-peptide antigen, as the case may be) on ELISA. Clones identified as positive on Western blot analysis using cell culture supernatant as having phospho-specificity, as indicated by a strong band in the induced lane and a weak band in the uninduced lane of the blot, are isolated and subcloned as clones producing monoclonal antibodies with the desired specificity.

Ascites fluid from isolated clones may be further tested by Western blot analysis. The ascites fluid should produce similar results on Western blot analysis as observed previously with the cell culture supernatant, indicating phospho-specificity against the phosphorylated target.

### EXAMPLE 4

### Production and Use of AQUA Peptides for Detecting and Quantitating Phosphorylation at a Novel Phosphorylation Site

Heavy-isotope labeled peptides (AQUA peptides (internal standards)) for the detecting and quantitating a novel phosphorylation site of the invention (Table 1) only when the serine and/or threonine residue is phosphorylated are produced according to the standard AQUA methodology (*see* Gygi *et al.,* Gerber *et al., supra.)* methods by first constructing a synthetic peptide standard corresponding to the phosphorylation site sequence and incorporating a heavy-isotope label. Subsequently, the MSⁿ and LC-SRM signature of the peptide standard is validated, and the AQUA peptide is used to quantify native peptide in a biological sample, such as a digested cell extract. Production and use of exemplary AQUA peptides is provided below.

### A. adolase A (serine 45).

An AQUA peptide comprising the sequence, SIAKRLQs*IGTENTE (SEQ ID NO: 20; y*= phosphoserine; Leucine being ¹⁴C/¹⁵N-labeled, as indicated in bold), which comprises the phosphorylation site derived from human adolase A (an enzyme, Ser 45 being the phosphorylatable residue), is constructed according to standard synthesis techniques using, *e.g.,* a Rainin/Protein Technologies, Inc., Symphony peptide synthesizer (see Merrifield, *supra*.) as further described below in Synthesis & MS/MS Signature. The adolase A (Ser 45) AQUA peptide is then spiked into a biological sample to quantify the amount of phosphorylated adolase A (Ser 45) in the sample, as further described below in Analysis & Quantification.

### B. glucokinase (threonine 49).

An AQUA peptide comprising the sequence DRGLRLEt*HEEASVK (SEQ ID NO: 21' y*= phosphothreonine; Valine being ¹⁴C/¹⁵N-labeled, as indicated in bold), which comprises the phosphorylation site derived from human glucokinase (a non-protein kinase, Thr 49 being the phosphorylatable residue), is constructed according to standard synthesis techniques using, *e.g.,* a Rainin/Protein Technologies, Inc., Symphony peptide synthesizer (see Merrifield, *supra*.) as further described below in Synthesis & MS/MS Signature. The glucokinase (Thr 49) AQUA peptide is then spiked into a biological sample to quantify the amount of phosphorylated glucokinase (Thr 49) in the sample, as further described below in Analysis & Quantification.

### C. PTPN14 (threonine 670).

An AQUA peptide comprising the sequence LPMARRNt*LREQGPP (SEQ ID NO: 23; t*= phosphothreonine; Proline being ¹⁴C/¹⁵N-labeled, as indicated in bold), which comprises the phosphorylation site derived from human PTPN14 (a phosphatase, Thr 670 being the phosphorylatable residue), is constructed according to standard synthesis techniques using, *e.g.*, a Rainin/Protein Technologies, Inc., Symphony peptide synthesizer (see Merrifield, *supra.*) as further described below in Synthesis & MS/MS Signature. The PTPN14 (Thr 670) AQUA peptide is then spiked into a biological sample to quantify the amount of phosphorylated PTPN 14 (Thr 670) in the sample, as further described below in Analysis & Quantification.

### D. DAPK2 (threonine 369).

An AQUA peptide comprising the sequence HPRRRSSt*S (SEQ ID NO: 30; t*= phosphothreonine; Proline being ¹⁴C/¹⁵N-labeled, as indicated in bold), which comprises the phosphorylation site derived from human DAPK2 (a ser/thr protein kinase (non-receptor), Thr 369 being the phosphorylatable residue), is constructed according to standard synthesis techniques using, *e.g.,* a Rainin/Protein Technologies, Inc., Symphony peptide synthesizer (see Merrifield, *supra*.) as further described below in Synthesis & MS/MS Signature. The DAPK2 (thr 369) AQUA peptide is then spiked into a biological sample to quantify the amount of phosphorylated DAPK2 (thr 369) in the sample, as further described below in Analysis & Quantification.

### Synthesis & MS/MS Spectra.

Fluorenylmethoxycarbonyl (Fmoc)-derivatized amino acid monomers may be obtained from AnaSpec (San Jose, CA). Fmoc-derivatized stable-isotope monomers containing one ¹⁵N and five to nine ¹³C atoms may be obtained from Cambridge Isotope Laboratories (Andover, MA). Preloaded Wang resins may be obtained from Applied Biosystems. Synthesis scales may vary from 5 to 25 µmol. Amino acids are *activated in situ* with 1-H-benzotriazolium, 1-bis(dimethylamino) methylene]-hexafluorophosphate
(1-),3-oxide:1-hydroxybenzotriazole hydrate and coupled at a 5-fold molar excess over peptide. Each coupling cycle is followed by capping with acetic anhydride to avoid accumulation of one-residue deletion peptide by-products. After synthesis peptide-resins are treated with a standard scavenger-containing trifluoroacetic acid (TFA)-water cleavage solution, and the peptides are precipitated by addition to cold ether. Peptides (i.e. a desired AQUA peptide described in A-D above) are purified by reversed-phase C18 HPLC using standard TFA/acetonitrile gradients and characterized by matrix-assisted laser desorption ionization-time of flight (Biflex III, Bruker Daltonics, Billerica, MA) and ion-trap (ThermoFinnigan, LCQ DecaXP or LTQ) MS.

MS/MS spectra for each AQUA peptide should exhibit a strong *y*-type ion peak as the most intense fragment ion that is suitable for use in an SRM monitoring/analysis. Reverse-phase microcapillary columns (0.1 Å∼ 150-220 mm) are prepared according to standard methods. An Agilent 1100 liquid chromatograph may be used to develop and deliver a solvent gradient [0.4% acetic acid/0.005% heptafluorobutyric acid (HFBA)/7% methanol and 0.4% acetic acid/0.005% HFBA/65% methanol/35% acetonitrile] to the microcapillary column by means of a flow splitter. Samples are then directly loaded onto the microcapillary column by using a FAMOS inert capillary autosampler (LC Packings, San Francisco) after the flow split. Peptides are reconstituted in 6% acetic acid/0.01% TFA before injection.

### Analysis & Quantification.

Target protein (*e.g.* a phosphorylated proteins of A-D above) in a biological sample is quantified using a validated AQUA peptide (as described above). The IAP method is then applied to the complex mixture of peptides derived from proteolytic cleavage of crude cell extracts to which the AQUA peptides have been spiked in.

LC-SRM of the entire sample is then carried out. MS/MS may be performed by using a ThermoFinnigan (San Jose, CA) mass spectrometer (LCQ DecaXP ion trap or TSQ Quantum triple quadrupole or LTQ). On the DecaXP, parent ions are isolated at 1.6 m/z width, the ion injection time being limited to 150 ms per microscan, with two microscans per peptide averaged, and with an AGC setting of 1 x 10⁸; on the Quantum, Q1 is kept at 0.4 and Q3 at 0.8 m/z with a scan time of 200 ms per peptide. On both instruments, analyte and internal standard are analyzed in alternation within a previously known reverse-phase retention window; well-resolved pairs of internal standard and analyte are analyzed in separate retention segments to improve duty cycle. Data are processed by integrating the appropriate peaks in an extracted ion chromatogram (60.15 m/z from the fragment monitored) for the native and internal standard, followed by calculation of the ratio of peak areas multiplied by the absolute amount of internal standard (e.g., 500 fmol).

## Claims

1. An antibody or antigen-binding fragment thereof, wherein the antibody specifically binds to a protein selected from Column A of Table 1, Rows 33, 37, 51, 96 and 2 only when phosphorylated at the serine or threonine listed in corresponding Column D of Table 1, comprised within the phosphorylatable peptide sequence listed in corresponding Column E of Table 1 (SEQ ID NOs: 32, 36, 50, 95 and 1), wherein said antibody does not bind said protein when not phosphorylated at said serine or threonine.

2. An antibody or antigen-binding fragment thereof, wherein the antibody specifically binds to a protein selected from Column A of Table1, Rows 33, 37, 51, 96 and 2 only when not phosphorylated at the serine or threonine listed in corresponding Column D of Table 1, comprised within the phosphorylatable peptide sequence listed in corresponding Column E of Table 1 (SEQ ID NOs: 32, 36, 50, 95 and 1), wherein said antibody does not bind said protein when phosphorylated at said serine or threonine.

3. A method selected from the group consisting of:
(a) a method for detecting a protein selected from Column A of Table 1, Rows 33, 37, 51, 96 and 2 wherein said protein is phosphorylated at the serine or threonine listed in corresponding Column D of Table 1, comprised within the phosphorylatable peptide sequence listed in corresponding Column E of Table 1 (SEQ ID NOs: 32, 36, 50, 95 and 1), comprising the step of adding an antibody or antigen-binding fragment thereof according to claim 1, to a sample comprising said protein under conditions that permit the binding of said antibody to said protein, and detecting bound antibody;
(b) a method for quantifying the amount of a protein listed in Column A of Table 1, Rows 33, 37, 51, 96 and 2 that is phosphorylated at the corresponding serine or threonine listed in Column D of Table 1, comprised within the phosphorylatable peptide sequence listed in corresponding Column E of Table 1 (SEQ ID NOs: 32, 36, 50, 95 and 1), in a sample using a heavy-isotope labeled peptide (AQUA^{™} peptide), said labeled peptide comprising a phosphorylated serine or threonine at said corresponding serine or threonine listed Column D of Table 1, comprised within the phosphorylatable peptide sequence listed in corresponding Column E of Table 1 as an internal standard; and
(c) a method comprising step (a) followed by step (b).

4. The method of claim 3, wherein said antibody or antigen-binding fragment thereof is capable of specifically binding QSK only when phosphorylated at T411, comprised within the phosphorylatable peptide sequence listed in Column E, Row 33, of Table 1 (SEQ ID NO: 32), wherein said antibody does not bind said protein when not phosphorylated at said threonine.

5. The method of claim 3, wherein said antibody or antigen-binding fragment thereof is capable of specifically binding eIF3-theta only when phosphorylated at T574, comprised within the phosphorylatable peptide sequence listed in Column E, Row 96, of Table 1 (SEQ ID NO: 95), wherein said antibody does not bind said protein when not phosphorylated at said threonine.

6. The method of claim 3, wherein said antibody or antigen-binding fragment thereof is capable of specifically binding eIF3-theta only when not phosphorylated at T574, comprised within the phosphorylatable peptide sequence listed in Column E, Row 96, of Table 1 (SEQ ID NO: 95), wherein said antibody does not bind said protein when phosphorylated at said threonine.

7. An antibody or antigen-binding fragment thereof capable of specifically binding the protein Rictor only when phosphorylated at T1135, comprised within the phosphorylatable peptide sequence listed in Column E, Row 2, of Table 1 (SEQ ID NO: 1), wherein said antibody or antigen-binding fragment thereof does not bind said protein when not phosphorylated at said threonine.

8. An antibody or antigen-binding fragment thereof capable of specifically binding the protein Rictor only when not phosphorylated at T1135, comprised within the phosphorylatable peptide sequence listed in Column E, Row 2, of Table 1 (SEQ ID NO: 1), wherein said antibody or antigen-binding fragment thereof does not bind said protein when phosphorylated at said threonine.

9. An antibody or antigen-binding fragment thereof capable of specifically binding the protein PKD3 only when phosphorylated at S252, comprised within the phosphorylatable peptide sequence listed in Column E, Row 37, of Table 1 (SEQ ID NO: 36), wherein said antibody or antigen-binding fragment thereof does not bind said protein when not phosphorylated at said serine.

10. An antibody or antigen-binding fragment thereof capable of specifically binding the protein PKD3 only when not phosphorylated at S252, comprised within the phosphorylatable peptide sequence listed in Column E, Row 37, of Table 1 (SEQ ID NO: 36), wherein said antibody or antigen-binding fragment thereof does not bind said protein when phosphorylated at said serine.

11. An antibody or antigen-binding fragment thereof capable of specifically binding the protein RapGEF1 only when phosphorylated at T1071, comprised within the phosphorylatable peptide sequence listed in Column E, Row 51, of Table 1 (SEQ ID NO: 50), wherein said antibody or antigen-binding fragment thereof does not bind said protein when not phosphorylated at said threonine.

12. An antibody or antigen-binding fragment thereof capable of specifically binding the protein RapGEF1 only when not phosphorylated at T1071, comprised within the phosphorylatable peptide sequence listed in Column E, Row 51, of Table 1 (SEQ ID NO: 50), wherein said antibody or antigen-binding fragment thereof does not bind said protein when phosphorylated at said threonine.

13. An antibody or antigen-binding fragment thereof capable of specifically binding the protein eIF3-theta only when phosphorylated at T574, comprised within the phosphorylatable peptide sequence listed in Column E, Row 96, of Table 1 (SEQ ID NO: 95), wherein said antibody or antigen-binding fragment thereof does not bind said protein when not phosphorylated at said threonine.

14. An antibody or antigen-binding fragment thereof capable of specifically binding the protein eIF3-theta only when phosphorylated at T574, comprised within the phosphorylatable peptide sequence listed in Column E, Row 96, of Table 1 (SEQ ID NO: 95), wherein said antibody or antigen-binding fragment thereof does not bind said protein when not phosphorylated at said threonine.
